(19) Europäisches Patentamt
European Patent Office
Office européen des brevets

(11) EP 2 027 266 B1

(12) **EUROPÄISCHE PATENTSCHRIFT**

(45) Veröffentlichungstag und Bekanntmachung des Hinweises auf die Patenterteilung:
**22.12.2010 Patentblatt 2010/51**

(21) Anmeldenummer: **07722435.0**

(22) Anmeldetag: **11.05.2007**

(51) Int Cl.:
***C12N 15/11*** *(2006.01)*

(86) Internationale Anmeldenummer:
**PCT/DE2007/000887**

(87) Internationale Veröffentlichungsnummer:
**WO 2007/131495 (22.11.2007 Gazette 2007/47)**

(54) **MULTIMER ZUR IMMUNSTIMULATION**

MULTIMER FOR IMMUNOSTIMULATION

MULTIMÈRE POUR L'IMMUNOSTIMULATION

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **11.05.2006 DE 102006023332**

(43) Veröffentlichungstag der Anmeldung:
**25.02.2009 Patentblatt 2009/09**

(73) Patentinhaber: **Mologen AG**
**14195 Berlin (DE)**

(72) Erfinder:
- **SCHROFF, Matthias**
**14195 Berlin (DE)**
- **WITTIG, Burghardt**
**14129 Berlin (DE)**
- **SCHMIDT, Manuel**
**14195 Berlin (DE)**
- **LÖHR, Janine**
**12053 Berlin (DE)**

(74) Vertreter: **Harrison, Robert John et al**
**24IP Law Group Sonnenberg Fortmann**
**Herzogspitalstraße 10a**
**80331 München (DE)**

(56) Entgegenhaltungen:
**WO-A-01/07055 WO-A-02/060476**

- **SCHMIDT M ET AL: "Cytokine and Ig-production by CG-containing sequences with phosphorodiester backbone and dumbbell-shape" ALLERGY (OXFORD), Bd. 61, Nr. 1, Januar 2006 (2006-01), Seiten 56-63, XP002461380 ISSN: 0105-4538**
- **KRIEG ARTHUR M: "CpG motifs: The active ingredient in bacterial extracts?" NATURE MEDICINE, Bd. 9, Nr. 7, Juli 2003 (2003-07), Seiten 831-835, XP002461381 ISSN: 1078-8956**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 027 266 B1

Printed by Jouve, 75001 PARIS (FR)

## Beschreibung

Die Erfindung betrifft ein multimeres nicht-kodierendes Nukleinsäuremolekül zur Modulation der Aktivität des menschlichen oder tierischen Immunsystems sowie ein Herstellungsverfahren desselben und eine Vakzine, die das multimere nichtkodierende Nukleinsäuremolekül enthält.

Während die adaptive Immunantwort nach Selektion der für das jeweilige Pathogen spezifischen Lymphozyten und deren klonaler Expansion und Differenzierung zu Effektorzellen erst verzögert (3-5 Tage) einsetzt und dann aber lang anhaltenden Schutz vor dem jeweiligen Pathogen durch Ausbildung eines "immunologischen Gedächtnisses" bietet, erkennen die Zellen des angeborenen Immunsystems Pathogene anhand konservierter Pathogen-assoziierter molekularer Muster (pathogenassociated molecular patterns = PAMPs) mit Keimzell-kodierten Rezeptoren und reagieren sofort. Zu den für verschiedene Zelltypen unterschiedlichen Reaktionen gehören die Sekretion von Cytokinen (z.B. IL-1, IL-6, TNF-$\alpha$) und Chemokinen (z.B. IL-8/CXCL8, MIP-1$\alpha$/$\beta$, MCP-1), Aktivierung von Effektormechanismen (Phagozytose, respiratorische Entladung, Freisetzung bakterizider Substanzen oder lytischer Granula), Expression von kostimulatorischen Molekülen (CD80, CD86) sowie verstärkter Expression von MHC-Molekülen. Dadurch werden zum Einen Effektorzellen rekrutiert und aktiviert, die das eingedrungene Pathogen eliminieren können, und zum Anderen erhalten die Zellen des adaptiven Immunsystems die für ihre Aktivierung notwendigen Signale.

Zur Erzeugung einer verbesserten Immunantwort wurden CpG-Oligonukleotide (CpG-ODN) als neue Klasse von immunmodulatorischen Molekülen eingesetzt. Solche nicht methylierte CG-Motive kommen in bakterieller DNA vor und stellen für das Immunsystem ein "danger signal" dar. Als "pathogen associated molecular pattern" (PAMP) bewirken sie vor allem die unspezifische Aktivierung des angeborenen Immunsystems (Krieg, Nat. Med 2003, 9: 831-835).

CpG-ODN induzieren über die Zytokine Interleukin-12, Interferon-gamma und Tumomekrosefaktor-alpha eine $T_H$1-betonte Immunantwort.

Immunstimulatorische Nukleinsäuresequenzen (ISS), die die benannten CpG-ODN tragen, sind nur einige Basen lang und wirken nicht über die Expression von auf ihnen kodierten Proteinen.

Bei den ISS handelt es sich um kovalent geschlossene Nukleinsäuremoleküle. Sie bestehen aus Oligonukleotiden, deren Basen partiell mit sich selbst paaren können und einer oder zwei Haarnadelschleifen (Loop), die 30 Basen umfassen und mehrere CG-Motive enthalten und im Weiteren als Trägermoleküle bezeichnet werden.

Die starke Stimulation der zellulären Immunantwort ermöglicht eine Einflussnahme auf Regelkreisläufe, die ohne Eingriff nicht zu einer für den Patienten befriedigenden Immunaktivität führen.

Die Modifikation von CpG-ODN mit Phosphorothioat-Rückgrat, wie sie zur Stabilisierung von "CpG-DNA" eingesetzt wird, weist einige gravierende Nachteile auf. Hierzu gehören insbesondere die beobachtete Toxizität [Heikenwalder 2004, Levin 1999] sowie unspezifische Bindung an Proteine [Brown 1994].

Aus diesem Grund wurde eine neue Klasse kovalent geschlossener immunstimulatorischer DNA entwickelt (EP 1196178). Diese DNA-Moleküle bestehen aus zwei chemisch synthetisierten DNA-Molekülen, die einen selbstkomplementären Bereich am 5'- und am 3'-Ende mit palindromischen, überlappenden Enden aufweisen, so dass durch Ligation der beiden DNA-Moleküle ein kovalent geschlossenes Molekül entsteht. Diese DNA-Moleküle mit CG-Motiven im nicht-komplementären Bereich zeigen ähnliche Aktivität wie CpG-ODN (verstärkte Expression der Oberflächenmoleküle CD80, CD40, MHC auf B-Zellen und Sekretion von IL-6, IFN-$\gamma$, IFN-$\alpha$, IL-12, TNF$\alpha$ durch PBMC), weisen aber im Vergleich zu CpG-ODN mit Phosphorothioat-Rückgrat Unterschiede im Expressionsmuster und eine deutlich geringere Toxizität in Mäusen auf. Diese immunstimulatorische DNA des Standes der Technik weist bezüglich der Modulation der Aktivität des menschlichen oder tierischen Immunsystems aber einige Nachteile auf. Es ist nicht immer möglich, die Aktivität des menschlichen oder tierischen Immunsystems in der gewünschten Stärke zu modulieren, insbesondere zu aktivieren.

Ausgehend von diesem Stand der Technik ist es die Aufgabe der vorliegenden Erfindung geeignete immunstimulatorische DNA-Moleküle, die zur Auslösung einer verbesserten Immunantwort fähig sind, sowie ein Verfahren zu ihrer Herstellung sowie Impfstoffe bereitzustellen, die diese immunstimulatorischen DNA-Moleküle enthalten.

Immunstimmulation heißt im Zusammenhang dieser Erfindung, dass die Mittler- und Effektorzellen des Immunsystems, also vor allem die zur Zeit bekannten Thymozyten mit Helferfunktion und die cytotoxischen Thymozyten, B-Zellen und sog. NK (natural killer)-Zellen, Makrophagen und Monozyten sowie dendritische Zellen und ihre Vorläufer, sowie bisher in ihrer Funktion nicht aufgeklärte Zellpopulationen mit Funktionen innerhalb des Immunsystems, durch die Verwendung von Nukleinsäuremolekülen zur Proliferation, Migration, Differenzierung oder Aktivität angeregt werden. Immunmodulation heißt, dass neben einer generellen Stimulation im oben definierten Sinne auch die Art oder der Charakter einer Immunreaktion beeinflusst wird, sei es, dass eine im Entstehen oder der Reifung begriffene Immunreaktion davon betroffen ist, sei es, dass eine schon etablierte Reaktion in ihrem Charakter verändert wird.

Die vorliegende Erfindung löst die Aufgabe dadurch, dass sie ein multimeres nicht-kodierendes Nukleinsäuremolekül zur Verfügung stellt. Das multimere Molekül ist herstellbar durch ein Verfahren, was die folgenden Schritte umfasst:

- Bereitstellung einer 5-phosphorylierten Oligodesoxyribonukleinsäuresequenz in Wasser,
- Lyophylisierung bis ein trockener Rückstand erhalten wird und anschließend Resuspension in einer Pufferlösung,
- Zugabe einer T4-DNAligase, wodurch ein Reaktionsgemisch gebildet wird und
- Inkubation des Reaktionsgemisches bei 37˚C für mindestens 30 Minuten.

Es war völlig überraschend, dass die Abfolge der genannten Schritte zu einem multimeren Molekül führt welches besser als die Moleküle des Standes zur Modulation der Aktivität des menschlichen oder tierischen Immunsystems verwendet werden können. Ein multimeres Molekül im Sinne der Erfindung ist ein im wesentlichen Desoxyribonukleinsäuremolekül wobei das Nukleinsäuremolkül bevorzugt eine Länge von mindestens 100 Nukleotiden aufweist, bevorzugt 200, besonders bevorzugt mehr als 300. Während in der EP 1 196 178 Moleküle offenbart werden, die mit ihrer Stamm-Schleifen-Struktur als Monomer bezeichnet werden können, werden durch das erfindungsgemäße Verfahren Moleküle bereitgestellt, bei denen mehrere dieser Stamm-Schleifen-Monomerstrukturen zu multimeren oder oligomeren Strukturen assemblieren. Die erhaltenen Assemblate sind überraschend effektiv bei der Modulation des Immunsystems. Überraschend war weiterhin, dass die an sich einfache und labortypischen oben genannten Verfahrensschritte zu der Ausbildung zur effektiver Strukturen führen. Im Vergleich zu den Strukturen gem. EP 1 196 178 stellen die erfindungsgemäßen multimeren Moleküle früher molekulare Komplexe dar.

In einer bevorzugten Ausführungsform der Erfindung ist das Oligomere bzw. Multimere Assemblat, d. h. das erfindungsgemäße Molekül, dadurch gekennzeichnet, dass die Oligodesoxyribonukleinsäuresequenz die folgenden Sequenzen umfasst

a) GTTCCTGGAG ACGTTCTTAG GAACGTTCTC CTTGACGTTG GAGAGAAC oder
b) ACCTTCCTTG TACTAACGTT GCCTCAAGGA AGGTTGATCT TCATAACGTT GCCTAGATCA, oder
c) eine Oligodesoxyribonukleinsäuresequenz der Basenfolge AACG TTCTTCGGGG CGTT umfasst,
d) und wobei die Oligodesoxyribonukleinsäuresequenz eine Länge von 40 bis 1.600 Nukleotide aufweist.

Die multimeren Assemlate im Sinne der Erfindung, die die bevorzugten Sequenzen aufweisen eignen sich besonders gut im Haustier und im humanen Bereich, um die Aktivität des Immunsystems zu stimulieren.

Besonders bevorzugt ist es, wenn die Basenfolge gemäß Merkmal c) in der Sequenz CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC enthalten ist. Überraschender Weise führt das Vorhandensein dieser Sequenz zu einer besonders guten Wirkung des Moleküls, wobei unter Wirkung im Sinne der Erfindung die Aktivierung des Immunsystems verstanden werden soll.

In einer weiteren bevorzugten Ausführungsform der Erfindung ist vorgesehen, dass das Molekül eine teilweise einsträngige, kovalent geschlossene Kette von Desoxyribonukleosidresten umfasst. Innerhalb der assemblierten oligomeren bzw. polymeren Struktur des Moleküls ist die teilweise einsträngig kovalent geschlossene Kette von Desoxyribonukleosidresten dafür verantwortlich, dass das Molekül dem Zielorganismus, in dem es eingebracht wird über einen längeren Zeitraum effektiv wirkt.

In einer weiteren bevorzugten Ausführungsform der Erfindung ist vorgesehen, dass das Molekül die Basenfolge $N^1N^2CGN^3N^4$ umfasst, wobei $N^1N^2$ ein Element aus der Gruppe GT, GG, GA, AT oder AA, $N^3N^4$ ein Element aus der Gruppe CT oder TT, sowie C Desoxycytosin, G Desoxyguanosin, A Desoxyadenosin und T Desoxythymidin ist.

In einer ganz besonders bevorzugten Ausführungsform ist vorgesehen, dass die Basenfolge $N^1N^2CGN^3N^4$ in dem einsträngigen Bereich der geschlossenen Kette von Desoxyribonukleosidresten positioniert ist. Insbesondere diese bevorzugten Moleküle zeigen eine sehr effektive Wirkung bei der Stimulierung des Immunsystems.

Die Erfindung betrifft auch das Erfindungsmolekül, welches mit mehreren Substituenten kovalent verbunden ist, wobei die Substituenten bevorzugt Peptide, Proteine, Sacharide, Antigene strukturen, DNA- und/oder RNA-Moleküle sind.

Die Erfindung betrifft auch ein Kombinationsmittel, welches mindestens ein erfindungsgemäßes Molekül und ein Chemotherapeutikum umfasst. Es war überraschend, dass die überraschend hohe Stimulation des Immunsystems durch das erfindungsgemäße Molekül noch einmal verbessert werden kann, wenn das erfindungsgemäße Mittel mit bekannten Chemotherapeutika kombiniert wird und das Kombinationsmittel gegen Tumoren eingesetzt wird. Das Kombinationsmittel im Sinne der Erfindung kann auch als Kit vorliegen, in welchem das erfindungsgemäße Molekül und das Chemotherapeutikum gemäß des Standes der Technik getrennt vorliegen. So können in bevorzugten Ausführungsformen die mindestens zwei Bestandteile des Kits zeitgleich oder zeitversetzt appliziert werden. Beispielsweise kann die Gabe des erfindungsgemäßen Kombinationsmittels das Immunsystem so aktivieren, dass eine nachfolgende Applikation eines Chemotherapeutikums seine Wirkung besonders effektiv entfalten kann. Selbstverständlich ist es aber auch möglich, dass zunächst das Chemotherapeutikum appliziert und anschließend zeitlich versetzt davon das erfindungsgemäße Molekül in den humanen oder tierischen Organismus gegeben wird. Bei bestimmten Tumoren ist die zeitgleiche Gabe von erfindungsgemäßen Molekül und Chemotherapeutikum bevorzugt.

In einer bevorzugten Ausführungsform der Erfindung ist das Chemotherapeutikum ausgewählt aus der Gruppe umfassend Antikörper, Alkylantien, Platinanaloga, Interkalentien, Antibiotika, Mitosehemmer, Taxane, Topoisomerasehemmer, Antimetabolite und/oder L-Asparaginase, Hydroxycarbamid, Mitotane und/oder Amanitine.

In einer bevorzugten Ausführungsform der Erfindung sind die Alkylantien ausgewählt aus der Gruppe umfassend

- Stickstoff-Lost-Derivate, insbesondere
- Cyclophosphamid,
- Ifosfamid,
- Trofosfamid,
- Melphalan und/oder
- Chlorambucil
- Akylsulfonate, insbesondere
- Busulfan und/oder
- Treosulfan
- Nitrosoharnstoffe, insbesondere
- Carmustin,
- Lomustin,
- Nimustin,
- Estramustin und/oder
- Streptozotocin
- Procarbazin und Dacarbazin,
- Temozolomid und/oder
- Thiotepa.

Die Alkylantien wirken besonders gut auf Tumoren, wodurch sie deren Wachstum inhibieren.

In einer bevorzugten Ausführungsform der Erfindung sind die Platinanaloga ausgewählt sind aus der Gruppe umfassend:

- Cisplatin,
- Carboplatin und/oder
- Oxaliplatin.

In einer weiteren bevorzugten Ausführungsform der Erfindung ist vorgesehen, dass die Interkalentien ausgewählt sind aus der Gruppe umfassend:

- Anthracycline, insbesondere
- Doxorubicin (Adriamycin),
- Daunorubicin,
- Epirubicin und/oder
- Idarubicin,
- Mitoxantron,
- Amsacrin und/oder
- Doxifluridin

In einer weiteren bevorzugten Ausführungsform der Erfindung ist vorgesehen, dass die Antibiotika ausgewählt sind aus der Gruppe umfassend:

- Bleomycin,
- Actinomycin D (Dactinomycin) und/oder
- Mitomycin

Weiterhin kann es in einer anderen bevorzugten Ausführungsform der Erfindung vorteilhaft sein, dass die Mitosehemmer ausgewählt sind aus der Gruppe umfassend:

- Alkaloide der Vinca rosea, insbesondere
- Vinorelbin,
- Vincristin (Oncovin),

- Vinblastin und/oder
- Vindesin

In einer weiter besonders bevorzugten Ausführungsform der Erfindung sind die Taxane ausgewählt sind aus der Gruppe umfassend:

- Paclitaxel und/oder
- Docetaxel.

Weiterhin kann es bevorzugt sein, dass die Topoisomerasehemmer ausgewählt sind aus der Gruppe umfassend:

- Topoisomerase-I-Inhibitoren, insbesondere
- Camptothecin,
- Topotecan und/oder
- Irinotecan und/oder
- Topoisomerase-II-Inhibitoren, insbesondere
- Etoposid
- Teniposid.

Weiterhin ist es bevorzugt, dass in einer besonderen Ausführungsform der Erfindung die Antimetabolite ausgewählt sind aus der Gruppe umfassend:

- Folsäureantagonist, insbesondere
- Methotrexat,
- Pyrimidianaloga, insbesondere
- 5-Fluorouracil,
- Capecitabin,
- Cytosinarabinosid (Cytarabin) und/oder
- Gemcitabin,
- Purinanaloga, insbesondere
- 6-Thioguanin,
- Pentostatin,
- Azathioprin,
- 6-Mercaptopurin,
- Fludarabin und/oder
- Cladribin.

Die Erfindung betrifft auch einen Kit, der das erfindungsgemäße Molekül und das Chemotherapeutikum umfasst, gegebenenfalls mit einer Information zum Kombinieren der Inhalte des Kits. Die Erfindung betrifft aber auch - wie bereits ausgeführt - ein pharmazeutisches Mittel, welches das erfindungsgemäße Molekül oder das Kombinationsmittel gegebenenfalls mit einem pharmazeutisch verträglichen Träger umfasst.

Die Erfindung betrifft weiterhin die Verwendung des Moleküls, des Kombinationsmittel oder des pharmazeutischen Mittels zur Herstellung eines Mittels zur Modulation eines menschlichen oder tierischen Immunsystems oder zur Modulation der Aktivität dieses Immunsystems. Unter Modulation des menschlichen oder tierischen Immunsystems wird jeder Einfluss auf das Immunsystem verstanden, der dazu führt, dass das Immunsystem auf Tumoren bzw. auf Krebs insbesondere inhibierend wirkt. Die Modulation der Aktivität des Immunsystems kann hierzu synonym verstanden werden oder sie beschreibt, die den Fachmann bekannten Aktivitäten des Immunsystems, die gegen Tumoren gerichtet sind und die durch die erfindungsgemäßen Mittel in Ihre Aktivität überraschend gesteigert werden. Die Modulation ist daher insbesondere eine Stimulierung oder Besteigerung von Wirkungen des Immunsystems bzw. des Immunsystems selbst. So können die erfindungsgemäßen Mittel in einer bevorzugten Ausführungsform dazu verwendet werden, die T-Zell vermittelte Immunantwort aber auch die T-Zell unabhängige Immunantwort zu stimulieren. Dieser Vorgang kann in einer bevorzugten Ausführungsform der Erfindung eine Proliferation von B-Zellen umfassen oder eine B-Zell-Aktivierung.

In einer ganz besonders bevorzugten Ausführungsform kommt es bei der Modulation der Aktivität des Immunsystems zu einer Stimulierung dergestalt, dass Zytokine ausgeschüttet bzw. verstärkt ausgeschüttet werden. Es kann besonders bevorzugt sein, wenn das erfindungsgemäße Molekül bzw. das Kombinationsmittel gemäß der Erfindung als Adjuvanz in der therapeutischen oder prophylaktischen Vakzinierung eingesetzt werden. Besonders wirksam können die erfindungsgemäßen Mittel zur Behandlung einer Zellwachstumsstörung eingesetzt werden, wobei in einer bevorzugten Ausführungsform die Zellwachstumsstörung eine Tumorerkrankung ist. Bevorzugt handelt es sich bei der Tumorerkrankung um eine Krankheit die ausgewählt ist aus der Gruppe umfassend Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nichtkleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehimmetastasen, Lungen-metastasen, Lebermetastasen, Knochenmetastasen, pleurale und perikardiale Metastasen und maligne Aszites.

Im folgenden soll die Erfindung an Hand von Beispielen näher erläutert werden oder auch ohne auf diese Beispiele beschränkt zu sein.

Die für die einzelnen Methoden benötigten Geräte, Materialien und Lösungen sind unter der jeweiligen Methode aufgeführt. Die folgende Tabelle enthält eine Auflistung von Geräten und Materialien, die verwendet wurden und nicht einer spezifischen Methode zuzuordnen sind sowie der für die Herstellung der Lösungen benötigten Chemikalien und Lösungen:

**Tabelle: Verwendete Geräte, Materialien, Chemikalien und Lösungen**

| **Name** | **Hersteller/Lieferant** |
|---|---|
| *Geräte* | |
| Biofuge Pico | Hereaus |
| Digital pH-Meter | Knick |
| Heizblock HLC Progress | Sigma |
| Magnetrührer IKA MAG REO | Janke u. Kunkel |
| Oberschatenwaage | Sartorius |
| Pipettboy Accu Jet | Brand |
| Vortex Genie 2 | Scientific Instruments |
| *Materialien* | |
| Einwegpipetten 5, 10, 25 mL | Corning B.V. Life Sciences |
| Flaschenaufsatzfilter 0,2 $\mu$m | Nunc |
| Pipettenspitzen versch. Grössen | Roth |
| Reaktionsgefäße 0,5 mL, 1,5 mL | Eppendorf |
| Spritzen (steril, 10 ml, 100 ml) | Becton Dickinson |
| Spritzenvorsatzfilter 0,45/0,2 $\mu$m | Schleicher & Schüll |

(fortgesetzt)

| *Chemikalien, Lösunge n* | |
|---|---|
| APS | Life Technologies |
| ATP | Sigma |
| Blasticidn S (10 mg/mL) | Invivogen |
| Borsäure | Roth |
| BSA | Sigma |
| Coomassie Brillant Blue R-250 | Serva |
| DMEM-Medium | BioWhittaker |
| Ethanol absolut | J.T. Baker |
| Ethidiumbromid | Sigma |
| FBS | BioWhittaker |
| $MgCl_2$ | Roth |
| $Na_2EDTA \cdot 2\ H_20$ | Roth |
| NaCl | Roth |
| $NaN_3$ | Merck |
| Natriumacetat (wasserfrei) | Roth |
| PBS ohne Ca und Mg | BioWhittaker |
| Penicillin/Streptomycin | BioWhittaker |
| RPMI-Medium | BioWhittaker |
| Saccharose | Roth |
| Salzsäure (HCl) 25% | Roth |
| Schwefelsäure (H2SO4) | Roth |
| TAE-Puffer (50 x) | AppliChem |
| TEMED | Roth |
| Tris Ultra | Roth |
| Trypsin-Lösung | BioWhittaker |
| Tween 20 | Roth |

**Verwendete ODN und dSLIM, Sequenzauswahl mit Hilfe von mfold**

Die Sequenzen für Herstellung von Modellmolekülen zur Untersuchung der Struktur von dSLIM wurden zum Großteil mit Hilfe der im WWW verfügbaren Software "mfold" erstellt (http://www.bioinfo.rpi.edu/applications/mfold). Dabei handelt es sich um ein Programm zur Sekundärstrukturvorhersage von Nukleinsäuren basierend auf thermodynamischen Daten [Zuker 2003]. Die Sequenzen der 58 Nukleotide langen DNA, im folgenden als ODN bezeichnet, zur Synthese von dSLIM wurden als lineare DNA in "DNA mfold" eingegeben und die Standardeinstellungen sowie folgende Parameter verwendet: Temperatur 37°C, Ionenkonzentration 150 mM $Na^+$ und 0,5 mM $Mg^{2+}$, Oligomer-Korrektur.

Als Grundlage für die Modellmoleküle zur Untersuchung der Ausbildung von G-Strukturen diente die Sequenz von dSLIM-30L1. Diese wurde zunächst so verändert, dass keine aufeinander folgenden Guanin-Reste mehr vorhanden waren, dabei wurde der GC-Anteil des komplementären Bereiches (nachfolgend als "Stamm" bezeichnet) beibehalten. Die Sequenz des nicht-komlementären Bereiches (nachfolgend als "Loop" bezeichnet) wurde so verändert, dass dort möglichst keine Watson-Crick-Basenpaarungen möglich waren und keine aufeinander folgenden Guanin-Reste vorhanden waren. Durch diese Veränderung des Loops wurden im Vergleich zu 30L1 die innerhalb dieses Bereiches liegenden die CG-Motive verändert. Das so konstruierte Modell-ODN wird nachfolgend als KG bezeichnet. Das ODN GL entspricht KG mit einer poly(G)-Sequenz im Loop, die jedoch im Gegensatz zu der in 30L1 vorkommenden nicht innerhalb der CG-Motive liegt. Das ODN MS entspricht dem Ausgangsmolekül 30L1, weist aber im Stamm zusätzliche Guanin-Reste auf. Die ODN GS, GLS und ML enthalten Kombinationen von Stamm und Loop der oben beschriebenen ODN.

Als Kontrollmoleküle für die Abhängigkeit der beobachteten Wirkung von CG-Motiven, dienten die Moleküle no30L1 und noGL, in denen jeweils CG durch TG ersetzt war.

Das Molekül dSLIM-60L1 diente als Modellmolekül eines "dimeren" dSLIM-30L1, wie es durch Concatemerisierung entstehen kann. Es besteht aus zwei teilweise komplementären, jedoch nicht-selbstkomplementären ODN (60L1vonw u. 60L1rev), die nach Hybridisierung einen 5'-Überhang aufweisen, an den ein weiteres ODN (30L1-AGGG) mit entsprechendem 3'-Überhang und selbstkomplementären 5' und 3'-Bereich ligiert werden kann. Die Sequenzen der Loops dieser 3 ODN entsprechen dabei denen von 30L1.

Fraktionen von dSLIM-30L1, die durch Separation einer großen Menge dSLIM-30L1 durch Auftrennung mittels kontinuierlichem NaCl-Gradienten in der HPLC erhalten wurden, wurden von Melanie Rothe (Mologen AG) zur Verfügung gestellt.

Die in der Arbeit verwendeten ODN wurden von der Firma TIB-Molbiol (Berlin) mit 5'-Phosphorylierung (Ausnahmen: M362, 2006) gelöst in $H_20$ in einer Konzentration von 3 g/L nach HPLC-Aufreinigung bezogen:

Tabelle: Verwendete ODN Phosphorothioat kleine Buchstaben)

| Name | Sequenz | Strukturelle Merkmale | Molekulargewicht (g/mol) |
|---|---|---|---|
| 30L1 | CCT AGG GGT TAC CAC CTT CAT TGG AAA ACG TTC TTC GGG GCG TTC TTA GGT GGT AAC C | $G_4$ in Stamm und Loop (S1, L1) | 17871 |
| KG | CTG CAG CTG TAG CAG CTT CAT TCC ATA TCG TTC TTC GTG TCG TTC TTA GCT GCT ACA G | keine poly(G)-Sequenzen, Loop ungepaart (S2, L2) | 17723 |
| ML | CCT AGG GGT TAC CAC CTT CAT TCC ATA TCG TTC TTC GTG TCG TTC TTA GGT GGT AAC C | Stamm von 30L1, Loop von KG (S1, L2) | 17723 |
| MS | CCT AGG GGT GGG GGC CTT CAT TGG AAA ACG TTC TTC GGG GCG TTC TTA GGC CCC CAC C | 30L1 mit zusätzlicher $G_5$ im Stamm (S3, L1) | 17874 |
| GL | CTG CAG CTG TAG CAG CTT CGG GGG GTA TCG TTC TTC GTG TCG TTC TTA GCT GCT ACA G | KG mit $G_6$ im Loop (S2, L3) | 17885 |
| GS | CCT AGG GGT GGG GGC CTT CAT TCC ATA TCG TTC TTC GTG TCG TTC TTA GGC CCC CAC C | KG mit Stamm von MS (S3, L2) | 17726 |
| GLS | CCT AGG GGT GGG GGC CTT CGG GGG GTA TCG TTC TTC GTG TCG TTC TTA GGC CCC CAC C | Stamm von MS, loop von GL (S3, L3) | 17888 |
| no30L1 | CCT AGG GGT TAC CAC CTT CAT TGG AAA ATG TTC TTT GGG GTG TTC TTA GGT GGT AAC C | 30L1 ohne CG-Motive (TG statt CG) | 17871 |
| noGL | CTG CAG CTG TAG CAG CTT TGG GGG GTA TTG TTC TTTC GTG TTG TTC TTA GCT GCT ACA G | GL ohne CG-Motive (TG statt CG) | 17900 |
| 60L1 vorw | CCT AGG GGT TAC CAC CTT CAT TGG AAA ACG TTC TTC GGG GCG TTC TTT CCC CAA TGG TGG AA | nicht selbstkomplementär, Loop von 30L1 | 19147 |
| 60L1 rev | CCC CTT CCA CCA TTG GGG ATC ATT GGA AAA CGT TCT TCG GGG CGT TCT TAG GTG GTA ACC CC | nicht selbstkomplementär, Loop von 30L1 | 19108 |
| 30L1-AGGG | GGG GTT ACC ACC TTC ATT GGA AAA CGT TCT TCG GGG GCT TCT TAG GTG GTA A | selbstkomplementär, 3'-Überhang | 16177 |
| M362 | tcg tcg tcg ttc gaa cga cgt tga t | CpG-C Phosphorothioat-rückgrat | 7640 |
| 2006 | tcg tcg ttt tgt cgt ttt gtc gtt | CpGB Phosphorothioat-rückgrat | 7303 |

Guanin ist wegen der Orientierung seiner vier H-Brücken-Bindungsstellen in der Lage, durch Guanin-Guanin-Basenpaarung ein zyklisches Basenquartett mit 8 H-Brücken auszubilden (G-Quartett). Eine DNA-Sequenz, die mehrere aufeinander folgende Guanin-Nukleotide enthält ist daher in der Lage, eine tetramere helikale Struktur auszubilden, bei der die Guanin-Basen eine starke Planarität mit besonderer Stapelwechselwirkung aufweisen. Je nach Lage, Anzahl und Verteilung von Guanin-Nukleotide in der Sequenz können verschiedene G-Strukturen ausgebildet werden, die in drei Gruppen eingeteilt werden können: G2'-DNA (bimolekulare parallele oder antiparallele Tetraplexe), G4'-DNA (unimolekulare antiparallele Tetraplexe) oder G4-DNA (tetramolekulare parallele Tetraplexe). G4-DNA ist in der Lage selbstassemblierende Nanostrukturen, so genannte G-wire, auszubilden. Die Bildung und die zum Teil sehr große Stabilität von G-Strukturen ist neben der Anzahl der zur Verfügung stehenden Guanin-Reste und deren Anordnung und umgebenden Watson-Crick-Basenpaarung, abhängig von der Temperatur, der DNA-Konzentration und der Anwesenheit verschiedener Kationen (z.B. $Na^+$, $K^+$, $Mg^{2+}$, $Ca^{2+}$). G4-Strukturen wurden beispielsweise in Telomersequenzen, Immunglobulin-Schalterregionen und HIV-DNA identifiziert [Sen 1992, Marsh 1994,1995].

Es wurden im folgenden verschiedene monomere und multimere Moleküle hergestellt, die Kombinationen verschiedener Loop- und Stammsequenzen enthielten. Es wurden jeweils drei Loop- und drei Stammsequenzen verwendet, die lange, kurze oder keine poly(G)-Motive enthielten, wodurch die Fähigkeit zur Ausbildung von G-Strukturen beeinflußt wurde. Stabile G-Strukturen können sich unter den entsprechenden Bedingungen in kurzen ODN (12-mer) mit nur vier aufeinanderfolgenden Guaninbasen ausbilden. Bei längeren ODN sind längere Motive notwendig, da die Wahrscheinlichkeit für die Ausbildung von stabilen G-Strukturen mit dem Anteil der beteiligten Guaninbasen an der Gesamtsequenz positiv korreliert [Sen 1992].

Die Moleküle die zusätzliche lange poly(G)-Motive aufwiesen zeigen eine verstärkte Ausbildung von multimeren Molekülen. Auch die Elutionsprofile dieser Moleküle unterschieden sich von denen, die keine langen poly(G)-Motive aufwiesen durch das Auftreten von zwei Gipfeln, von denen der Gipfel bei höherem Elutionsvolumen, der im oberen Bereich der Agarosegele auftretenden DNA-Verteilung entsprach. Die auffälligste Veränderung war bei GL, dem Molekül, dessen poly(G)-Motive im Loop lokalisiert sind, zu beobachten. Hier waren im Elutionsprofil zwei deutlich getrennte Gipfel zu unterscheiden, wobei der Gipfel bei höherem Elutionsvolumen eine größere Intensität aufwies als der bei kleinerem Elutionsvolumen. Bei GL war dann auch der Anteil der diffus im Gel verteilten DNA oberhalb der monomeren Bande vergleichsweise groß und reichte in seiner Verteilung bis zur Tasche des Gels. Diese Beobachtungen lassen darauf schließen, dass ein relativ großer Anteil der DNA in mutimeren Komplexen vorliegt. Die Verteilung der Gipfel im Elutionsprofil der HPLC und die Verteilung der DNA nach Agaraosegel-Elektrophorese ähnelten sich in den Konstrukten, die poly(G)-Motive im Stamm enthalten (GS, MS). Diese zeigten im Elutionsprofil einen niedrigen zweiten Gipfel bei höheren Elutionsvolumina, und eine schwach sichtbare, indiskrete Verteilung von DNA oberhalb der monomeren Bande im Agarosegel. Das bei den Molekülen mit poly(G)-Motiven in Loop und Stamm (GLS) beobachtete Laufverhalten in HPLC und im Agarosegel lag für das Gipfelmuster (HPLC) und die DNA-Verteilung (Agarosegel) zwischen denen von GL und GS und MS. Im Elutionsprofil wurden zwei separierte Gipfel mit gleicher Höhe beobachtet und der Anteil der im oberen Bereich des Gels verteilten DNA war etwas stärker als bei den Molekülen mit poly(G)-Motiven im Stamm (GS und MS), jedoch schwächer als bei GL. Demnach ist die Ausbildung von multimeren Molekülen besonders begünstigt zu sein, wenn sich die poly(G)-Motive im Loop befinden, wie es bei GL der Fall ist. Der Grund hierfür ist die fehlende Konkurrenz zwischen der Ausbildung von Watson-Crick-Basenpaaren und der Ausbildung von G-Strukturen im einzelsträngigen Loop. Diese Voraussetzung ist auch im Molekül GLS gegeben. Hier befinden sich jedoch sowohl im Stamm wie im Loop poly(G)-Motive als mögliche Interaktionspartner. Es können sich also intramolekulare und daher weitestgehend konzentrationsunabhängig G2-Strukturen ausbilden. Diese stellen somit eine Konkurrenz zur intermolekularen Ausbildung von G-Strukturen dar, so dass die Ausbildung von höhermolekularen Komplexen bei GLS im Vergleich zu GL verringert ist. Die geringste Tendenz zur Ausbildung von höhermolekularen Komplexen wurde, bei den Molekülen mit poly(G)-Motiven im Stamm beobachtet, obwohl die poly(G)-Motive hier länger waren als in GL.

Bei dem monomeren Molekül 30L1, das ebenfalls, ähnlich wie GLS, poly(G)-Motive in Stamm und Loop aufweist, wurden die oben beschriebenen Charakteristika bei gleichen Mengen an Ausgangsmaterial nicht beobachtet. Dies könnte darauf zurückzuführen sein, dass zum einen die poly(G)-Motiven in diesem Molekül kürzer sind als in den multimeren Molekülen. Zum anderen ergibt die Strukturvorhersage von "DNA mfold" eine größere Tendenz zur Ausbildung von Watson-Crick-Basenpaaren im Loop, was die Wahrscheinlichkeit für die Ausbildung von G-Strukturen weiter verringern sollte. Allerdings zeigt die Fraktion F4, die aus einer großen Menge 30L1 durch HPLC-Trennung gewonnen wurde, ähnlich wie die multimeren Moleküle mit langen poly(G)-Motiven, unter Polyacralamidgel-Elektrophorese auch die beschriebene diffuse DNA-Verteilung im oberen Bereich des Gels und in den Geltaschen verbliebene DNA (s. Abb 3.1.9). Offensichtlich ist bei 30L1 der Anteil an höhermolekularen Komplexen aus G-Strukturen so gering, dass sie nur bei größeren Ausgangsmengen detektiert werden können.

Im Gegensatz dazu verschwinden bei GL und GLS, die poly(G)-Motive im Loop aufweisen, die DNA-Anteile in der Tasche nicht vollständig und es wird ein regelmäßiges, zusätzliches Bandenmuster erkennbar. Dabei handelt es sich vermutlich um Dimere oder Tetramere oder aus in den Aggregaten verbliebenen, nicht abgebauten ODN, die durch die Denaturierung aus den Aggregaten gelöst wurden und über Guanin-Guanin-Interaktion noch miteinander verbunden sind.

Dass die multimeren Moleküle mit langen poly(G)-Motiven tatsächlich durch G-Strukturen gebildet werden, zeigen die experimentellen Eigenschaften. Hierzu gehören neben dem Auftreten einer indiskreten DNA-Verteilung im oberen Gelbereich, das Auftreten von regelmäßigen Banden nach Denaturierung, wie es bei GL und GLS im Polyacrylamidgel beobachtet wurde.

Im Gegensatz zu der aus 30L1 isolierten Bande im oberen Geldrittel, waren die bei KG und ML beobachteten Banden nach Denaturierung nicht mehr erkennbar (s. Abb 3.1.6). Es handelt sich daher bei diesen um Dimere, die durch Basenpaarwechselwirkungen zwischen zwei monomeren Molekülen gebildet wurden und daher durch thermische Denaturierung leicht getrennt werden können. Dass diese bevorzugt bei KG und ML auftraten, könnte an deren gemeinsamer Loopsequenz liegen, die eine Interaktion zweier Moleküle begünstigt.

Durch HPLC-Fraktionierung einer großen Menge des Moleküls 30L1, wurden 4 Fraktionen erhalten, welche sehr unterschiedliches Laufverhalten im Gel zeigten. Durch Aufkonzentrieren der einzelnen Fraktionen konnten diese einzeln aufgelöst werden, so dass hier mehrere, vorher im Gel, nicht erkennbare Banden sichtbar wurden. Dabei entsprach das Laufverhalten der ersten Fraktion den beiden beobachteten monomeren Konformationen, das der zweiten Fraktion entsprach einer kontinuierlichen Mischung aus beiden Konformationen, wobei nach Denaturierung dieser Fraktion die offene Konformation überwog und das der dritten Fraktion entsprach hauptsächlich, wie oben beschrieben einem dimeren Molekül. Die vierte Fraktion ähnelte in ihrem Laufverhalten, mit ihren deutlich sichtbaren Anteilen an in der Geltasche verbliebener DNA, dem von MS.

Auffällig war die Verschiebung der einem Dimer entsprechenden Bande bei 30L1 und der 30L1-Fraktion 3 im Zellkulturmedium gegenüber ihrer in Wasser beobachteten Laufweite im Gel. Der Vergleich mit dem Coomassie-gefärbten Gel zeigt, dass die entsprechende Bande in Medium auf gleicher Höhe mit der unteren Proteinbande läuft. Daher kann angenommen werden, dass die Verschiebung der Bande durch Bindung des dimeren Moleküls an Proteine verursacht ist.

Das Laufverhalten der der Monomerkonformationen entsprechenden Banden war durch die Zugabe von Medium kaum verändert, lediglich bei GL wurde eine Zunahme der Intensität der der offenen Konformation entsprechenden Bande beobachtet. Nach fünfstündiger Inkubation bei 37˚C konnte bei KG und 30L1 eine diffuse Verteilung der DNA zwischen den beiden zuvor beobachteten Banden beobachtet werden, dabei nahm bei KG die Intensität der der offenen Konformation entsprechenden Bande ab. Unter den gewählten Bedingungen tritt eine leichte Bevorzugung der offenen Konformation im Vergleich zur wässrigen Lösung auf. Nach 27 h Inkubation in Medium waren die beobachteten DNA Intensitäten bei ML und GL stark vermindert und auch bei KG war nur noch die untere Bande mit verringerter Intensität sichtbar, die geringste Abnahme der Intensität war dagegen bei 30L1 zu beobachten. Eine mögliche Erklärung hierfür wäre, dass die offenen Konformationen leichter degradiert werden als doppelsträngige Konformationen

Die immunstimulatorische Wirkung der verschiedenen monomeren und multimeren Moleküle sollte mit Hilfe der Bestimmung der Konzentration von IFN-gamma, IFN-alpha und IL-6 in den Überständen von PBMC aus humanen Blutspenden nach Stimulation mit diesen Molekülen untersucht werden.

Bei dem Testsystem zur Bestimmung der Aktivität der Moleküle mit Hilfe von PBMC aus humanen Blutspenden handelt es sich um sehr komplexes System, in dem die verschiedenen Zellen sich durch Interaktion und/oder Cytokinsekretion gegenseitig beeinflussen können, wodurch eine Zuordnung der beobachteten Antwort zu einem bestimmten Zelltyp oder Reaktionsweg schwer möglich ist. Zudem treten zwischen den Ergebnissen von einzelnen Spendern große Variationen auf, so dass eine größere Anzahl von unabhängigen Versuchen nötig ist. Der Vorteil dieses Testsystems liegt darin, dass es von allen möglichen etablierten *in vitro* Systemen der *in vivo* Situation am nächsten kommt.

Die Versuche zeigten, dass der verwendete Nachweis für die Aktivierung der Zellen relativ stark durch die Kultivierungsbedingungen der Zellen beeinflusst wird, da IL-8/CXCL8 auch als Reaktion auf Zellstress-induzierende Faktoren ausgeschüttet wird. Dies wird besonders deutlich an den Ergebnissen die mit Zellen gewonnen wurden, deren Medium nicht vor der Stimulation gewechselt wurde (s. Abb 3.3.1) und bei denen eine hohe Konzentration von IL-8/CXCL8 bereits in den unstimulierten Zellen nachgewiesen wurde. Das ermittelte Verhältnisse aus der Chemokin-Konzentration, die in unstimulierten Zellen gemessen wurde zur Chemokin-Konzentration, die in stimulierten Zellen nachgewiesen wurde, war dann mit 1,3 nur sehr gering im Vergleich zu Zellen, die einen Mediumwechsel erfahren hatten. Bei denen betrug das Verhältnis 2,3. Um möglichst aussagefähige Ergebnisse zu erhalten, ist es daher für diesen Test besonders wichtig, die Zellen unter möglichst Zellstress-freien Bedingungen zu kultivieren. Zellstress ist vermutlich auch der Grund, dafür, dass die Ergebnisse, die mit Zellen erhalten wurden, welche 24 h nach dem Aussäen stimuliert wurden nicht so eindeutig ausfielen, wie die mit konfluent gewachsenen Zellen. Die Umsetzung der Zellen und die damit verbundenen Zellkulturtechniken erzeugen hohen Zellstress.

In dem Versuch zur Abhängigkeit von der Stimulationsdauer (s. Abb 3.3.2) konnte beobachtet werden, dass bereits nach 6 h ein Unterschied in der ausgeschütteten Chemokin-Menge zwischen unstimulierten und stimulierten Zellen detektierbar war. Die Menge an IL-8/CXCL8 nahm zwischen 6 h und 24 h zu, dagegen war der beobachtete Anstieg von 24 h zu 48 nur noch gering.

In zwei Versuchen wurden die Aktivitäten der Molekülen in dem Testsystem untersucht. Dabei konnten Unterschiede zwischen den einzelnen eingesetzten Molekülen beobachtet werden (s. Abb 3.3.3).

Die höchste Sekretion von IL-8/CXCL8 wurde in den Versuchen durch Stimulation mit GL induziert, dabei war diese etwas größer als die durch das ODN 2006 hervorgerufene. Etwas geringer waren die nach Stimulation mit KG und 30L1 gemessenen IL-8/CXCL8-Mengen, wobei KG im Vergleich zu 30L1 eine etwas stärkere Aktivität zeigte. Diese beobachteten Unterschiede in der Aktivität der verschiedenen monomeren und multimeren Moleküle könnten durch die beobachteten strukturellen Verschiedenheiten begründet sein. So kann gezeigt werden, dass in KG und GL im Vergleich zu 30L1 eher eine offene Konformation des Loop-Bereiches mit den CG-Motiven vorliegt, so dass diese bevorzugt einzelsträngig vorliegen. Wie Rutz et. al. durch Surface-Plasmonen-Resonanz zeigen konnten, bindet einzelsträngige DNA mit größerer Affinität an TLR9 als doppelsträngige DNA [Rutz 2004], so dass die etwas größere Aktivität von KG und GL gegenüber 30L1 hierdurch begründet sein könnte. Allerdings sind die CG-Motive selbst in 30L1 und KG/GL verschieden, so dass die unterschiedliche Aktivität auch durch die verschiedene Affinität der unterschiedlichen CG-Motive zum Rezeptor begründet sein kann.

In den beiden Versuchen zeigte GL, das Molekül mit der größten Potenz zur Ausbildung von Aggregaten, die größte Aktivität der getesteten Moleküle, was möglicherweise, durch eine größere Quervernetzung der Rezeptoren durch die höhermolekularen Komplexe begründet sein könnte. Allerdings konnte eine verstärkte Aktivität von GL in den Versuchen, die mit PBMC durchgeführt wurden, nicht beobachtet werden. Es wäre aber möglich, dass auch andere in dem komplexeren System der PBMC vorhandene Mustererkennunsrezeptoren an der dort beobachtete immunmodulatorische Aktivität der monomeren und multimeren Molekülen beteiligt sind, die andere strukturelle Merkmale der Moleküle erkennen. So wäre es z.B. möglich, dass eine Erkennung auch durch andere Nukleinsäure-spezifische Rezeptoren wie TLR8 oder TLR7 erfolgt, wodurch eine kooperative oder modulierende Wirkung hervorgerufen wird.

Zusammenfassend kann gesagt werden, dass insbesondere die Anwesenheit von poly(G)-Motiven im Loop der monmeren Molekülen die Ausbildung von multimeren Komplexen begünstig. Diese Komplexe weisen zudem eine hohe Stabilität gegenüber thermischer Denaturierung auf.

Um Unterschiede der immunmodulatorischen Wirkung verschiedener Konstrukte zu identifizieren, wurden diese in Stimualtionsexperimenten mit PBMC eingesetzt.

Die Ergebnisse zeigen, dass die multimeren Moleküle eine besonders hohe Aktivität aufweisen.

DNA-Konzentrationsbestimmung

Die Konzentration der ODN und der monomeren DNA-Moleküle wurde durch Messung der Absorption bei 260 nm ($A_{260}$) bestimmt. Für die Absorption bei 260 nm sind die aromatischen Ringe der Nukleinsäurebasen verantwortlich, wobei sich die molaren Absorptionskoeffizienten ($\varepsilon$) der einzelnen Basen unterscheiden (A>G>T>C). Durch Chromophor-Chromophor-Wechselwirkungen weisen doppelsträngige Nukleinsäuren eine geringere Absorption auf als einzelsträngige Nukleinsäuren (Hypochromie-Effekt).

Für die Konzentrationsbestimmung wurde das entsprechende Volumen der zu bestimmende DNA-Lösung zunächst im Eppendorfgefäß mit TE-Puffer in einem Endvolumen von 300 μL verdünnt (ODN 1:200, dSLIM 1:100). Die DNA-Lösung wurde anschließend in eine Quarzküvette überführt und die Absorption bei 260 nm im Photometer gegen TE-Puffer gemessen. Die Berechnung der Konzentration aus der gemessenen Absorption erfolgte nach der Gleichung:

$$c\ (\mu g/mL) = A_{260} \times \text{Verdünnungsfaktor} \times \text{Umrechnungsfaktor}$$

Der Umrechnungsfaktor ist ein Näherungswert und wird auf 50 μg/mL für doppelsträngige DNA und 33 μg/mL für einzelsträngige DNA geschätzt. Wegen des Anteils an doppelsträngigen Bereichen in ODN und der monomeren DNA-Moleküle wird ein standardisierter Umrechnungsfaktor 50 μg/mL verwendet. Es wurden jeweils Doppelbestimmungen mit separaten Verdünnungen durchgeführt, aus denen der Mittelwert berechnet wurde.

Herstellung der monomeren DNA-Moleküle

Für die Herstellung der monomeren DNA-Moleküle wurden sterile (Einweg)materialien und frische oder bei -20°C gelagerte sterilfiltrierte Puffer verwendet, um eine Kontamination mit Bakterien zu vermeiden, da bereits geringe Mengen an Endotoxinen die Stimulationsergebnisse verfälschen können.

Der erste Schritt bei der Herstellung der monomeren DNA-Molekülen ist die Ligation zweier ODN mit Hilfe der T4 DNA Ligase. T4 DNA Ligase katalysiert die Bildung einer Phosphodiesterbindung zwischen 5'-Phosphat- und 3'-OH-Enden in doppelsträngiger DNA oder RNA mit stumpfen oder kohäsiven Enden. Daneben ist sie in der Lage Einzelstrangbrüche in doppelsträngiger DNA, RNA oder DNA:RNA-Hybriden zu reparieren.

Die Ligation zweier ODN erfolgt jeweils zwischen dem 5'-Phosphat-Überhang des einen mit dem 3'-Ende des anderen ODN, so dass ein zirkuläres Molekül entsteht. Bei der Reaktion Die Reaktion entstehen ebenfalls die erfindungsgemäßen multimeren DNA-Moleküle.

Die ODN-Ausgangsmenge für die Herstellung der Moleküle betrug 500-1000 μg. Für die Ligation wurden die ODN in einer Konzentration von 0,55 g/L in 1 x Ligase-Puffer eingesetzt. Dazu wurden sie mit Aqua Spüllösung verdünnt und die entsprechende Menge 10 x Ligase-Puffer zugegeben. Nach gründlichem Mischen wurde so viel T4 DNA Ligase zugegeben, dass ein Verhältnis von 0,01 U/μg DNA vorlag. Der Ligationsansatz wurde im Wasserbad bei 37˚C für 15-24 h inkubiert.

Der T7-Verdau dient dem Abbau nicht ligierter ODN. T7 DNA Polymerase ist eine Template-abhängige DNA-Polymerase, die die DNA-Synthese in 5'-3'-Richtung katalysiert, aber auch eine 3'-5'-Exonukleaseaktivität gegenüber einzel- und doppelsträngiger DNA aufweist, wodurch sie sich für den Abbau nicht ligierter ODN eignet.

Vor Durchführung des T7-Verdaus wurde der Ligationserfolg überprüft. Dazu wurden je 0,3 μg der ODN, des Ligationsansatzes und eines Probe T7-Verdaus in einem 3% Agarosegel aufgetrennt (s. 2.4.1). Für den Probe T7-Verdau wurde ein Überschuss an T7 DNA Polymerase im Verhältnis 10 U/1,1 μg DNA in einem Volumen von 21 μL eingesetzt. Die Reaktion erfolgte für 1 h bei 37˚C und wurde durch 10 minütiges Erhitzen auf 70˚C gestoppt.

Für den T7-Verdau wurde der Ligationsansatz mit Aqua Spüllösung und der entsprechenden Menge 10 x Ligase-Puffer verdünnt, so daß die DNA in einer Konzentration von 0,3 g/L in 1 x Ligasepuffer vorlag. Es wurde die für ein Verhältnis von 0,03 U/μg DNA benötigte Menge T7 DNA Polyermase zugegeben. Der Ansatz wurde im Wasserbad bei 37˚C für 15-24 h inkubiert. Der Erfolg der Reaktion wurde durch Auftrennung von 0,3 μg des Ligationsansatzes neben 0,3 μg und 0,9 μg des T7-Verauansatzes auf einem 3% Agarosegel überprüft.

Aufreinigung der erfindungsgemäßen Moleküle

Die Aufreinigung erfolgte mit Hilfe der Anionenaustausch-Chromatographie. Das Prinzip dieser Methode beruht auf der Anwesenheit von positiv geladenen Gruppen in einer porösen Matrix (stationäre Phase), die mit den oberhalb von pH 2 negativ geladenen Phosphatgruppen der Nukleinsäuren interagieren, so dass diese an die stationäre Phase gebunden werden. Die stärke der Interaktion ist abhängig vom pH-Wert und der Ionenstärke der mobilen Phase sowie der im Molekül vorhandenen negativen Ladungen. Ungeladene Moleküle interagieren nicht oder nur geringfügig mit der stationären Phase und werden mit einer mobilen Phase geringer Ionenstärke rasch eluiert. Durch Erhöhung der Ionenstärke der mobilen Phase werden die an die stationäre Phase gebundenen Moleküle von der Säule verdrängt. Dabei eluieren größere Nukleinsäuren wegen der stärkeren Interaktion mit der stationären Phase später als kleinere. [Lottspeich 1998, Mülhardt 2003]

Für die Aufreinigung wurde als stationäre Phase Fractogel-DMAE, ein Polymerharz mit Dimethylaminoethylgruppen, verwendet. Zur Auftrennung wurde ein NaCl-Stufengradienten (0%, 50%, 100% 1 M NaCl) eingesetzt.

Vor Beginn der Aufreinigung wurden alle Aus- und Eingänge der HPLC-Anlage, die im Ruhezustand mit 20% Ethanol gefüllt ist, zunächst mit Aqua Spüllösung gespült und die Säule gepackt. Dazu wurden 1,6-1,8 mL DMAE in die Säule gefüllt, mit Wasser bis zum Rand aufgefüllt und die Säule geschwenkt, um eine gleichmäßige Verteilung zu gewährleisten. Die Säule wurde dann luftblasenfrei an die HPLC-Analge angeschlossen. Mit einer Flussgeschwindigkeit von 1 mL/min wurde die Säule so lange gespült, bis sich das Säulenmaterial vollständig abgesetzt hatte. Der obere Stempel wurde luftblasenfrei auf die Matrix geschraubt und so das überflüssige Wasser aus der Säule verdrängt. Die Säule wurde erneut an die HPLC-Anlage angeschlossen und mit einer Flussrate von 1 mL/min durchspült, gegebenenfalls auftretende Totvolumina zwischen Matrix und Stempel wurden durch Wiederholung des letzten Schrittes beseitigt. Die so gepackte Säule, wie auch die gesamte HPLC-Anlage, wurde anschließend mit einer Flussrate von 1 mL/min mit Puffer T20 equilibiert. Die Equilibrierung der Säule erfolgte mit ca. dem 10fachen Volumen der Säulenmatrix und wurde mit pH-Indikatorpapier überprüft. Die Ansätze wurden mit Hilfe einer Hamilton-Spritze über eine 2 mL Probenschleife in die HPLC-Anlage injiziert. Die Steuerung der HPLC-Analge erfolgte mit Hilfe eines Software-Protokolls, das so konzipiert war, dass die Fraktionierung automatisch erfolgte. In einigen Fällen wurden auftretende Nebenmaxima jedoch manuell fraktioniert. Vor dem Probenauftrag wurde die Säule mit dem zweifachen Säulenvolumen (column volume: CV) equilibiert, dann erfolgte die Elution von nicht gebundenen Molekülen (Proteinen, Nukleotide) mit 5 CV Puffer T20, gefolgt von der Elution schwach gebundener Moleküle (ATP, ODN) mit 7 CV 50% Puffer T20N1000 und der Elution von dSLIM mit 7 CV 100% Puffer T20N1000. Die Flussrate betrug 1 mL/min. Anschließend wurde die Säule für den nächsten Probenauftrag mit 10 CV Puffer T20 re-equilibriert, die Aus-und Eingänge der HPLC-Anlage manuell mit Puffer T20 gespült.

Die HPLC-aufgereinigten Moleküle wurden durch Ethanol-Fällung konzentriert und entsalzt. In Gegenwart monovalenter Kationen bilden Nukleinsäuren in Alkohol einen unlöslichen Niederschlag, der durch Zentrifugation isoliert werden kann. Mit gefälltes Salz kann durch Waschen in 70 %igem Ethanol größtenteils entfernt werden, da es im Gegensatz zu Nukleinsäuren in diesem löslich ist. Um die Ausbeute bei geringeren, weniger gut präzipitierender Mengen an Nukleinsäuren zu erhöhen, kann die Fällung bei niedrigen Temperaturen durchgeführt und/oder $Mg^{2+}$-Ionen zugegeben werden. Falls für die nachfolgenden Anwendungen nicht störend, können auch Trägermaterialien, welche die effektive Konzentration der Nukleinsäuren erhöhen, wie tRNA oder Glykogen zugegeben werden. [Lottspeich 1998, Mülhardt 2003]

Für die Fällung wurden zu den jeweiligen Fraktionen der HPLC-Aufreinigung in Corex-Zentrifugengläser überführt und 1/100 des Volumens 1 M $MgCl_2$ sowie 1/10 des Volumens 3 M Natriumacetatlösung sowie das 2,5fache Volumen 96% Ethanol zugegeben. Die Ansätze wurden gemischt und 2-24 h bei -20˚C gefällt. Die Ansätze wurden anschließend für 30-60 min bei 10.000 U/min (11.000 g) und 4˚C zentrifugiert, der Überstand abgenommen und mit 5 ml 70% Ethanol gewaschen und erneut für 10-30 min bei 10.000 U/min (11.000 g) und 4˚C zentrifugiert. Der Überstand wurde abgenommen und das DNA-Pellet an der Luft getrocknet, bis kein Alkohol mehr zu riechen war. Die DNA wurde in 100-350 μL Aqua Spüllösung aufgenommen und in sterile 1,5 ml Reaktionsgefäße überführt. Das Volumen, in dem resuspendiert wurde, wurde anhand der für die Herstellung eingesetzten DNA-Menge so abgeschätzt, das eine Konzentration von 1 g/L bei einer Ausbeute von 50% resultiert.

Agarosegelelektrophorese

Für die Auftrennung und Charakterisierung von Nukleinsäuren eignet sich die Gelelektrophorese. Die über einen weiten pH-Bereich negativ geladenen Nukleinsäuren werden in einer Matrix aus Agarose oder Polyacrylamid einem elektrischen Feld ausgesetzt und wandern zur Anode. Dabei unterscheidet sich ihre Wanderungsgeschwindigkeit entsprechend ihrer Größe. Das Verhalten von Nukleinsäuren (bis ca. 10 kb) während der Gelelektrophorese kann als Mischung aus zwei Theorien beschrieben werden. Der Ogstron-Sieb-Effekt beruht auf der Annahme, das Nukleinsäuren eine globuläre Form annehmen und um so häufiger mit der Gelmatrix kollidieren, je größer der Umfang des Partikels ist, wodurch größere Moleküle stärker abgebremst werden als kleine. Moleküle, deren Durchmesser größer ist als der Porendurchmesser des Gels sollten laut dieser Theorie nicht durch das Gel wandern. Die Reptationstheorie geht davon aus, dass die Nukleinsäuren im Gel ihre globuläre Form aufgeben und sich schlangenartig mit einem Ende voran durch das Gel bewegen, wobei längere Moleküle für diese Bewegung mehr Zeit benötigen als kurze. Wegen des Einflusses der Größe auf die Wanderungsgeschwindigkeit von Nukleinsäuren, wirkt sich auch die Ausbildung von Sekundärstrukturen auf das Laufverhalten im Gel aus. So unterscheidet sich beispielsweise das Laufverhalten von Plasmid-DNA entsprechend ihrer Konformation. Die Wanderungsgeschwindigkeit nimmt von offener (Form I) über lineare (Form III), superhelikale (Form II) zu denaturierter (Knäuel) Plasmid-DNA zu. Für lineare doppelsträngig DNA (Form III) besteht über einen weiten Bereich ein Zusammenhang zwischen dem $\log_{10}$ der Länge (in bp) und der relativen Wanderungsstrecke im Gel, so dass anhand von Längenstandards eine relativ genaue Größenbestimmung erfolgen kann.

Der Nachweis von Nukleinsäuren in Gelen kann mit Ethidium-Bromid erfolgen, welches aufgrund seiner planaren Struktur in die DNA interkaliert, wodurch es mit Licht im UV-Bereich (254-366 nm) zur Fluoreszenz angeregt werden kann, deren Emission im organge-roten Bereich (590 nm) beobachtet wird. [Lottspeich 1998].

Agarose ist ein Polysaccharid, das aus Meeralgen gewonnen wird und in wässriger Lösung nach Abkühlung relativ großporige Gele ausbildet (1% (w/v) ca. 150 nm). Die Porengröße ist dabei umgekehrt proportional zur Agarosekonzentration. Agarosegele eignen sich zur Trennung von Nukleinsäuren von 0,1 bis 25 kb Länge. Der Vorteil von Agarosegelen besteht in ihrem relativ großen Auftrennungsbereich und der einfachen Handhabung, allerdings ist die Auflösung für kleine DNA-Fragmente sehr gering. Für die Auftrennung von kleineren DNA-Fragmenten eignet sich Sieving-Agarose, eine derivatisierte Agaroseform. [Lottspeich 1998, Mühlhardt 2003]

Für die Auftrennung von ODN und erfindungsgemäßen Molekülen wurden 3% Agarosegele in 1 x TAE-Puffer mit 0,125 μg/mL Ethidium-Bromid verwendet. Es wurden horizontale Gele mit Hilfe des BioRad Systems gegossen (40 ml für kleine Gele mit 8 Taschen, 100 ml für große Gele mit 20 Taschen). Die zu analysierenden Proben wurden in 1 x Probenpuffer neben entsprechenden Längenstandards (s. 2.4.3) aufgetragen. Die Elektrophorese erfolgte in 1 x TAE-Puffer bei 120 V für 30 min. Die Gele wurden mit der Geldokumentationsanlage fotographhiert.

Native Polyacrylamid-Gelelektrophorese (PAGE)

Polyacrylamid-Gele entstehen durch radikalische Kopolymerisation von Acrylamidmonomeren mit dem Vernetzer N,N'-Methylenbisacrylamid. Die Porengröße der Gele liegt im Bereich von ca. 3-6 nm und ist abhängig von der totalen Acrylamidkonzentration (% T = $m_{Acrytamid} + m_{Bisacrylamid}$ (w/v)) und dem Vernetzungsgrad (% C = $m_{Bisacrylamid} / m_{Acrylamid} + m_{Bisacrylamid}$). Sie nimmt bei konstantem C mit steigendem T ab und weist bei konstantem T ein Minimum bei 5% C auf. Polyacrylamidgele weisen ein sehr gutes Auflösungsvermögen besonders für kleine DNA-Fragmente (< 1 kb) auf, allerdings ist der Auftrennungsbereich im Vergleich zu Agarosegelen wesentlich geringer. Durch Verwendung von Gradientengelen kann der Auftrennungsbereich erweitert werden. Das bessere Auflösungsvermögen von Polyacrylamid-Gelen ermöglicht die Unterscheidung verschiedener DNA-Konformationen, die wegen ihrer verschiedenen Form geringfügig unterschiedliches, durch Temperatur und die Ionenkonzentration beeinflussbares Laufverhalten im nativen Gel aufweisen.

Für die Auftrennung der Moleküle erwiesen sich Gele mit 8% T, 2,6 % C und für die Auftrennung von ODN Gele mit 12% T, 5% C in 1 x TBE als optimal. Die horizontalen Gele wurden am Tag vor der Elektropohorese gegossen (15 ml/$Gel_{klein}$, 25 ml/$Gel_{gross}$) und über. Nacht im Kühlschrank aufbewahrt. Vor dem Probenauftrag wurden die Gele bei 70 V (170 $V_{gross}$) vorelektrophoriert, bis ein keine Änderung der Stromstärke mehr zu beobachten (10-11 mA) war. Die Proben wurden in 1 x Probenpuffer ohne Zusatz von Farbstoffen neben den entsprechenden Längenstandards (s. 2.4.3) aufgetragen. Zur Verfolgung des Elektrophoresefortschrittes wurde 1 x Loading Dye in einer separaten Spur aufgetragen. Die Elektrophorese erfolgte in 1 x TBE bei konstanter Spannung von 70 V (170 $V_{gross}$) (≈ 9 V/cm) bei Raumtemperatur und wurde gestoppt, wenn Bromphenolblau das untere Ende des Gels erreicht hatte (ca. 2 h). Nach der Elektrophorese wurden die Gele in Ethidiumbromid-Lösung für 10-15 min gefärbt und mit der Geldokumentationsanlage fotographiert. Bei zu starker Hintergrundfärbung wurden die Gele für 10-30 min in deminieralisiertem Wasser gewaschen.

Thermische Denaturierung und Renaturierung zur Zuordnung der Banden

Da es sich bei monomeren DNA-Molekülen um zirkuläre DNA-Moleküle handelt, die ähnlich wie Plasmid-DNA, ein komplexes Laufverhalten im Gel zeigen, können die im Gel beobachteten Banden nicht ohne weiteres einer Konformation oder Größe zugeordnet werden. Zur Unterscheidung kann die Tatsache dienen, dass verschiedene Konformationen sich im Gegensatz zu unterschiedliche großen Molekülen unter entsprechenden Bedingungen reversibel ineinander umwandeln lassen.

Die unterschiedlichen Konformationen weisen einen verschiedenen Kompaktierungs-grad auf und sollten daher im Gel durch ihr unterschiedliches Laufverhalten zu unterscheiden sein, dabei weisen kompakte DNA-Formen im Gel meist eine höhere Mobilität auf, als voluminösere offene DNA-Formen. Durch Zerstörung von Wasserstoffbrückenbindungen sollten sich die kompakten Formen in die offenen Formen umwandeln lassen.

Für eine Zuordnung der im Polyacrylamid-Gel beobachteten Banden wurden die Moleküle durch Erhitzen für 10 min auf 95°C thermisch denaturiert. Die denaturierten Proben wurden sofort auf Eis gestellt, um eine Renaturierung zu verhindern. Dazu wurde ein Mastermix, der das jeweilige dSLIM-Molekül in einer Konzentration von 0,05 g/L in 1 x TE-Puffer enthielt, hergestellt. Dieser Ansatz wurde halbiert und je 1 Ansatz erhitzt und 1 Ansatz bei Raumtemperatur belassen. Von den Ansätzen wurden Proben für den Gelauftrag entnommen und mit der entsprechenden Menge 5 x Probenpuffer versetzt und auf einem nativen 8% Polyacrylamidgel aufgetrennt. Um die Renaturierung zu beobachten, wurde der verbliebene denaturierte Ansatz erneut aufgeteilt und anschließend für 3 Tage jeweils bei 4°C bzw. 37°C inkubiert. Der verbliebene nicht-denaturierte Ansatz wurde bei 4°C gelagert und nach 3 Tagen ebenfalls geteilt, wobei ein Teil wie oben beschrieben denaturiert und der andere Teil bei Raumtemperatur belassen wurde. Die Ansätze wurden mit der entsprechenden Menge 5 x Probenpuffer versetzt und auf einem nativen 8% Polyacrylamidgel aufgetrennt.

Inkubation im Zellkulturmedium

Die Aktivität der Moleküle wurde mit Hilfe von PBMC und HEK293-Zellen *in vitro* getestet. Die Moleküle sind nicht in Puffer, sondern in proteinhaltigem Zellkulturmedium gelöst und werden bei einer Temperatur von 37°C inkubiert. Der Einfluss dieser veränderten Parameter auf die Struktur und Stabilität der Moleküle und ODN sollte daher untersucht werden.

Ein wichtiger Faktor, der die Wirkung von DNA-Molekülen in verschiedenster Weise beeinflussen kann, ist die unspezifische Bindung an Proteine. Durch Bindung an Proteine ändert sich das Laufverhalten von DNA in der nicht denaturierenden Polyacrylamidgel-Elektrophoreses (native PAGE), so dass eine Verschiebung der DNA-Bande in Anwesenheit von Protein erkennbar ist. In den meisten Fällen ist die elektrophoretische Mobilität des DNA-Proteinkomplexes im Vergleich zur DNA vermindert, bei DNA-Minizirkeln, kann es auch zu einer Erhöhung der elektrophoretischen Mobilität kommen, wenn der Kompaktierungsgrad durch die Proteinbindung erhöht wird [Toulmé 1995].

Für die Untersuchung des Verhaltens der in den Stimulationsversuchen eingesetzten Moleküle und ODN unter Bedingungen wie sie in den Stimulationsversuchen herrschten, wurden die ODN und Molekül-Lösungen in einer Konzentration von 1 μM in Medium verdünnt und für 5 h oder 24 h bei 37°C im Heizblock inkubiert oder direkt, nach Zugabe der entsprechenden Menge 5 x Probenpuffer, auf einem Polyacrylamid-Gel aufgetrennt. Zum Vergleich wurden ODN und Molekül-Lösungen, die entsprechend in $H_2O$ verdünnt waren, aufgetragen. Um zu verifizieren, dass die beobachteten Veränderungen durch die Anwesenheit von Proteinen verursacht waren, wurden Ansätze mit ODN und erfindungdsgemäßen Molekülen in einer Konzentration von 1 μM in Medium verdünnt, dem kein FCS zugesetzt war. Für den Vergleich der Laufweiten von DNA- und Proteinbanden im Gel durch den Rückschlüsse über mögliche Proteinbindung gezogen werden können, wurde in den Gelen neben der DNA auch die Proteine nachgewiesen.

Der zusätzliche Nachweis von Proteinen erfolgte im Anschluss an den Nukleinsäurenachweis. Die Gele wurden für 30 min in Fixierlösung fixiert und dann 30-60 min in Coomassie-Lösung gefärbt. Überschüssiger Farbstoff wurde durch Inkubation in Entfärbelösung über Nacht entfernt und die Gele mit der Geldokumentationsanlage fotographiert.

Verwendete DNA-Längenstandards:

Von den Längenstandards wurde die vom Lieferanten empfohlene Menge/mm Taschenbreite in jeweils 1 x Probenpuffer aufgetragen. Es wurden folgende Standards verwendet: GeneRuler 100 bp DNA Ladder Plus (MBI Fermentas)

GeneRuler 50 bp DNA Ladder (MBI Fermentas)
10 bp DNA Ladder (Invitrogen)
25 bp DNA Ladder (Invitrogen)

Zellkultur

Alle Zellkulturarbeiten wurden unter sterilen Bedingungen mit sterilen Einwegmaterialien durchgeführt.
Im Brutschrank herrschten folgende Bedingungen: 37˚C, 5 % $CO_2$, 90% Luftfeuchtigkeit.

Untersuchung der immunmodulatorischer Wirkung der Moleküle an PBMC

Für die Untersuchung der immunmodulatorischen Wirkung der multimeren Molekülen wurde die Cytokinsekretion von Lymphozyten und Monozyten, die aus menschlichen Blutspenden gewonnen wurden, mit Hilfe von ELISA gemessen.
Ausgangsmaterial für die Isolation von Lymphozyten und Monozyten, die auch als *Peripheral Blood Mononuclear Cells* (PBMC) = mononukleäre Zellfraktion des peripheren (herzfernen) Blutes bezeichnet werden, war ein Leukozytenkonzentrat, auch als *Buffy coat* bezeichnet, das durch Zentrifugation von Vollblut gewonnen wird und vom DRK-Blutspendedienst Berlin Wannsee bezogen wurde. Durch eine Ficoll-Hypaque-Dichtegradientenzentrifugation lassen sich die PBMC von den anderen Bestandteilen des Leukozytenkonzentrats (Plasma, Thrombozyten, Erythrozyten, Granulozyten) abtrennen. Bei dem Separationsmedium Ficoll-Hypaque handelt es sich um eine Lösung mit einer Mischung aus Ficoll 400, einem stark verzweigtem Polymer aus Saccharosemonomeren, welche über Epichlorhydrin kreuzvernetzt sind und Natriumdiatrizoat (Hypaque), mit einer Dichte von 1,077 g/mL. Durch isopyknische Zentrifugation des mit verdünntem Leukozytenkonzentrats überschichteten Separationsmediums kann eine Auftrennung der verschiedenen Bestandteile entsprechend ihrer unterschiedlichen Dichte erfolgen und man erhält die in Abbildung 2.5.1 dargestellten Fraktionen. [Luttmann 2004]
Für die Isolation der PBMC wurde je ein Leukozytenkonzentrat in eine sterile 250 mL Flasche überführt und mit PBS 1:2 verdünnt. In vier 50 mL Zentrifugenröhrchen wurden je 15 mL Ficoll-Hypaque-Lösung vorgelegt und diese mit einer 10 mL Pipette vorsichtig mit ca. 30 mL des Blut-PBS-Gemisches überschichtet und bei 800 g für 20 min ohne Bremse zentrifugiert (Gesamtdauer ca. 50 min). Die PBMC-haltige Interphase wurde vorsichtig mit einer 5 mL-Pipette abgesaugt und in 50 mL Zentrifugenröhrchen mit 20 mL kaltem PBS überführt. Um Kontaminationen von Erythrozyten, Ficoll/Hypaque und Thrombozyten zu entfernen, wurden die Zellen anschließend mehrfachen Waschschritten unterzogen. Während bis zur Isolation der PBMC alle Schritte bei Raumtemperatur ausgeführt wurden, um die Aggregation von Thrombozyten zu vermeiden, wurden die Waschschritte bei 4˚C ausgeführt, um Adhäsion von Monozyten an die verwendeten Plastikmaterialien und damit deren Verlust zu verhindern. Im ersten Waschschritt wurden die Zellen bei 400 g für 8 min bei 4˚C zentrifugiert. Der Überstand wurde abgesaugt und das Zellpellet in 5 ml kaltem PBS resuspendiert und auf 45 ml mit kaltem PBS aufgefüllt. Die nachfolgenden Waschschritte wurden ebenso durchgeführt, die Zentrifugation erfolgte jedoch bei 300 g für 5 min. Die Waschschritte wurden so lange wiederholt, bis der Überstand klar war und das Pellet eine gelbe Farbe aufwies (ca. 5-6 x). Die Zellen wurden anschließend in jeweils 5 mL kaltem Medium resuspendiert, vereinigt und das Volumen auf 30 mL mit kaltem Medium aufgefüllt. Die Zellzahl wurde mit Hilfe des automatischen Zellzählgerätes bei einer Ausschlussgröße von 8 $\mu$m bestimmt und mit kaltem Medium eine Konzentration von 4 x $10^6$ Zellen/mL eingestellt.
Es wurden je 600 $\mu$L der zuvor isolierten PBMC in einer Konzentration von 4 x $10^6$ Zellen/mL in eine Vertiefung von 24 Loch-Zellkulturplatten gegeben. Anschließend wurde das entsprechende Volumen der zu testenden Moleküle zugegeben, so dass die Konzentration im Ansatz 1 $\mu$M betrug. Die Zellen wurden für 2 Tage (42-48 h) im Brutschrank inkubiert. Die Zellsuspension wurde in ein Eppendorf-Reaktionsgefäß überführt und bei 3000 U/min in der Biofuge für 4 min zentrifugiert. Die so erhaltenen zellfreien Überstände wurden in ein neues Reaktionsgefäß überführt und entweder direkt zur Bestimmung der Cytokinkonzentraition mit Hilfe von ELISA eingesetzt oder bei -70˚C aufbewahrt.

Kultivierung von HEK293-Zellen

Die Zellen wurden in 162 $cm^2$ Zellkulturflaschen kultiviert. Sie wurden in einer Dichte von 1,3-1,9 x $10^5$ Zellen/$cm^2$ ausgesät. Nach 2-3 Tagen Inkubation im Brutschrank wurden die Zellen 1:3 geteilt. Dazu wurde das Medium abgesaugt, die Zellen mit 10 mL PBS gewaschen und anschließend bei Raumtemperatur 3-5 min bis zur Ablösung mit 5 mL Trypsin/EDTA-Lösung inkubiert. Die Reaktion wurde durch Zugabe von 5 mL Medium gestoppt. Die Zellen wurden resuspendiert und weitere 5 mL Medium zugegeben. Je 5 mL der Zellsuspension wurden entweder in der Zellkulturflasche belassen oder in eine neue überführt und 45 mL Medium zugegeben. Gelegentlich kam es zur Ablösung der Zellen vom Boden der Flasche, bevor das Medium abgesaugt war. In diesem Fall wurden die Zellen in ein steriles Zentrifugenröhrchen überführt und bei 300 g für 4 min abzentrifugiert. Anschließend wurde das Medium abgesaugt und die Zellen in 5 mL Trypsin/EDTA-Lösung resuspendiert, 10 mL Medium zugegeben und wie oben beschrieben in eine neue Zellkulturflasche überführt.

Stimulation von HEK293-Zellen

Für die Etablierung des Tests wurden jeweils HEK293-TLR9- und HEK293-Null-Zellen in Konzentrationen von 0,6-1 x $10^6$ Zellen/mL in einem Volumen von 400 μL in 24 Loch-Zellkulturplatten ausgesät und für 1 bis 5 Tage kultiviert. Die Zellzahl wurde mit Hilfe des Zellzählgerätes bei einer Ausschlussgröße von 8 μm bestimmt und die entsprechende Konzentration eingestellt. Bei längerer Kultivierung erfolgte ein Mediumwechsel nach 48 h. Dazu wurde das Medium vorsichtig abgesaugt und jeweils 400 μL frisches Medium vorsichtig auf die Zellen gegeben. In einigen Ansätzen erfolgte ein Mediumwechsel auch unmittelbar vor der Zugabe der Stimulanzien. Die Stimulation erfolgte durch Zugabe eines entsprechenden Volumens der ODN/Moleküle, so dass eine Endkonzentration von 2,5 μM bzw. 1 μM erreicht wurde. LPS wurde in einer Konzentration von 0,5 μg/mL eingesetzt. Die Zellen wurden anschließend für verschiedene Zeiten (6-48 h) im Brutschrank inkubiert. Schließlich wurde das Medium in 1,5 mL Reaktionsgefäße überführt, bei 1400 U/min in der Biofuge zentrifugiert, um gegebenenfalls enthaltene Zellen zu pelletieren und die Überstände zur Bestimmung der IL-8 Konzentration mit Hilfe von ELISA eingesetzt.

Cytokin/Chemokin-Nachweis mit Hilfe von ELISA

Für die Untersuchung der immunmodulatorischen Wirkung von dSLIM auf PBMC wurde die Induktion von IL-6, IFN-alpha und IFN-gamma gemessen.

IL-6 ist ein multifunktionelles Cytokin, das von einer Vielzahl aktivierter Lymphozyten und Monozyten sezerniert wird und, neben der Induktion von Akute-Phase-Proteinen in Hepatozyten, auf Lymphozyten eine Wachstums- und Differenzierungssowie eine Phagozytose-fördernde Wirkung hat [Kirchner 1994].

IFN-alpha von dem mehrere Subtypen existieren, gehört zur Familie der Typ I Interferone, die hauptsächlich antiviral wirken. Große Mengen von IFN-$\alpha$ werden von pDC sezerniert, nach Aktivierung von TLR7, 8 oder 9 [Perry 2005] s.a. Abschnitt 1.3.2

IFN-gamma wird hauptsächlich von NK- und T-Zellen, aber auch von Monozyten, Dendritischen Zellen und B-Zellen sezerniert. Es ist das einzige Interferon vom Typ II und hat im Gegensatz zu Typ I Interferonen eher eine immunmodulatorische als eine antivirale Wirkung. Neben seiner Rolle bei der Differenzierung, ist IFN-gamma das wichtigste Effektorcytokin einer $T_H$1-gerichteten Immunantwort.

Die Sekretion von IL8/CXCL8 diente als Nachweis der Aktivierung von TLR9 in transformierten HEK293-Zellen. IL-8 ist ein CXC-Chemokin, das von Leukozyten aber auch von Firbroblasten, Endo- und Epithelzellen sezerniert wird. Die Induktion von IL8/CXCL8 erfolgt z.B. durch IL-1 und TNF-gamma, aber auch durch PRR und Umweltfaktoren wie Hypoxie. Die Expression von IL8/CXCL8 wird durch Regulation der Transkription über kooperative Aktivierung von NF-alpha und AP-1 sowie auf der Ebene der mRNA-Stabilität reguliert. Neben seiner Wirkung als Aktivator von Neutrophilen, wirkt IL8/CXCL8 chemotaktisch auf verschiedene Leukozyten und ist am Prozess der Transmigration von Leukozyten in Gewebe beteiligt [Mukaida 2003].

Zum Nachweis von Cytokinen in den Zellkulturüberständen von PBMC und HEK293-Zellen wurde der *Enzyme-linked Immunosorbent Assay* (ELISA) als *"Sandwich-ELISA"* angewendet. Es handelt sich um einen quantitativen Immunoassay, bei dem für das zu detektierende Protein spezifische Antikörper an der Oberfläche einer Mikrotiterplatte durch Adsorption immobilisiert werden. Durch Bindung der Antigene an den jeweiligen Antikörper werden diese ebenfalls immobilisiert und andere Bestandteile können durch Waschen entfernt werden. Da es sich bei der Bindung von Antigen und Antikörper um eine Gleichgewichtsreaktion handelt, ist die Menge des gebundenen Antigens konzentrationsabhängig. Der Nachweis des Antigens erfolgt mit einem zweiten spezifischen, biotinylierten Antikörper, der wiederum das zur Detektion eingesetzte Streptavidin-konjugierte Enzym bindet. Im hier durchgeführten Assay handelte es sich hierbei um Meerrettichperoxidase (*horseradishperoxidase* = HRP). Die eigentliche Messgröße stellt die vom Enzym in einer bestimmten Zeit umgesetzte Menge (Konzentration) eines chromogenen Substrates dar. Hier wurde als Substrat TMB (3,3',5,5'-Tetramethylbenzidin) verwendet, das in Gegenwart von $H_20_2$ oxidiert wird und dann Licht einer Wellenlänge von 370 nm absorbiert (blau). Durch zugabe von $H_2SO_4$ wird die Reaktion gestoppt wodurch sich die Absoptions-Wellenlänge auf 450 nm (gelb) ändert [Luttmann 2004]. Mit Hilfe einer Standardverdünnungsreihe einer bekannten Konzentration des zu bestimmenden Cytokins können auf diese Weise die unbekannten Cytokinkonzentrationen in den Zellkulturüberständen bestimmt werden.

Die benötigten Antikörperpaare, Standard-, Enzym- und Substratlösungen wurden als Kits von der Firma R&D Systems bezogen. Die für die Durchführung benötigten Geräte und Materialien sind in der folgenden Tabelle aufgeführt.

IL-6 und IFN-gamma

Der Nachweis von IL-6 und IFN-gamma in den Zellkulturüberständen von PBMC erfolgte mit den "DuoSet ELISA Development System" Kits der Firma R&D Systems entsprechend der beiliegenden Anleitungen. Dazu wurde der "Capture Antibody" mit PBS auf eine Konzentration von 4 μg/mL (IFN-gamma) bzw. 2 μg/mL (IL-6) verdünnt und je 100 μL in eine Vertiefung der Mikrotiterplatte gegeben. Die Platte wurde über Nacht bei Raumtemperatur inkubiert, anschließend 3 x mit Waschpuffer gewaschen und mit je 300 μl Blockierungspuffer pro Vertiefung zum Absättigen freier Bindungsstellen für 1-2 h inkubiert. Die Platte wurde 3 x mit Waschpuffer gewaschen und anschließend je 100 μL der Proben und Standards auf die Platte gegeben. Die Überstände wurden für den IL-6 Nachweis in einer Verdünnung von 1:2 in Reagenzpuffer und für den IFN-gamma Nachweis unverdünnt eingesetzt. Die Standardverdünnungsreihe für IL-6 umfasste folgende Konzentrationen: 12,5; 25; 50; 100; 200; 350; 700 pg/ml. Die Standardverdünnungsreihe für IFN-gamma umfasste folgende Konzentrationen: 12,5; 25; 50; 100; 250; 500; 1000 pg/mL. Es wurden jeweils Doppelbestimmungen durchgeführt. Die Inkubationsdauer betrug 2 h. Nach dreimaligem Waschen mit Waschpuffer wurden je 100 μL des "Detection Antibody" in einer Konzentration von 100 ng/mL (IL-6) bzw. 200 ng/mL (IFN-gamma) in Reagenzpuffer auf die Platte gegeben. Nach einer Inkubationszeit von 2 h wurde die Platte 3 x mit Waschpuffer gewaschen und je 100 μl "Streptavidin-HRP" 1:200 verdünnt in Reagenzpuffer in die Vertiefungen gegeben. Die Inkubationszeit betrug 20 min. Nach einem letzten 3maligem Waschschritt wurden je 100 μL der Substratlösung ("Color Reagent" A und B im Verhältnis 1:1) auf die Platten gegeben und im Dunkeln für 20 min inkubiert. Die Reaktion wurde durch Zugabe von 50 μL 1M $H_2SO_4$ gestoppt und die Absorption in den einzelnen Vertiefungen im Mikroplatten Lesegerät bei 450 nm gemessen. Die Auswertung erfolgte mit der Software SoftMax Pro 2.6. Die Standardkurve wurde mit einem 4-Parameter Fit berechnet.

IFN-alpha

Der Nachweis von IFN-alpha in den Zellkulturüberständen von PBMC erfolgte mit dem "Human Interferon-alpha ELISA" Kit der Firma Biosource entsprechend der beiliegenden Anleitung. Je Vertiefung wurden 100 μl der Standards und Proben in die bereits mit Antikörper versehenen und blockierten im Kit enthaltenen Mikrotiterstreifen gegeben und für 1 h bei RT inkubiert. Die Überstände wurden 1:2 verdünnt mit "Dilution Buffer" eingesetzt und die Standardverdünnungsreihe umfasste die folgenden Konzentrationen: 156; 312; 625; 1250; 2500; 5000 pg/ml. Nach einem Waschschritt wurden je 100 μl des "Antibody Concentrate D" in "Dilution buffer C" pro Vertiefung zugegeben und 1 h bei RT inkubiert. Die Platte wurde 3 x gewaschen und je 100 μl HRP in "HRP-Diluent" auf die Platten gegeben und für 1 h inkubiert. Die Lösung wurde durch 4maliges Waschen entfernt und je 100 μL "Substratsolution G" in die Vertiefungen pipettiert. Die Inkubation erfolgte im Dunkeln für 20 min und die Reaktion wurde durch Zugabe von 100 μL "Stopp-Solution H" gestoppt. Im Mikroplatten Lesegerät wurde die Absorption bei 450 nm gemessen. Die Auswertung erfolgte mit der Software SoftMax Pro 2.6. Die Standardkurve wurde mit einem Point-to-Point Fit berechnet.

IL-8/CXCL8

Der Nachweis von IL-8/CXCL8 in den Zellkulturüberständen von HEK293-Zellen erfolgte mit den "DuoSet ELISA Development System" Kit der Firma R&D Systems entsprechend der beiliegenden Anleitung und war identisch mit der bei IL-6 und IFN-□ beschriebenen. Die Standardverdünnungsreihe umfasste folgende Konzentrationen: 31,25; 62,5; 125; 250; 500; 1000; 2000 pg/ml. Die Überstände wurden unverdünnt eingesetzt. Die eingesetzten Antikörperkonzentrationen betrugen: "Capture Antibody" 4 μg/mL, "Detection Antibody" 20 ng/mL.

Herstellung und Beschreibung von multimeren DNA-Moleküle

Um den Einfluss struktureller Merkmale zu untersuchen, die insbesondere die Fähigkeit zur Ausbildung von G-Strukturen begünstigen, wurden Kombinationen von monomeren DNA-Moleküle mit poly(G)-Motiven in verschiedenen Bereichen mit dem erfindungsgemäßen Verfahren hergestellt, dass abgesehen von der Lyophilisierung der Herstellung des Monomere entspricht. Dazu wurden je drei unterschiedliche Sequenzen für Stamm (S) und Loop (L) miteinander kombiniert, die sich zum einen in der Anzahl und Lokalisation von Guaninbasen, aber auch in der durch das Programm "DNA mfold" vorhergesagten Sekundärstruktur des Loops unterschieden. Die einzelnen Sequenzen und ihre strukturellen Merkmale sind in der folgenden Tabelle wiedergegeben:

**Tabelle 3.1: Strukturelle Merkmale und Sequenzen L1-L3, S1-S3 (rot: poly(G))**

| Name | Sequenz | Strukturelle Merkmale | |
|---|---|---|---|
| | | Poly(G)-Sequenz | Loop tend. offen |
| L1 | T CAT TGG AAA ACG TTC TTC GGG GCG TTC TT | kurz ($G_4$) innerhalb CG-Motiv | nein |
| L2 | T CAT TCC ATA TCG TTC TTC GTG TCG TTC TT | keine | ja |
| L3 | T CGG GGG GTA TCG TTC TTC GTG TCG TTC TT | lang ($G_6$) außerhalb CG-Motiv | ja |
| S1 | 5'-CCT AGG GGT TAC CAC CT ...<br>3'- CCA ATG GTG GA ... | kurz | |
| S2 | 5'-CTG CAG CTG TAG CAG CT ...<br>3'-GAC ATC GTC GA ... | keine | |
| S3 | 5'-CCT AGG GGT GGG GGC CT ...<br>3'-CCA CCC CCG GA ... | lang ($G_4TG_5$) | |

Das folgende Schema gibt einen Überblick über die verwendeten Kombinationen und deren im Weiteren verwendete Nomenklatur:

Tabelle 3.2: Nomenklatur der Moleküle mit verschiedenen Loop/Stamm-Kombinationen

| | S1 | S2 | S3 |
|---|---|---|---|
| L1 | 30L1 | - | MS |
| L2 | ML | KG | GS |
| L3 | - | GL | GLS |

Es konnte gezeigt werden, dass die polymeren dSLIM-Moleküle, die im Sinne der Erfindung auch als multimere Moleküle bzw. oligomere Moleküle bezeichnet werden können, sehr gut geeignet sind, um in einem Organismus eine gesteigerte Immunantwort hervorzurufen. Diese gesteigerte Immunantwort ermöglichte es, die Mittel vorteilhaft im Zusammenhang mit Chemotherapeutika einzusetzen. Die Kombination aus Chemotherapeutika und erfindungsgemäßen Mittel führt gegenüber dem additiven Effekt der beiden Mittel zu einem synergistischen Effekt bei der Behandlung von Tumoren. Durch die Kombination von erfindungsgemäßem Mittel, welches durch die genannten Verfahrensschritte erhältlich ist (siehe Hauptanspruch), werden alle Wirkungen der zusätzlich eingesetzten Chemotherapeutika bzw. Zytostatika verstärkt. Auch wenn monomere Formen von dSLIM-Molekülen bereits mit Chemoptherapeutika bzw. Zytostatika kombiniert wurden, war der Effekt, der durch die Kombination der multimeren bzw. polymeren Form von dSLIM mit Zytostatika erreicht wird, völlig überraschend. In der praktischen Anwendung eines Kombinationsmittel aus der monomeren Form von dSLIM und den Zytostatika zeigte sich, dass nur bestimmte Eigenschaften der Zytostatika verbessert werden konnten, nicht aber - wie bei der polymeren Form - die Gesamtheit ihrer Eigenschaften. Auch war die Aktivierung des Immunsystems durch die polymere / multimere Form von dSLIM überraschend höher, so dass ein Gesamtergebnis bei der Behandlung von Tumoren erzielt werden konnte, was gegenüber der Verwendung der monomeren Form von Zytostatika überraschend verbessert war. So konnte insbesondere die Ausbildung von Metastasen durch den Einsatz von multimeren dSLIM-Molekülen in einem Organismus verhindert werden, wenn zusammen mit diesen Mitteln Zytostatika / Chemotherapeutika gegeben wurden. Das Zusammengeben im Sinne der Erfindung bedeutet die zeitgleiche wie auch die nicht zeitgleiche Gabe der beiden Mittel.

Abbildung 3.1.1 zeigt exemplarisch die beiden unterschiedlichen, für die drei Loopsequenzen von "DNA mfold" berechneten Sekundärstrukturen der dSLIM-Moleküle 30L1 (wie MS) und KG (wie ML, GS, GL, GLS). Bei KG wurden unter den gewählten Bedingungen im Gegensatz zu 30L1 keine Basenpaarungen im Loop vorhergesagt.

Herstellung verschiedener monomerer und multimerer Moleküle

Abbildung 3.1.2 zeigt das Ergebnis der Auftrennung von je 0,3 μg der Ligationsansätze und des Probe-T7-Verdaues der verschiedenen dSLIM-Moleküle neben dem ODN 30L1 in einem 3%igen Agarosegel. In allen Spuren, auf denen die verschiedenen Moleküle aufgetrennt wurden, ist eine Bande kurz unterhalb der Höhe, auf der das 100 bp Fragment des Markers gelaufen ist, zu sehen, die dem monomeren Molekül entspricht. In der Spur, die das ODN 30L1 enthielt ist eine Bande unterhalb der Bande für die monomeren Moleküle sichtbar. Bei den Molekülen, die L3 enthielten (GL, GLS) ist eine unscharfe Verteilung der DNA im oberen Bereich des Gels bis hin zu den Taschen zu sehen, die bei GL deutlich ausgeprägter und bei GLS im T7-Verdau-Ansatz im Vergleich zum Ligationsansatz verringert ist. Bei den Molekülen, die lange poly(G)-Motive enthielten (GL, GS, GLS MS), sind die Banden im Gel im Vergleich zu den anderen Molekülen (30L1, KG, ML) relativ unscharf. In den Spuren, auf die die T7-Verdau-Ansätze aufgetragen wurden, sind die Fluoreszenz-Intensitäten, besonders im oberen Bereich des Gels, geringer als in den Spuren, die mit Ligationsansätzen beschickt wurden.

In Abbildung 3.1.3 sind die Chromatogramme einer nachfolgenden HPLC-Aufreinigung der verschiedenen Moleküle wiedergegeben. Es ist jeweils die Absorption bei 260 nm (blaue Linie) gegen das nach Injektion (gestichelte pinkfarbene Linie) der zu trennenden Probe durch die Säule geflossene Volumen aufgetragen. Die grüne Linie stellt den NaCl-Puffergradienten von 0%, 50% und 100% des 1 M NaCl-Puffers (T20N1000) dar. Der erste Gipfel mit seinem Maximum bei 2,5 mL und 0% T20N1000 (F2) entspricht den ungebundenen Molekülen, der zweite Gipfel mit seinem Maximum bei 8 mL und 50% T20N1000 (F3) den Molekülen mit geringer Affinität zur stationären Phase (in beiden Fraktionen konnte, trotz Auftragung der 12fachen Menge im Vergleich zur Fraktion F4 im Agarosegel keine DNA nachgewiesen werden). Der dritte Gipfel mit seinem Maximum bei 14 mL und 100% T20N1000 (F4) entspricht den monomeren Molekülen.

<u>Abbildung 3.1.3</u>: Chromatogramme der HPLC-Aufreinigung verschiedener monomerer und multimerer Moleküle

a) 30L1 b) ML, c) KG, d) MS, e) GS, f) GLS, g) GL, Säule: 1 mL DMAE; Auftragungsmenge: 700 μg; Flußrate 1 mL/min; Puffer A: 20 mM Tris pH 7; Puffer B: 20 mM Tris, 1 M NaCl, pH7; Gradient:: 0%, 50% 100% Puffer B,

Beim Vergleich der Chromatogramme der verschiedenen Moleküle fällt auf, dass F4 bei den Molekülen, die keine langen poly(G)-Motive, also weder S3 noch L3 enthalten (30L1, ML, KG) eingipfelig ist. Dagegen kann bei den Molekülen, die S3 enthalten eine Schulter an F4 beobachtet werden. Bei den Molekülen, die L3 enthalten (GL, GLS) ist das Maximum zweigipfelig, wobei die Intensitätenverteilung sich zwischen GL und GLS unterscheidet. Bei GLS haben beide Teilmaxima in etwa dieselbe Intensität, während bei GL das bei größerem Volumen beobachtete Teilmaximum eine höhere Intensität aufweist, als das bei geringerem Volumen. Die auftretenden Nebenmaxima bzw. Schultern wurden durch manuellen Eingriff separat fraktioniert. Die Fraktionen wurden entsprechend der zeitlichen Abfolge ihrer Elution mit F1 und F2 bezeichnet.

Auf dem Agarosegel, das in Abbildung 3.1.4 dargestellt ist, sind die oben beschriebenen dSLIM-Fraktionen der HPLC-Trennung nach Ethanol-Fällung, also die fertigen Produkte, aufgetragen. In allen Spuren ist die den monomeren Molekülen entsprechende Bande kurz unterhalb der Laufweite des 100 bp Fragments des Markers zu sehen. Bei ML und KG ist eine zweite schwächere Bande kurz über dieser Bande erkennbar, die bei ML eine größere Intensität aufweist. GL, MS und GLS zeigen eine etwas größere Mobilität im Gel als die anderen Moleküle. Oberhalb der dSLIM-Bande ist bei den Molekülen, die keine langen poly(G)-Motive enthalten (30L1, KG, MS) eine nicht-diskrete Intensitätsverteilung bis zur Höhe von ca. 200 bp zu erkennen. Bei KG und ML ist außerdem eine schwache Bande auf Höhe des 200 bp-Fragmentes des Markers zu erkennen.

Abbildung 3.1.4: Verschiedene monomere und multimere Moleküle nach Ethanolfällung

3% Agarosegel, Auftragungsmenge 0,3 μg, Marker 100 bp 0,5 μg (100 bp, 30L1, 30L1, KG, ML, GL-F1, GL-F2, MS-F1, MS-F2, GS-F1, GS-F2 GLS-F1, GLS-F2, 100 bp)

Bei den Molekülen, bei denen bei der HPLC-Aufreinigung eine mehrgipfelige Verteilung beobachtet und die Maxima fraktioniert wurden, entspricht jeweils die Fraktion 1 hauptsächlich der monomeren-Bande, während die Spuren, auf die die Fraktionen 2 aufgetragen wurden, hauptsächlich eine Intensitätsverteilung über einen weiten Bereich oberhalb der monomeren-Bande zeigen. Dabei ähneln sich die Intensitätsverteilungen von MS und GS, die ungefähr ausgehend von der monomeren-Bande bis zu einer Laufhöhe entsprechend 500 bp reichen. Bei GL dagegen beginnt diese oberhalb von 200 bp und reicht bis zur Tasche des Gels. Bei GLS hingegen ist ausgehend von der monomeren-Bande eine Verteilung der DNA über die gesamte Spur zu beobachten.

Abbildung 3.1.6: Thermische Denaturierung verschiedener monomerer ud multimerer Moleküle

Native PAGE 8% Gel (2,6% C), Auftragungsmenge 0,25 μg, Marker 25 bp 0,3 μg (25 bp, unbehandelt, Denaturierung 95°C 10 min: 30L1, MS, ML, KG, GS, GL-F2, GLS-F2)

Auf dem Gel in Abbildung 3.1.6 ist zu sehen, wie sich die thermische Denaturierung der verschiedenen Moleküle auf deren Laufverhalten im Gel auswirkt. Dazu wurden die Ansätze geteilt und ein Teil für 10 min auf 95°C erhitzt und sofort auf Eis gekühlt und aufgetragen. Die zu beobachtenden Banden entsprechen denen im unter 3.1.5 beschriebenen Gel, jedoch ist hier auch bei MS und GS nicht-aufgetrennte DNA in der Tasche des Gels sichtbar und bei MS ist eine zweite Bande zu sehen, die auf gleicher Höhe mit der zweiten Bande von 30L1 läuft. Bei GS und MS handelt es sich um eine andere Produktionscharge als in dem in Abbildung 3.1.5 gezeigten Gel, bei der die Nebenmaxima bei der HPLC-Fraktionierung nicht getrennt gesammelt wurden. Der Vergleich der Banden im Gel von denaturierten mit nicht-denaturierten dSLIM zeigt, dass nach Erhitzung bei allen Molekülen die Intensität der 1. Bande verringert und die der 2. Bande verstärkt ist. Die in den Spuren ML und KG auftretende Bande im oberen Drittel des Gels ist nach Denaturierung nicht mehr zu sehen. Auch die DNA in den Taschen der Spuren, auf die MS und GS aufgetragen wurden, ist nach Denaturierung nicht mehr erkennbar. In den Spuren GL-F2 und GLS-F2 ist die Menge der DNA in der Tasche ebenfalls vermindert, dafür wird eine größere Anzahl regelmäßig angeordneter Banden oberhalb der 2. Bande sichtbar.

Um zu überprüfen, ob die durch die Denaturierung verursachte Veränderung reversibel ist, wurden die denaturierten Proben geteilt und jeweils für 3 Tage bei 4°C bzw. 37°C inkubiert. Die unbehandelten dSLIM-Ansätze wurden bei 4°C aufbewahrt und nach 3 Tagen ebenfalls aufgeteilt und je ein Aliquot für 10 min bei 95°C erhitzt und sofort auf Eis gestellt. Die Ergebnisse der nativen PAGE, mit der die Ansätze aufgetrennt wurden, sind in Abbildung 3.1.7 und 3.1.8 dargestellt. Die unbehandelten und denaturierten Proben zeigen im Gel dasselbe Laufverhalten, wie es für das in Abbildung 3.1.6 dargestellte Gel beschrieben wurde. Die denaturierten Proben, die bei 4°C inkubiert wurden, entsprechen in ihrem Laufverhalten den direkt vor Auftragung denaturierten Proben. Die bei 37°C inkubierten Proben zeigen ein Laufverhalten im Gel, das dem der unbehandelten Proben weitgehend entspricht. Jedoch ist die Intensität der zweiten Bande bei 30L1, MS und GS größer als bei diesen. Ein Unterschied besteht auch in der Menge der in der Tasche des Gels verbliebenen DNA bei GS und MS, die im Vergleich zur unbehandelten Probe nur noch sehr gering ist.

Dimeres Molekül und monomere 30L1 Fraktionen

Um zu überprüfen, ob es sich bei der im oberen Drittel zu erkennenden Bande, um eine einem Dimer entsprechende Bande handelt, wurde eine Fraktion (F3), die aus einer großen Menge monomere Moleküle durch HPLC-Trennung gewonnen wurde und diese Bande repräsentiert, zusammen mit dem Dimer Molekül 60L1 auf ein native PAGE aufgetragen.

Abbildung 3.1.9: Vergleich Fraktion 3 mit Dimer-Molekül 60L1

Native PAGE 8% Gel (5% C), Auftragungsmenge 0,25 μg, Marker 25 bp 0,3 μg (25 bp, F3, F3 95°C, 60L1, 60L1 95°C, Fraktion 1 (F1), Fraktion 2 (F2), Fraktion 3 (F3), Fraktion 4 (F4) aus HPLC-Aufreinigung von dSLIM30L1, 60L1)

Die beobachteten Banden laufen auf gleicher Höhe. Werden die Proben denaturiert auf ein Gel aufgetragen, so finden sich die Banden im Vergleich zu einer Spur, auf die unbehandelte Proben aufgetragen wurden, weiter oberhalb im Gel. Die entsprechenden Spuren der nativen PAGE sind in Abbildung 3.1.9 zusammen mit dem Ergebnis der Auftrennung der vier verschiedenen durch HPLC-Fraktionierung erhaltenen Fraktionen von Monomer 30L1 dargestellt.

Inkubation Medium, Proteinbindung

Um zu untersuchen, inwieweit sich die Bedingungen in der Zellkultur, insbesondere die Anwesenheit von Proteinen, auf die für die Stimulation verwendeten Moleküle auswirken, wurden diese in Zellkulturmedium für 0, 5 und 27 h bei 37°C inkubiert und neben den in Wasser gelösten Molekülen mittels nativer PAGE separiert. Die im Medium befindlichen Proteine, wurden im Gel anschließend an die DNA-Färbung mit Hilfe von Coomassie-Färbung nachgewiesen. Die Ergebnisse sind in Abbildung 3.1.10. und 3.1.11 dargestellt.

Abbildung 3.1.10: Inkubation von monomeren Molekülen in Medium

Native PAGE 8% Gel (5% C), Auftragungsmenge 0,25 μg, Marker 25 bp 0,3 μg, Marker 50 bp 0,3 μg (50 bp, 27 h Medium, 5 h Medium, 0 h Medium, $H_20$: 30L1, ML, F3, KG, 25 bp)

Native PAGE 8% Gel (2,6% C), Auftragungsmenge 0,25 μg, 25 bp 0,35 μg (27 h Medium, 5 h Medium, 0 h Medium, $H_20$: GL, 25 bp)

Ausschnitt Coomassie-Färbung native 8% PAGE (5% C) von Abbildung 3.1.11 a) (27 h Medium, 5 h Medium, 0 h Medium, $H_20$: ML)

Abbildung 3.1.10 a u. 3.1.10 b zeigen die mit Ethidiumbromid gefärbten PAGE, auf welche die Moleküle aufgetragen wurden. In Abbildung 3.1.10 c ist ein Ausschnitt aus dem Coomassie-gefärbte Gel zum Vergleich der Laufweiten exemplarisch dargestellt. Der Vergleich der Spuren, mit den unterschiedlichen Ansätzen, zeigt, dass bei allen Molekülen in Medium eine Bande in der Geltasche auftritt, die in den $H_2O$ Spuren nicht vorhanden ist (mit Ausnahme von GL). Die im oberen Bereich des Gels in $H_2O$ bei 30L1 und der Fraktion 3 auftretenden Bande, weist in Medium eine geringere Laufweite auf, die auf der Höhe der untersten Proteinbande liegt. Die Intensität sowohl der DNA- als auch der Protein-Banden nimmt mit zunehmender Inkubationsdauer ab. Bei GL nimmt die Intensität der 2. Bande in Medium im Vergleich zu $H_2O$ zu.

Abbildung 3.1.11: Inkubation von ODN M362 in Medium

Native PAGE 12% Gel (5% C), Auftragungsmenge 0,25 μg, Marker 25 bp 0,3 μg (25 bp, $H_2O$, 0 h Medium, 5 h Medium, 27 h Medium)

Ausschnitt Coomassie-Färbung native PAGE 12% Gel (5% C) von Abbildung 3.1.12 a) ($H_2O$, 0 h Medium, 5 h Medium, 27 h Medium)

Abbildung 3.1.11 zeigt das native PAGE auf dem das Phosphorothioat-geschützte Kontrollmolekül M362 aufgetragen wurde. Hier ist deutlich zu sehen, das die in $H_2O$ am unteren Rand des Gels sichtbare DNA-Bande in Medium verschwunden ist, Stattdessen sind jedoch eine Bande am oberen Rand des Gels, die in der Laufweite mit der unteren Proteinbande (Abbildung 3.1.11 b) übereinstimmt sowie eine Bande in den Geltaschen zu sehen. Auch hier ist eine Abnahme der Intensität der DNA-Banden mit zunehmender Inkubationszeit zu beobachten.

Um zu verifizieren, dass die beobachteten Änderungen durch die Anwesenheit von Proteinen und nicht durch andere Bestandteile des Mediums verursacht wurden, wurden die Moleküle zum Vergleich auch in Medium ohne Zusatz von FCS verdünnt. In Abbildung 3.1.12 sind die entsprechenden Spuren der Gele exemplarisch für dSLIM (KG) und M362 dargestellt. In den Spuren, auf denen die ODN und die Moleküle in Medium ohne FCS aufgetrennt wurden entspricht die Intensitätsverteilung denen, auf die ODN und dSLIM in Wasser gelöst aufgetragen wurden. Die in Medium mit FCS gelösten ODN und die Moleküle zeigen dagegen ein Laufverhalten in der nativen PAGE, das dem für Abbildung 3.1.10 und. 3.1.11 für die Spuren, auf die in Medium verdünnte monomere Moleküle und ODN aufgetragen wurden, beschriebenen entspricht.

Immstimulatorische Wirkung: Stimulation PBMC

Die immunmodulatorische Wirkung der verschiedenen Moleküle wurde an PBMC untersucht, die aus humanen Blutspenden isoliert wurden. Dazu wurden die PBMC mit dem jeweiligen Molekül oder dem Kontrollmolekül M362 in einer Endkonzentration von 1 μM für 48 h inkubiert und die Menge an IL-6, IFN-alpha und IFN-gamma in den Zellkulturüberständen mit Hilfe von ELISA gemessen. Als Kontrolle dienten Zellen, die ohne Zusätze inkubiert wurden.

Die ELISA-Ergebnisse von PBMC-Überständen, die aus 4 Blutspenden (Spender A-D) isoliert wurden, sind in den Diagrammen in Abbildung 3.2.1 a-c dargestellt. Die Werte entsprechen jeweils den Mittelwerten aus Doppelbestimmungen und die Fehlerbalken repräsentieren die daraus ermittelte zweifache Standardabweichung. Für die mit * gekennzeichneten Werte wurden zusätzlich Doppelbestimmungen bei der Stimulation der PBMC durchgeführt und die angegebenen Werte entsprechen den Mittelwerten aus den erhaltenen vier ELISA-Ergebnissen. Die Fehlerbalken entsprechen dem Doppelten der Standardabweichung dieser vier Werte.

Abbildung 3.2.1 a-c: Ergebnisse ELISA IFN-gamma (a), IFN-alpha (b), IL-6 (c) PBMC

Überstände PBMC (2,4 x 106 Zellen pro Ansatz in 600 μL) verschiedener Spender A-D, Stimulation 48 h mit 1 μM dSLIM/ODN M362, Standardmeßbereich **--,** * Doppelbest Zellkultur, Fehlerbalken: 2 x Standardabweichung aus Doppelbestimmung ELISA (* Doppelbest. Zellkultur + Doppelbest. ELISA = 4 Werte)

Bei Betrachtung der Diagramme fällt zunächst auf, dass sich die Cytokin-Konzentrationen in den Überständen der verschiedenen Spender stark unterscheiden, wodurch ein Vergleich der Daten erschwert wird. Die Standardabweichungen der zusätzlich in der Zellkultur als Doppelbestimmung ermittelten Werte sind vergleichbar mit den Standardabweichungen, die lediglich aus der Doppelbestimmung im ELISA ermittelt wurden, was darauf hindeutet, dass die Unterschiede auf Verschiedenheiten der PBMC zurückzuführen sind. In den unstimulierten Ansätzen wurden bei allen Spendern keine oder nur sehr geringe Cytokin-Konzentrationen nachgewiesen. Bei der Bestimmung der Konzentration von IL-6 und IFN-gamma liegen einige ermittelte Werte am oberen Rand bzw. außerhalb des Standardmessbereiches, der bei IFN-gamma bis 10.000 pg/mL und bei IL-6 für DSLIM bis 3500 pg/mL und für M362 bis 7000 pg/mL reichte.

Um eine bessere Vergleichbarkeit der Daten der verschiedenen Spender zu ermöglichen, wurden die erhaltenen Cytokin-Werte normiert. Dazu wurde der prozentuale Anteil der bestimmten Cytokin-Konzentrationen am für M362 ermittelten Wert für jeden Spender berechnet. Die Mittelwerte der so normierten Daten der vier Spender sind in den Diagrammen in Abbildung 3.2.2 a-c wiedergegeben. Die Fehlerbalken entsprechen der jeweiligen Mittelwertabweichung. Wegen der großen Unterschiede, die zwischen den einzelnen Spendern beobachtet wurden, sind diese sehr groß. Die Darstellung ermöglicht aber zumindest die Abschätzung einer Tendenz für die einzelnen Moleküle.

Abbildung 3.2.2 a-c: Normierte Mittelwerte der Ergebnisse ELISA IFN-gamma (a), IFN-alpha (b), IL-6 (c)

Ergebnisse ELISA s. Abb. 3.2.1 normiert: Cytokin-Menge in % von M362 je Spender, Mittelwerte aus 4 Spendern, Fehlerbalken: Mittelwertabweichung.

Für IFN-gamma lagen die berechneten Werte für die getesteten Moleküle zwischen 40% und 80% bezogen auf die Werte von M362, wobei die Werte für KG, ML und GLS-F2 eher im unteren und für GL-F2 eher im oberen Bereich lagen.

Die prozentualen Mittelwerte der verschiedenen Konstrukte für IFN-alpha entsprachen in etwa denen von M362, mit Ausnahme der für GL-F1, GLS-F1 und GL-F2 berechneten Werte, die nur ca. 50% derer von M362 betrugen.

Die Mittelwerte der prozentualen Anteile an M362 für It_-6 lagen für 30L1, MS, GS, GL-F2 und GLS-F2 unter 50%, wobei MS und GLS-F2, die Konstrukte mit der niedrigsten Endotoxin-Konzentration, auch die niedrigsten Werte aufwiesen. Leicht über 50% bezogen auf M362 lagen die Werte für GL-F1 und GL-F2, für die keine Endotoxinwerte vorliegen. Die höchsten IL-6 Werte, die denen von M362 entsprechen bzw. über 100% liegen, wiesen die mit KG und ML stimulierten Zellen auf. Bei diesen beiden Konstrukten wurde auch die höchste Endotoxin-Konzentration gemessen.

Abbildung 3.3.2: Ergebnisse ELISA IL-8 HEK293-TLR9 Stimulationsdauer

Überstände HEK293-TLR9: 400 μL/Ansatz (5 x $10^5$ Zellen/mL); Stimulation 6 h, 24 h, 48 h mit 2,5 μM ODN 2006; 0,5 μg/ml LPS; Stimulation 3 Tage nach Aussäen der Zellen (Mediumwechsel nach 48 h und vor Stimulation); Fehlerbalken: 2 x Standardabweichung aus Doppelbestimmung ELISA.

Verhältnisse der Chemokin-Mengen stimulierte zu unstimulierten Ansätzen aus a); Fehlerbalken: Summe relative Fehler aus Doppelbestimmung ELISA.

Die gemessenen Chemokin-Konzentrationen sind relativ gering. Es kann jedoch eine Zunahme der IL-8/CXCL8 Menge mit steigender Inkubationsdauer in allen Ansätzen beobachtet werden, die jedoch von 24 h zu 48 h nur noch sehr gering ausfällt. Auch hier sind die jeweiligen Werte für LPS stimulierte und unstimulierte Zellen in etwa gleich groß und die für Zellen, die mit ODN 2006 stimuliert wurden sind im Vergleich zu den unstimulierten Zellen um den Faktor 1,5 erhöht. Bei den Verhältnissen der zu den verschiedenen Zeitpunkten bestimmten IL-8/CXCL8-Menge von stimulierten zu unstimulierten Ansätzen ist kein Unterschied erkennbar. Daher erfolgte im Weiteren die Stimulation für 24 h.

Abbildung 3.3.4: Ergebnisse ELISA IL-8 HEK293-Null verschiedener monomerer Moleküle.

Überstände HEK293-Null: 400 μL/Ansatz (1 x $10^6$ Zellen/mL); Stimulation 24 h mit 2 μM ODN 2006/dSLIM; 0,5 μg/ml LPS; Stimulation 4 Tage nach Aussäen der Zellen (Mediumwechsel nach 48 h und vor Stimulation); Fehlerbalken: 2 x Standardabweichung aus Doppelbestimmung ELISA.

Verhältnisse der Chemokin-Mengen stimulierte zu unstimulierten Ansätzen aus a); Fehlerbalken: Summe relative Fehler aus Doppelbestimmung ELISA.

Es kann bei keinem der mit den verschiedenen Stimulanzien inkubierten Ansätze ein signifikanter Unterschied der IL-8/CXCL8-Sekretion im Vergleich zum unstimulierten Ansatz beobachtet werden.

## Patentansprüche

1. Multimeres Molekül zur Modulation der Aktivität des menschlichen oder tierischen Immunsystems herstellbar durch ein Verfahren was folgende Schritte umfasst:

   - Bereitstellung einer 5-phosphorylierten Oligodesoxyribonukleinsäuresequenz in Wasser,
   - Lyophylisierung bis ein trockener Rückstand erhalten wird und anschließend Resuspension in einer Pufferlösung,
   - Zugabe einer T4-DNAligase wodurch ein Reaktionsgemisch erhalten wird und
   - Inkubation des Reaktionsgemisches bei 37˚C für mindestens 30 Minuten.

**2.** Molekül nach Anspruch 1, **dadurch gekennzeichnet, dass** die Oligodesoxyribonukleotidsequenz die folgenden Sequenzen umfasst:

a) GTTCCTGGAG ACGTTCTTAG GAACGTTCTC CTTGACGTTG GAGAGAAC oder
b) ACCTTCCTTG TACTAACGTT GCCTCAAGGA AGGTTGATCT TCATAACGTT GCCTAGATCA, oder
c) eine Oligodesoxyribonukleinsäuresequenz der Basenfolge AACG TTCTTCGGGG CGTT umfasst,
d) und wobei die Oligodesoxyribonukleinsäuresequenz eine Länge von 40 bis 1.600 Nukleotide aufweist.

**3.** Molekül nach dem vorhergehenden Anspruch, **dadurch gekennzeichnet, dass** die Basenfolge gemäß Merkmal c) in der Sequenz CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC enthalten ist.

**4.** Molekül nach einem der vorhergehenden Ansprüche, **dadurch gekennzeichnet, dass** das Molekül eine teilweise einsträngige, kovalent geschlossene Kette von Desoxyribonukleosidresten umfasst.

**5.** Molekül nach einem der vorgehenden Ansprüche, **dadurch gekennzeichnet, dass** das Molekül die Basenfolge $N^1N^2CGN^3N^4$ umfasst, wobei $N^1N^2$ ein Element aus der Gruppe GT, GG, GA, AT oder AA, $N^3N^4$ ein Element aus der Gruppe CT oder TT, sowie C Desoxycytosin, G Desoxyguanosin, A Desoxyadenosin und T Desoxythymidin ist.

**6.** Molekül nach einem der vorgehenden Ansprüche, **dadurch gekennzeichnet, dass** die Basenfolge $N^1N^2CGN^3N^4$ in dem einsträngigen Bereich der geschlossenen Kette von Desoxyribonukleosidresten positioniert ist.

**7.** Molekül nach einem der vorherigen Ansprüche, **dadurch gekennzeichnet, dass** ein oder mehrere Substituenten durch kovalente Bindungen mit dem Molekül verknüpft sind.

**8.** Molekül nach Anspruch 7, wobei der Substituent ausgewählt ist, aus einer der Gruppen umfassend Peptide, Proteine, Sacchariden, antigenen Strukturen, DNA und/oder RNA.

**9.** Kombinationsmittel **dadurch gekennzeichnet, dass** es ein Molekül gemäß einem der Ansprüche 1 bis 6 und ein Chemotherapeutikum umfasst.

**10.** Kombinationsmittel nach einem der vorhergehenden Ansprüchen, **dadurch gekennzeichnet, dass** das Chemotherapeutikum ausgewählt ist aus der Gruppe umfassend Antikörper, Alkylantien, Platinanaloga, Interkalentien, Antibiotika, Mitosehemmer, Taxane, Topoisomerasehemmer, Antimetabolite und/oder L-Asparaginase, Hydroxycarbamid, Mitotane und/oder Amanitine.

**11.** Kombinationsmittel nach einem der vorhergehenden Ansprüchen, **dadurch gekennzeichnet, dass** die Alkylantien ausgewählt sind aus der Gruppe umfassend:

- Stickstoff-Lost-Derivate, insbesondere
  - Cyclophosphamid
  - Ifosfamid,
  - Trofosfamid,
  - Melphalan und/oder
  - Chlorambucil
- Akylsulfonate, insbesondere
  - Busulfan und/oder
  - Treosulfan
- Nitrosoharnstoffe, insbesondere
  - Carmustin,
  - Lomustin,
  - Nimustin,
  - Estramustin und/oder

- Streptozotocin

- Procarbazin und Dacarbazin,
- Temozolomid und/oder
- Thiotepa

und/oder, dass die Platinanaloga ausgewählt sind aus der Gruppe umfassend:

- Cisplatin,
- Carboplatin und/oder
- Oxaliplatin

und/oder, dass die Interkalentien ausgewählt sind aus der Gruppe umfassend:

- Anthracycline, insbesondere
    - Doxorubicin (Adriamycin),
    - Daunorubicin,
    - Epirubicin und/oder
    - Idarubicin,
- Mitoxantron,
- Amsacrin und/oder
- Doxifluridin

und/oder, dass die Antibiotika ausgewählt sind aus der Gruppe umfassend:

- Bleomycin,
- Actinomycin D (Dactinomycin) und/oder
- Mitomycin

und/oder, dass die Mitosehemmer ausgewählt sind aus der Gruppe umfassend:

- Alkaloide der Vinca rosea, insbesondere
    - Vinorelbin,
    - Vincristin (Oncovin),
    - Vinblastin und/oder
    - Vindesin

und/oder, dass die Taxane ausgewählt sind aus der Gruppe umfassend:

- Paclitaxel und/oder
- Docetaxel

und/oder, dass die Topoisomerasehemmer ausgewählt sind aus der Gruppe umfassend:

- Topoisomerase-I-Inhibitoren, insbesondere
    - Camptothecin,
    - Topotecan und/oder
    - Irinotecan und/oder
- Topoisomerase- II- Inhibitoren, insbesondere
    - Etoposid
    - Teniposid

und/oder, dass die Antimetabolite ausgewählt sind aus der Gruppe umfassend:

- Folsäureantagonist, insbesondere
    - Methotrexat,
- Pyrimidianaloga, insbesondere
    - 5-Fluorouracil,
    - Capecitabin,
    - Cytosinarabinosid (Cytarabin) und/oder
    - Gemcitabin,
- Purinanaloga, insbesondere
    - 6-Thioguanin,
    - Pentostatin,
    - Azathioprin,
    - 6-Mercaptopurin,
    - Fludarabin und/oder
    - Cladribin.

**12.** Molekül nach einem der Ansprüche 1 bis 8 und/oder Kombinationsmittel nach einem der Ansprüche 9 bis 11 zur Verwendung als Arzneimittel.

**13.** Verwendung eines Moleküls gemäß der Ansprüche 1 bis 8 und/oder eines Kombinationsmittel gemäß einem der Ansprüche 9 bis 11, zur Herstellung eines Mittels zur Modulation eines menschlichen oder tierischen Immunsystems oder zur Modulation der Aktivität des Immunsystems, bevorzugt ist die Modulation eine Stimulierung oder eine Steigerung der Aktivität des Immunsystems.

**14.** Verwendung nach dem vorhergehenden Anspruch, **dadurch gekennzeichnet, dass** die Stimulierung eine T-Zell vermittelte oder -unabhängige Immunantwort umfasst, bevorzugt umfasst die Immunantwort eine Proliferation von B-Zellen und/oder eine B-Zell-Aktivierung.

**15.** Verwendung nach einem der vorhergehenden Ansprüche, **dadurch gekennzeichnet, dass** die Stimulierung des Immunsystems eine Sekretion von Zytokinen umfasst.

**16.** Verwendung nach einem der vorhergehenden Ansprüche, **dadurch gekennzeichnet, dass** das Molekül gemäß einem der Ansprüche 1 bis 8 und/oder das Kombinationsmittel gemäß einem der Ansprüche 9 bis 11 als Adjuvanz in der therapeutischen oder prophylaktischen Vakzinierung eingesetzt wird.

**17.** Verwendung eines Moleküls gemäß einem der Ansprüche 1 bis 8 und/oder eines Kombinationsmittels gemäß einem der Ansprüche 9 bis 11 zur Herstellung eines Mittels zur Behandlung einer Zellwachstumsstörung, bevorzugt ist die Zellwachstumsstörung eine Tumorerkrankung.

## Claims

**1.** A multimeric molecule for modulating the activity of the human or animal immune system, producible by a method comprising the following steps:

- providing a 5'-phosphorylated oligodeoxyribonucleic acid sequence in water,
- lyophilizing until a dry residue is obtained, followed by resuspension in a buffer solution,
- adding a T4 DNA ligase, thereby forming a reaction mixture, and
- incubating the reaction mixture at 37˚C for at least 30 minutes.

**2.** The molecule according to claim 1, **characterized in that** the oligodeoxyribonucleotide sequence comprises the following sequences:

a) GTTCCTGGAG ACGTTCTTAG GAACGTTCTC CTTGACGTTG GAGAGAAC or
b) ACCTTCCTTG TACTAACGTT GCCTCAAGGA AGGTTGATCT TCATAACGTT GCCTAGATCA, or
c) comprises an oligodeoxyribonucleic acid sequence having the base sequence AACG TTCTTCGGGG CGTT,
d) and wherein said oligodeoxyribonucleic acid sequence has a length of 40 to 1600 nucleotides.

**3.** The molecule according to the preceding claim, **characterized in that** the base sequence according to feature c) is comprised in the sequence CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC.

**4.** The molecule according to any of the preceding claims, **characterized in that** the molecule comprises a partially single-stranded, covalently closed chain of deoxyribonucleoside residues.

**5.** The molecule according to any of the preceding claims, **characterized in that** the molecule comprises the base sequence $N^1N^2CGNY$ wherein $N^1N^2$ is an element from the group of GT, GG, GA, AT or AA, $N^3N^4$ is an element from the group of CT or TT, and C is deoxycytidine, G is deoxyguanosine, A is deoxyadenosine, and T is deoxythymidine.

**6.** The molecule according to any of the preceding claims, **characterized in that** the base sequence $N^1N^2CGN^3N^4$ is positioned within the single-stranded region of the closed chain of deoxyribonucleoside residues.

**7.** The molecule according to any of the preceding claims, **characterized in that** one or more substituents are linked to the molecule via covalent bonds.

**8.** The molecule according to claim 7, wherein the substituent is selected from one of the groups comprising peptides, proteins, saccharides, antigenic structures, DNA and/or RNA.

**9.** A composition, **characterized in that** it comprises a molecule according to any of claims 1 to 6 and a chemotherapeutic.

**10.** The composition according to any of the preceding claims, **characterized in that** the chemotherapeutic is selected from the group comprising antibodies, alkylating agents, platinum analogs, intercalating agents, antibiotics, mitosis inhibitors, taxanes, topoisomerase inhibitors, antimetabolites and/or L-asparaginase, hydroxycarbamide, mitotane and/or amanitins.

**11.** The composition according to any of the preceding claims, **characterized in that** the alkylating agents are selected from the group comprising:

- nitrogen mustard derivatives, especially
    - cyclophosphamide,
    - ifosfamide,
    - trofosfamide,
    - melphalan and/or
    - chlorambucil,
- alkylsulfonates, especially
    - busulfan and/or
    - treosulfan
- nitrosoureas, especially
    - carmustine,
    - lomustine,
    - nimustine,
    - estramustine and/or
    - streptozotocin,

- procarbazine and dacarbazine,
- temozolomide and/or
- thiotepa

and/or that platinum analogs are selected from the group comprising:

- cisplatin,
- carboplatin and/or
- oxaliplatin

and/or that the intercalating agents are selected from the group comprising:

- anthracyclines, especially
    - doxorubicin (adriamycin),
    - daunorubicin,
    - epirubicin and/or
    - idarubicin,
- mitoxantrone,
- amsacrin and/or
- doxifluridine

and/or that the antibiotics are selected from the group comprising:

- bleomycin,
- actinomycin D (dactinomycin) and/or
- mitomycin

and/or that the mitosis inhibitors are selected from the group comprising:

- alkaloids of Vinca rosea, especially
    - vinorelbine,
    - vincristine (oncovin),
    - vinblastine and/or
    - vindesine

and/or that the taxanes are selected from the group comprising:

- paclitaxel and/or
- docetaxel

and/or that the topoisomerase inhibitors are selected from the group comprising:

- topoisomerase I inhibitors, especially
    - camptothecin,
    - topotecane and/or
    - irinotecane and/or
- topoisomerase II inhibitors, especially
    - etoposide
    - teniposide

and/or that the antimetabolites are selected from the group comprising:

- folic acid antagonist, especially

    - methotrexate,

- pyrimidine analogs, especially

    - 5-fluorouracil,
    - capecitabine,
    - cytosine arabinoside (cytarabine) and/or
    - gemcitabine,

- purine analogs, especially

    - 6-thioguanine,
    - pentostatine,
    - azathioprine,
    - 6-mercaptopurine,
    - fludarabine and/or
    - cladribine.

**12.** The molecule according to any of claims 1 to 8 and/or the composition according to any of claims 9 to 11 for use as a pharmaceutical.

**13.** Use of a molecule according to any of claims 1 to 8 and/or of a composition according to any of claims 9 to 11 for the production of an agent for modulating a human or animal immune system or modulating the activity of the immune system, the modulation being preferably a stimulation or an enhancement of the activity of the immune system.

**14.** The use according to the preceding claim, **characterized in that** the stimulation comprises a T cell-mediated or T cell-independent immune response, the immune response preferably comprising a proliferation of B cells and/or B cell activation.

**15.** The use according to any of the preceding claims, **characterized in that** the stimulation of the immune system comprises secretion of cytokines.

**16.** The use according to any of the preceding claims, **characterized in that** the molecule according to any of claims 1 to 8 and/or the composition according to any of claims 9 to 11 are used as adjuvant in therapeutic or prophylactic vaccination.

**17.** Use of a molecule according to any of claims 1 to 8 and/or of a composition according to any of claims 9 to 11 for the production of an agent for the treatment of a cell growth disorder, the cell growth disorder being preferably a tumor disease.

## Revendications

**1.** Molécule multimère pour la modulation de l’activité du système immunitaire humain ou animal, fabricable par un procédé comprenant les étapes consistant à :

- préparer une séquence d’acide oligodésoxyribonucléique 5 fois phosphorylisé dans l’eau,
- lyophiliser jusqu’à obtention d’un état sec, suivi d’une resuspension dans une solution tampon,
- ajouter une ligase T4-DAN, un mélange de réaction étant ainsi obtenu, et
- incuber le mélange de réaction à 37˚C pendant au moins 30 minutes.

**2.** Molécule selon la revendication 1, **caractérisée en ce que** la séquence d’acide oligodésoxyribonucléique comprend les séquences suivantes:

a) GTTCCTGGAG ACGTTCTTAG GAACGTTCTC CTTGACGTTG GAGAGAAC ou

b) ACCTTCCTTG TACTAACGTT GCCTCAAGGA AGGTTGATCT TCATAACGTT GCCTAGATCA, ou
c) une séquence d'acide oligodésoxyribonucléique comprenant la séquence de bases AACG TTCTTCGGGG CGTT,
d) et la séquence d'acide oligodésoxyribonucléique ayant une longueur de 40 à 1.600 nucléotides.

**3.** Molécule selon la revendication précédente, **caractérisée en ce que** la séquence de bases selon la caractéristique c est comprise dans la séquence CCTAGGGGTT ACCACCTTCA TTGGAAAACG TTCTTCGGGG CGTTCTTAGG TGGTAACC CCTAGGGGTT ACCACCTTCA TTGGAAAACG TTCTTCGGGG CGTTCTTAGG TGGTAACC.

**4.** Molécule selon l'une quelconque des revendications précédentes, **caractérisée en ce que** la molécule comprend une chaîne fermée de manière covalente, en partie simple brin, de résidus de désoxyribonucléoside.

**5.** Molécule selon l'une quelconque des revendications précédentes, **caractérisée en ce que** la molécule comprend la suite de bases $N^1N^2CGN^3N^4$, dans laquelle $N^1N^2$ est un élément choisi dans le groupe constitué par GT, GG, GA, AT et AA, $N^3N^4$ un élément choisi dans le groupe constitué par CT et TT, et C est désoxycytosine, G désoxyguanosine, A désoxyadenosine et T désoxythymidine.

**6.** Molécule selon l'une des revendications précédentes, **caractérisée en ce que** la suite de bases $N^1N^2CGN^3N^4$ est positionnée dans la région simple brin de la chaîne fermée de résidus de désoxyribéonucléonucléoside.

**7.** Molécule selon l'une quelconque des revendications précédentes, **caractérisée en ce que** un ou plusieurs substituants sont liés avec la molécule par des liaisons covalentes.

**8.** Molécule selon la revendication 7, dans laquelle le substituant est choisi dans le groupe constitué par : peptides, protéines, saccharides, structures antigènes, ADN et/ou ARN.

**9.** Composition, **caractérisée en ce qu'**elle comprend une molécule selon l'une des revendications 1 à 6 et un médicament chimique.

**10.** Composition selon l'une quelconque des revendications précédentes, **caractérisé en ce que** le médicament chimique est choisi dans le groupe constitué par les anticorps, agents alkylants, analogues du platine, agents intercalants, antibiotiques, inhibiteurs de mytose, taxane, inhibiteurs de topo-isomérase, anti-métabolites et/ou L-asparaginase, hydroxycarbamide, mitotane et/ou amanitines.

**11.** Composition selon l'une quelconque des revendications précédentes, **caractérisé en ce que** les agents alkylants sont choisis dans le groupe constitué par :

- dérivés de la moutarde à l'azote, en particulier
    - cyclophosphamide,
    - ifosfamide,
    - trofosfamide,
    - melphalane et/ou
    - chlorambucile
- alkylsulfonates, en particulier
    - busulfane et/ou
    - tréosulfane
- urées azotées, en particulier
    - carmustine,
    - lomustine,
    - nimustine,
    - estramustine et/ou streptozotocine
    - procarbazine et dacarbazine,
    - témozolomide et/ou

- thiotepa

et/ou, **en ce que** les analogues de platine sont choisis dans le groupe constitué par :

- Cisplatine,
- Carboplatine et/ou
- Oxaliplatine

et/ou **en ce que** les agents intercalants sont choisis dans le groupe constitué par :

- anthracyclines, en particulier
  - doxorubicine (Adriamycine),
  - daunorubicine,
  - épirubicine et/ou
  - Idarubicine,
- mitoxantrone,
- amsacrine et/ou
- doxifluridine

et/ou **en ce que** les antibiotiques sont choisis dans le groupe constitué par :

- bléomycine,
- actinomycine D (dactinomycine) et/ou
- mitomycine

et/ou, **en ce que** les inhibiteurs de mytose sont choisis dans le groupe constitué par :

- alcaloïde de Vinca rosea, en particulier
  - vinorelbine,
  - vincristine (oncovine),
  - vinblastine et/ou
  - vindesine

et/ou, **en ce que** les taxanes sont choisis dans le groupe constitué par :

- paclitaxel et/ou
- docétaxel

et/ou **en ce que** les inhibiteurs de topo-isomérase sont choisis dans le groupe constitué par :

- inhibiteurs de topo-isomérase-1, en particulier
  - camptothécine,
  - topotécane et/ou
  - irinotécane et/ou
- inhibiteurs de topo-isomérase-II, en particulier
  - étoposide
  - teniposide

et/ou, **en ce que** les anti-métabolites sont choisis dans le groupe constitué par :

- antagonistes de l'acide folique, en particulier

- méthotréxate,

- analogues de la pyrimidine, en particulier

  - 5-fluorouracile,
  - capécitabine,
  - cytosine arabinoside (cytarabine) et/ou
  - gemcitabine,

- analogues de la purine, en particulier

  - 6-thioguanine,
  - pentostatine,
  - azathioprine,
  - 6-Mmercaptopurine,
  - fludarabine et/ou
  - cladribine.

**12.** Molécule selon l'une quelconque des revendications 1 à 8 et/ou composition selon l'une des revendications 9 à 11 pour utilisation en tant que médicament.

**13.** Utilisation d'une molécule selon l'une quelconque des revendications 1 à 8 et/ou d'une composition selon l'une quelconque des revendications 9 à 11, pour la fabrication d'un agent de modulation d'un système immunitaire humain ou animal ou pour la modulation de l'activité du système immunitaire, la modulation étant de préférence une stimulation ou une augmentation de l'activité du système immunitaire.

**14.** Utilisation selon la revendication précédente, **caractérisée en ce que** la stimulation comprend une réponse immunitaire indépendante des cellules T ou facilitée par les cellules T, la réponse immunitaire comprenant en particulier une prolifération de cellules B et/ou une activation de cellule B.

**15.** Utilisation selon l'une quelconque des revendications précédentes, **caractérisée en ce que** la stimulation du système immunitaire comprend une sécrétion de cytokines.

**16.** Utilisation selon l'une quelconque des revendications précédentes, **caractérisée en ce que** la molécule selon l'une quelconque des revendications 1 à 8 et/ou la composition selon l'une quelconque des revendications 9 à 11 est/sont employé(es) en tant qu'adjuvant dans une vaccination thérapeutique ou prophylactique.

**17.** Utilisation d'une molécule selon l'une quelconque des revendications 1 à 8 et/ou d'une composition selon l'une quelconque des revendications 9 à 11, pour la fabrication d'un agent de traitement d'un trouble de croissance cellulaire, le trouble de croissance cellulaire étant de préférence une maladie tumorale.

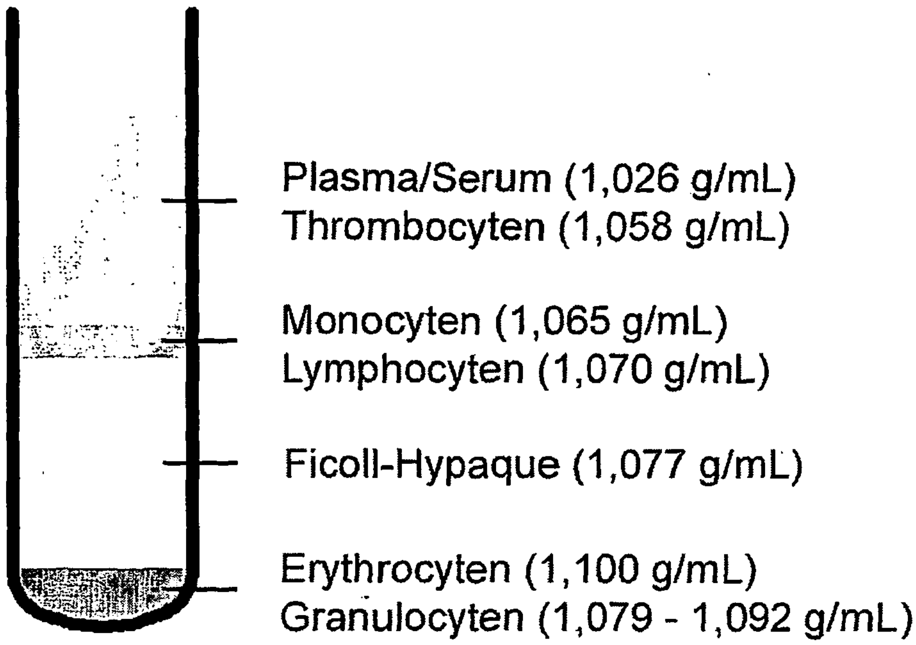


Abbildung 2.5.1: Ficoll-Hypaque Dichtegradientenzentrifugation zur Isolation von PBMC nach [Luttmann 2004

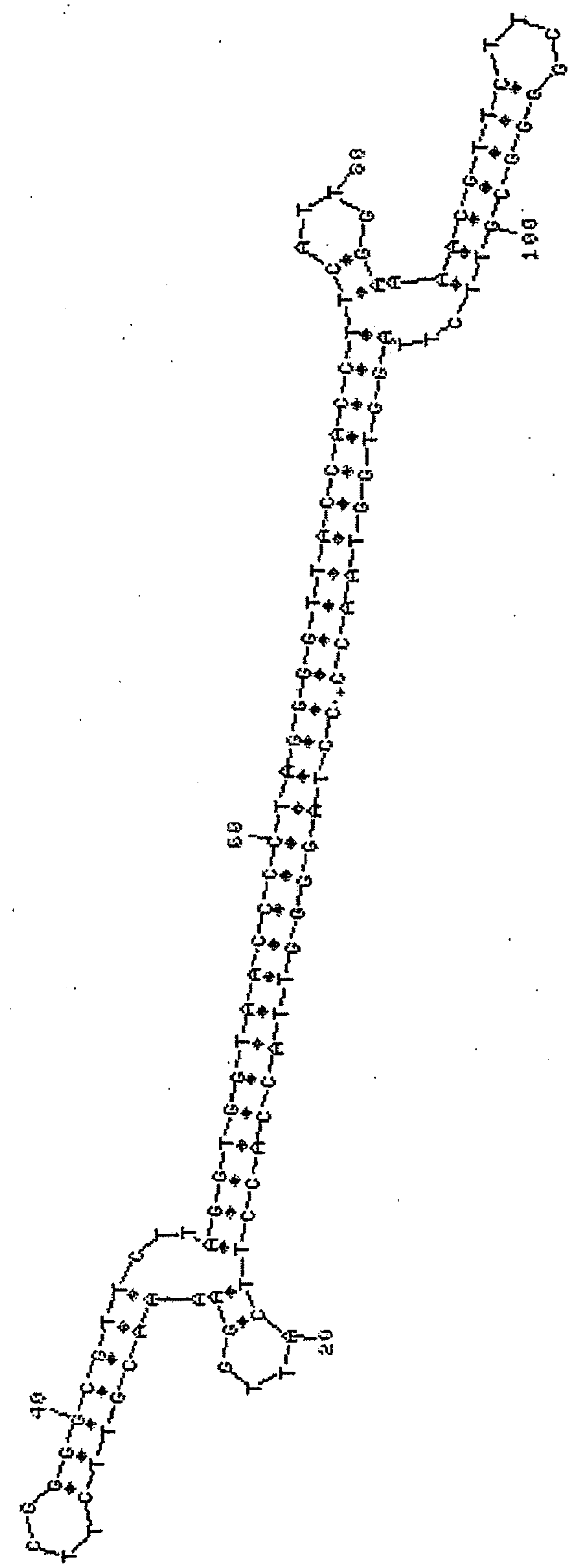

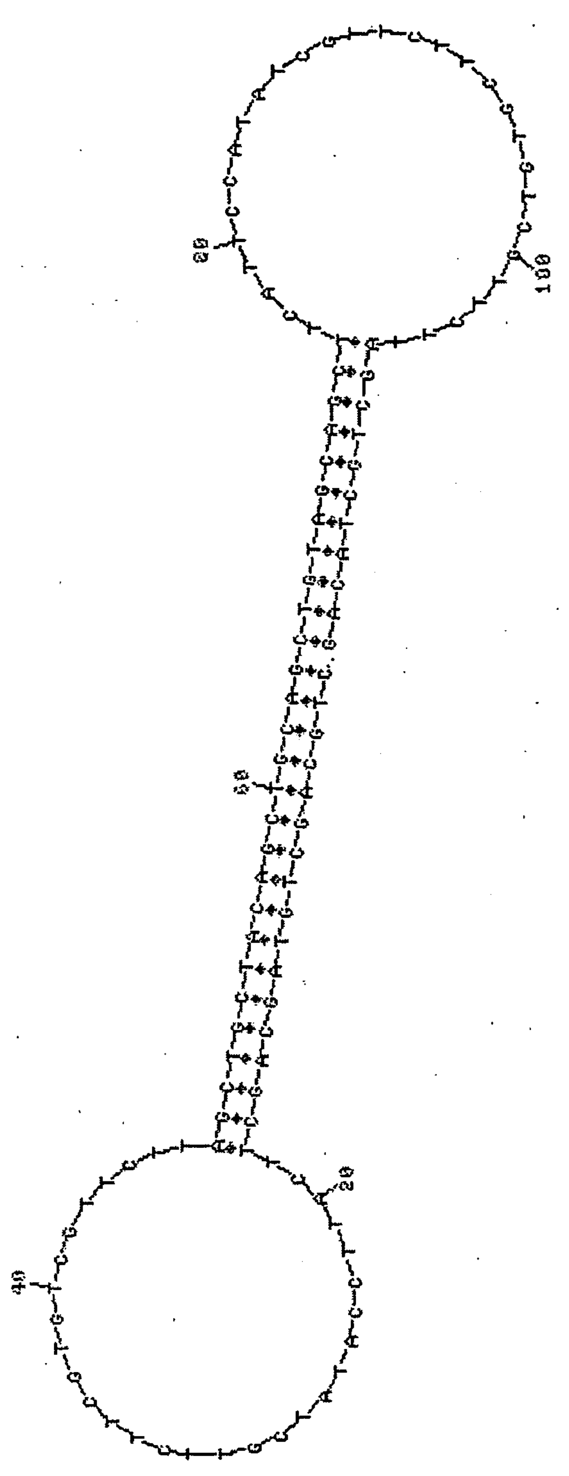

Abbildung 3.1.1: Von „DNA mfold" berechnete Sekundärstruktren
Bedingungen: 150 mM NaCl, 0,5 mM MgCl2 und 37°C a) 30L1, b) KG

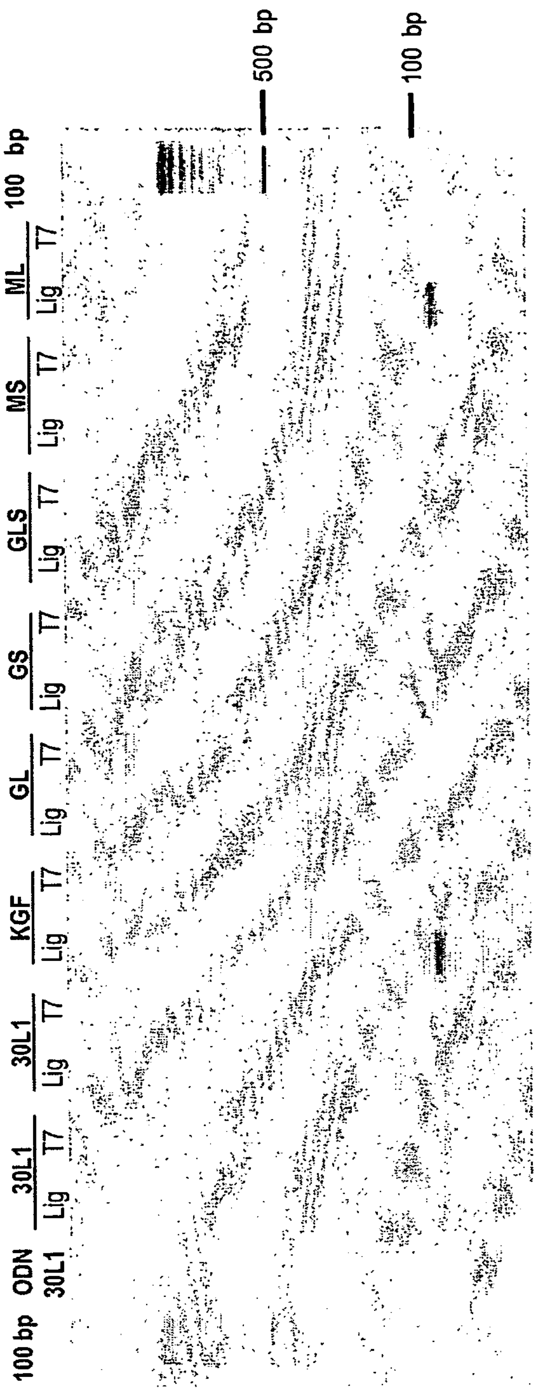


Abbildung 3.1.2: Ligation und T7-Verdau verschiedener dSLIM
3% Agarosegel, Auftragungsmenge je 0,3 µg, Marker 100 bp 0,5 µg (100 bp, ODN30L1, jeweils Ligation, T7-Verdau: 30L1, 30L1, KGF, GL, GS, GLS, MS, ML)

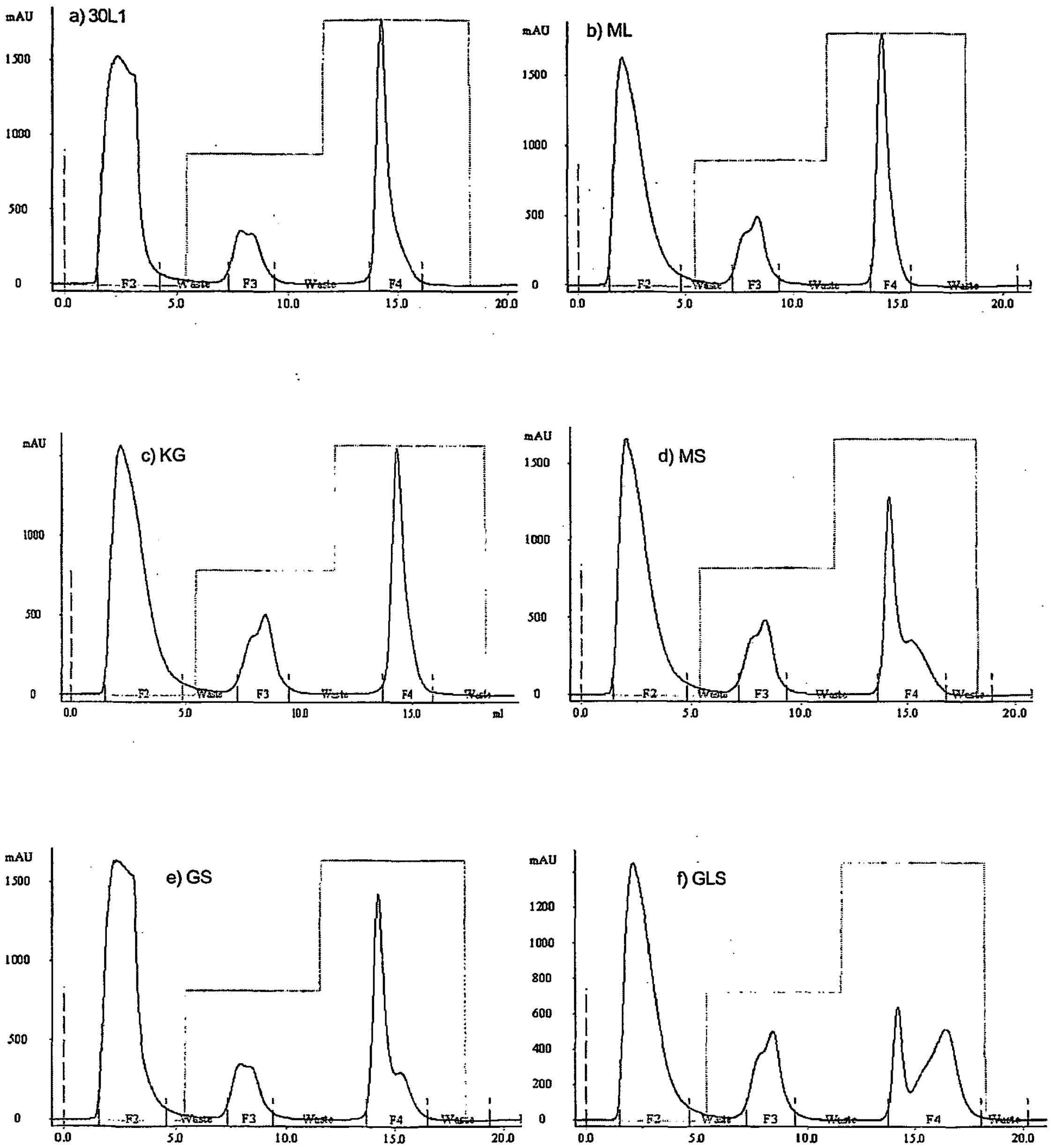
a) 30L1
b) ML
c) KG
d) MS
e) GS
f) GLS
mAU
1500
1000
500
0
0.0
5.0
10.0
15.0
20.0
F2
Waste
F3
F4
ml
1200
800
600
400
200

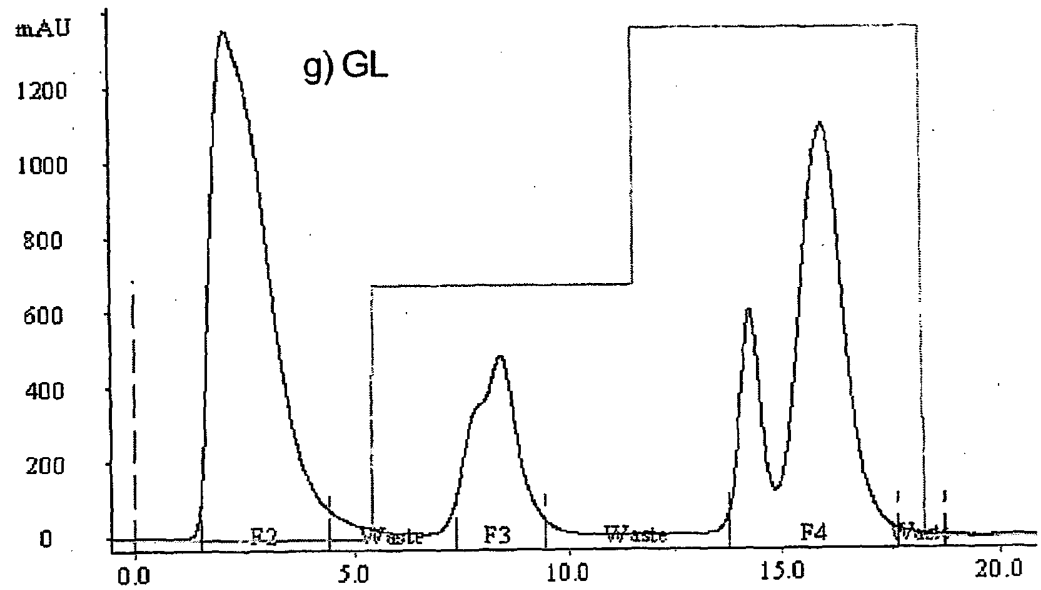


Abbildung 3.1.3: Chromatogramme der HPLC-Aufreinigung verschiedener dSLIM a) 30L1 b) ML, c) KG, d) MS, e) GS, f) GLS, g) GL, Säule: 1 mL DMAE; Auftragungsmenge: 700 µg; Flußrate 1 mL/min; Puffer A: 20 mM Tris pH 7; Puffer B: 20 mM Tris, 1 M NaCl, pH7; Gradient:: 0%, 50% 100% Puffer B, ——— Absorption 260 nm, ——— NaCl-Gradient,- - - - - - Injektion, F2-F4 Frationierung

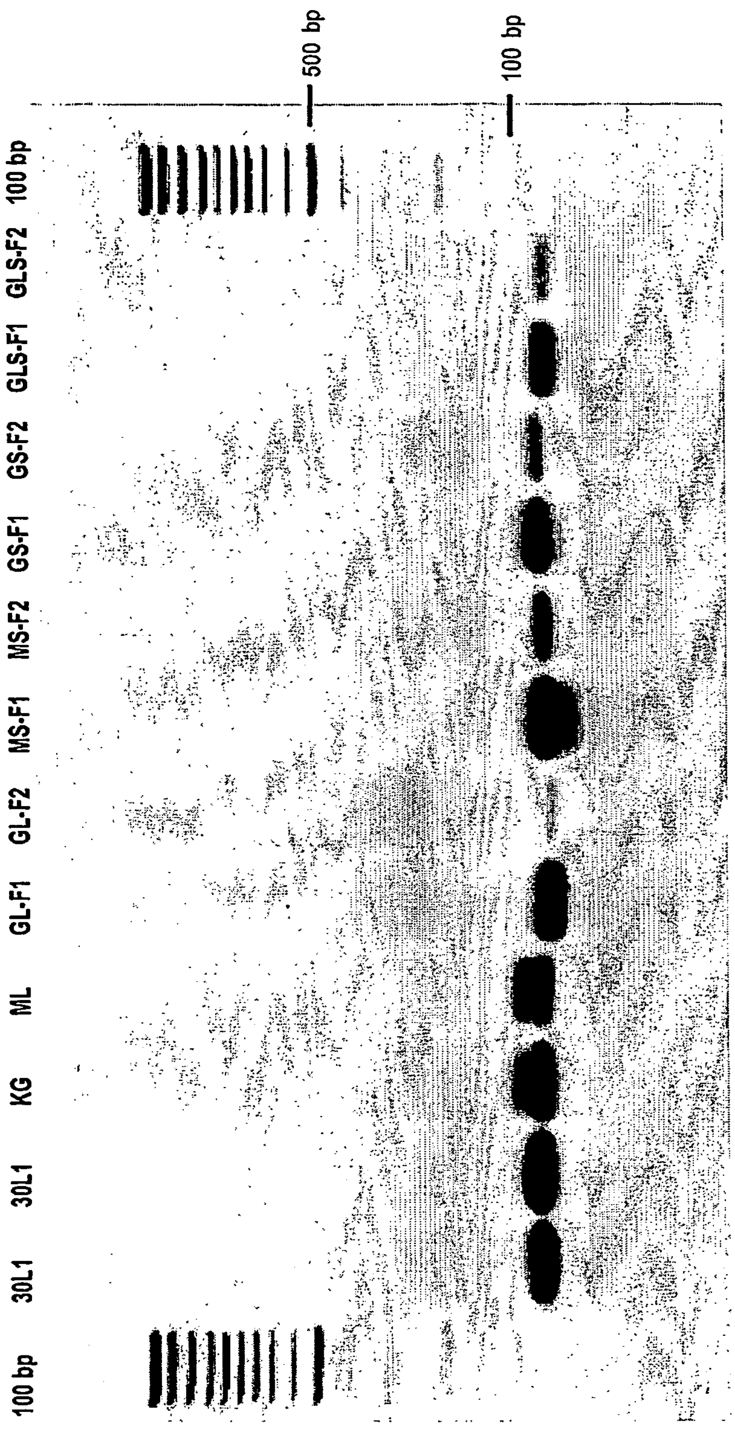


Abbildung 3.1.4: Verschiedene dSLIM nach Ethanolfällung

3% Agarosegel, Auftragungsmenge 0,3 µg, Marker 100 bp 0,5 µg (100 bp, 30L1, 30L1, KG, ML, GL-F1, GL-F2, MS-F1, MS-F2, GS-F1, GS-F2 GLS-F1, GLS-F2, 100 bp)

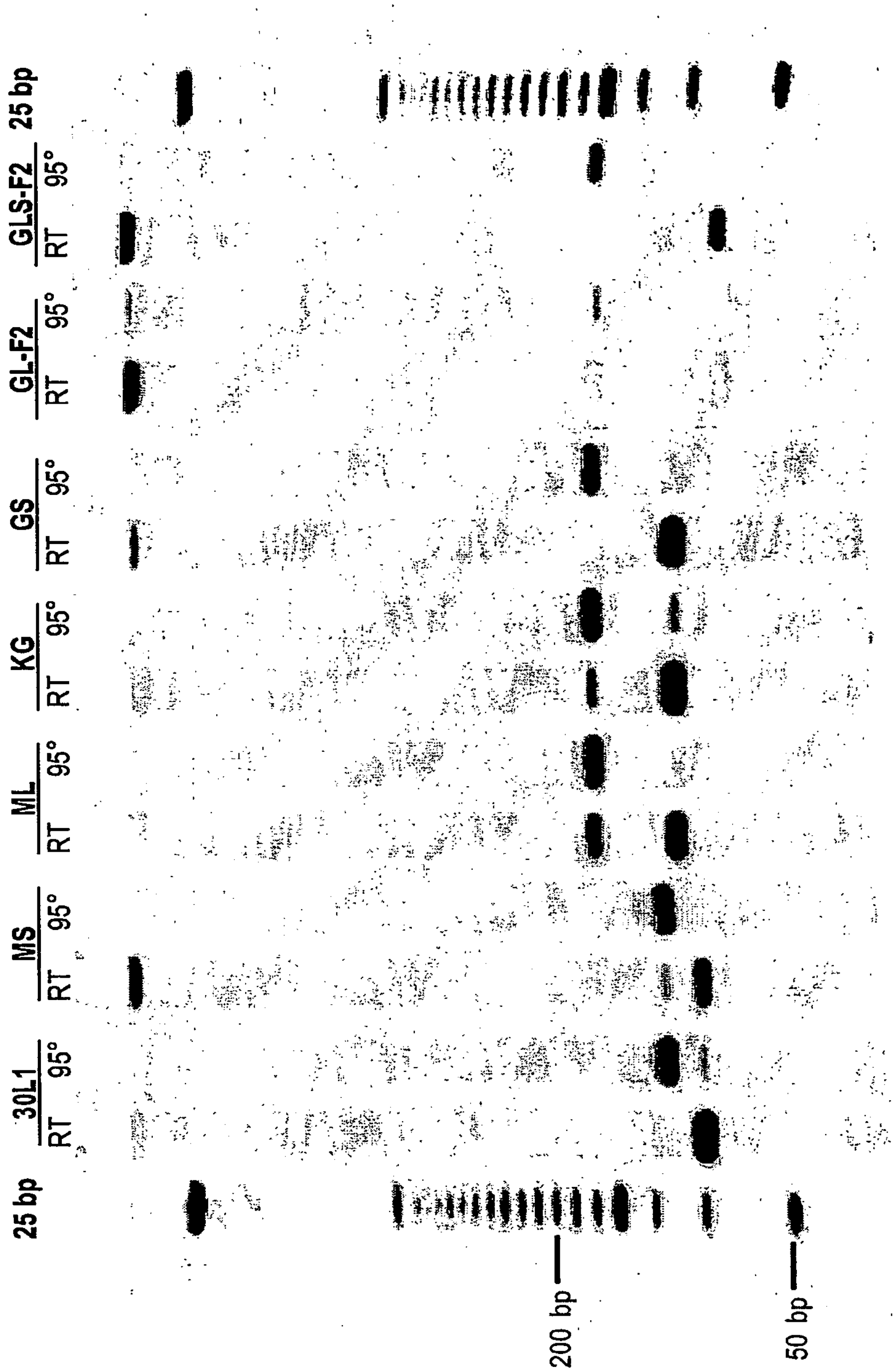


Abbildung 3.1.6: Thermische Denaturierung verschiedener dSLIM
Native PAGE 8% Gel (2,6% C), Auftragungsmenge 0,25 µg, Marker 25 bp 0,3 µg (25 bp, unbehandelt, Denaturierung 95°C 10 min: 30L1, MS, ML, KG, GS, GL-F2, GLS-F2)

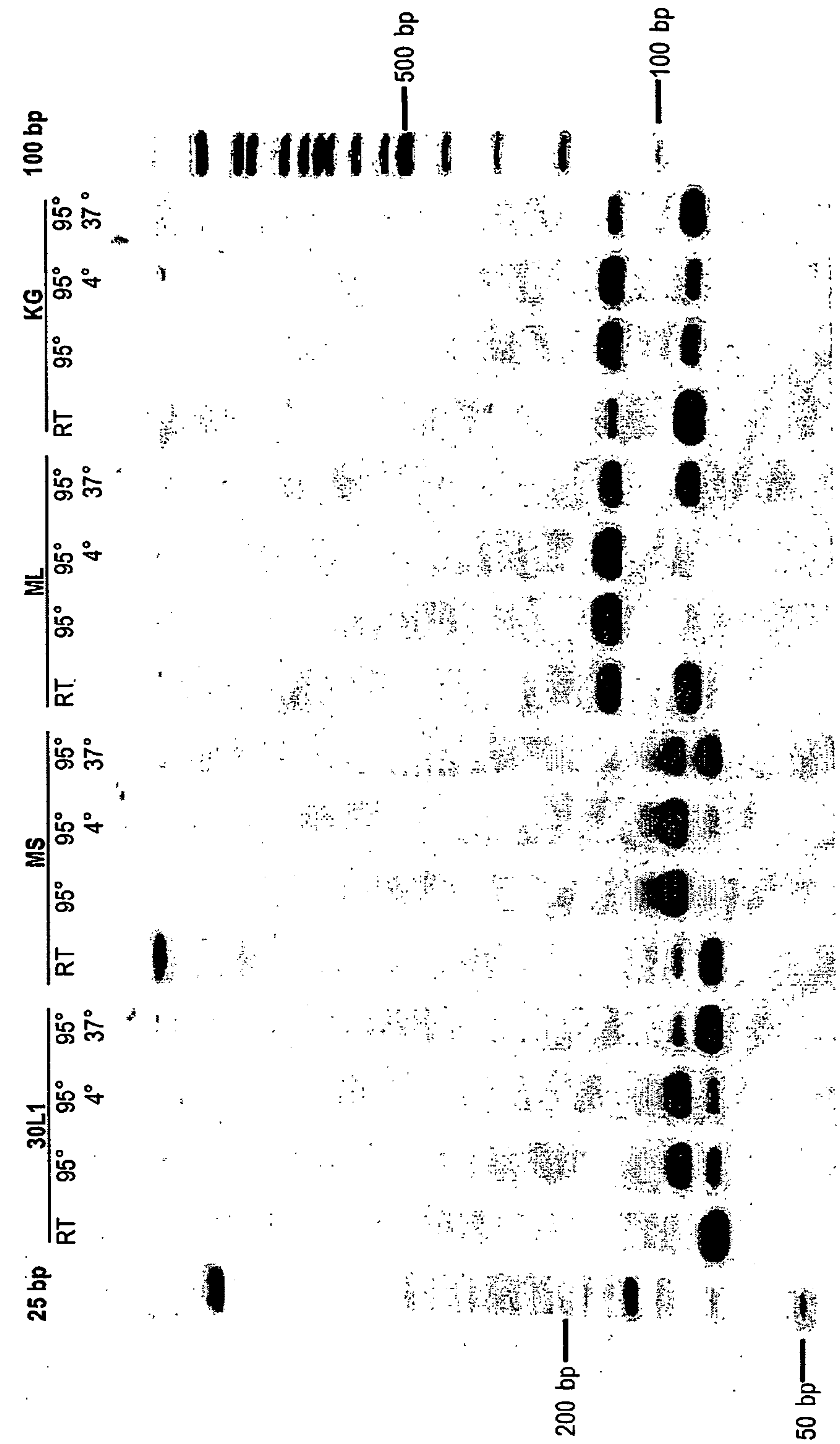


Abbildung 3.1.7: Thermische Denaturierung und Renaturierung verschiedener dSLIM Native PAGE 8% Gel (2,6% C), Auftragungsmenge 0,25 µg, Marker 25 bp 0,3 µg, Marker 100 bp 0,3 µg (25 bp, unbehandelt, Denaturierung 95°C 10 min, 3 d 4°C nach Denaturierung, 3 d 37°C nach Denaturierung: 30L1, MS, ML, KG, 100 bp)

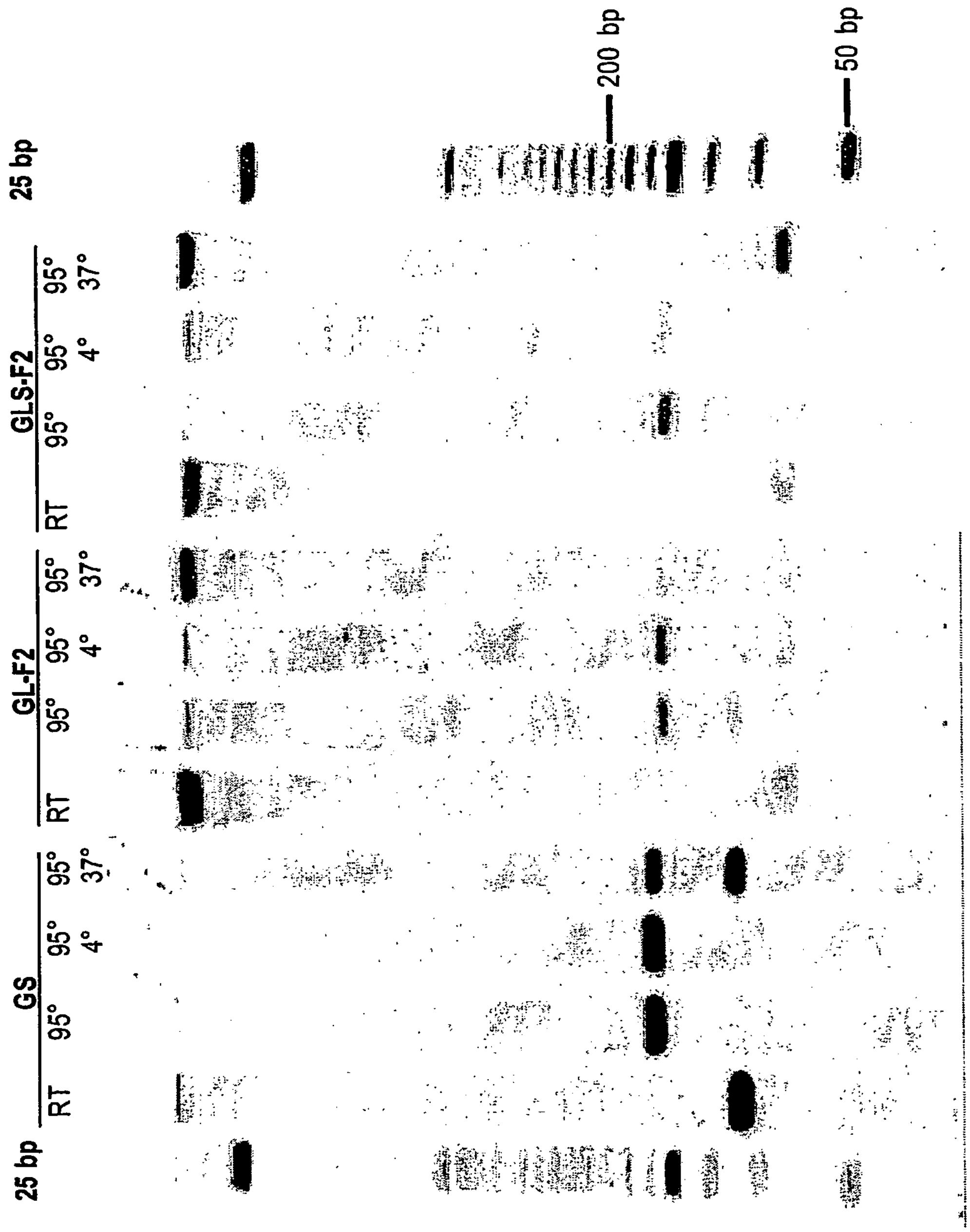


Abbildung 3.1.8: Thermische Denaturierung und Renaturierung verschiedener dSLIM Native PAGE 8% Gel (2,6% C), Auftragungsmenge 0,25 µg, Marker 25 bp 0,3 µg, Marker 100 bp 0,3 µg (25 bp, unbehandelt, Denaturierung 95°C 10 min, 3 d 4°C nach Denaturierung, 3 d 37°C nach Denaturierung: GS, GL-F2, GLS-F2, 100 bp)

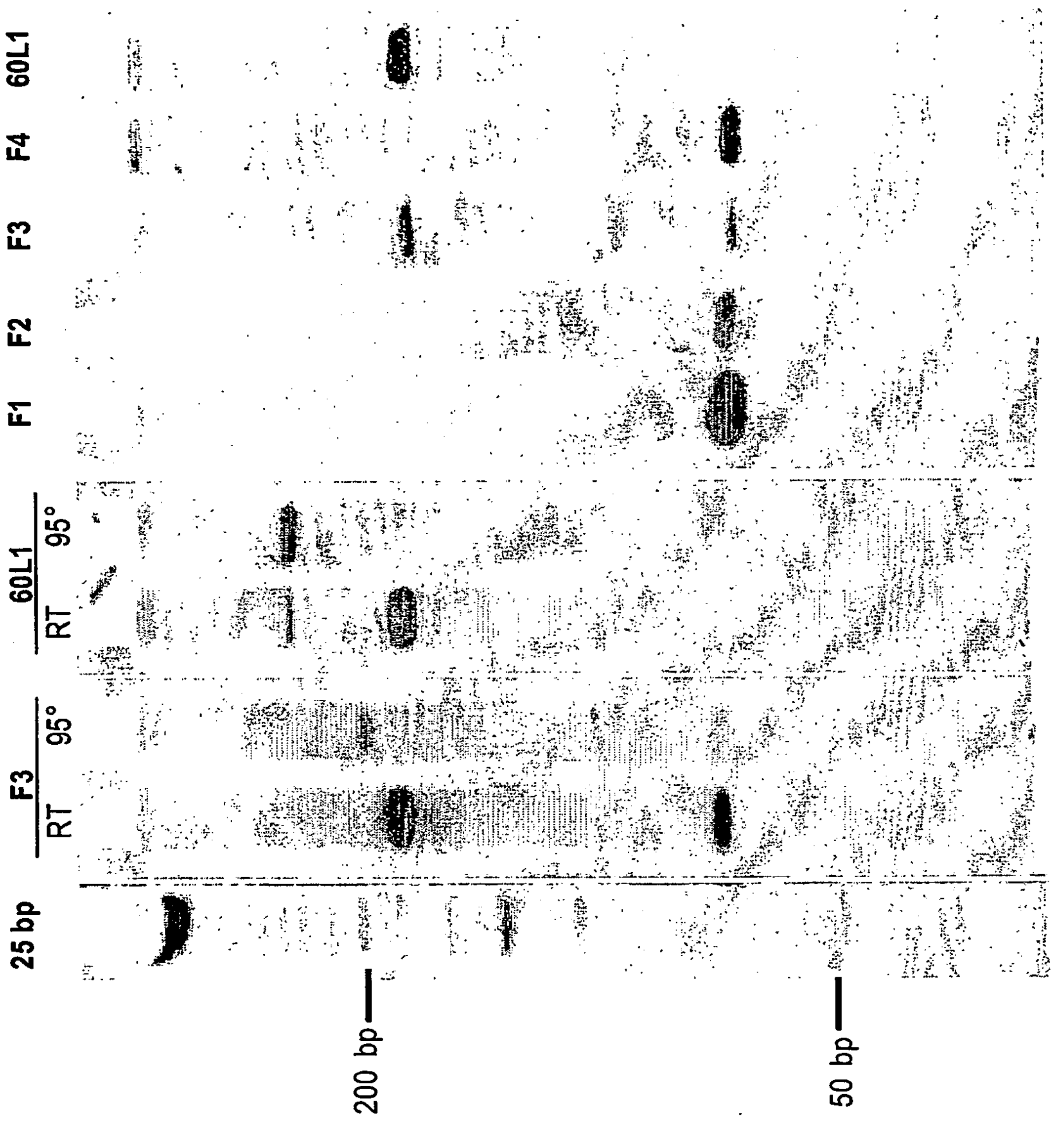


Abbildung 3.1.9: Vergleich Fraktion 3 mit Dimermodell-Molekül 60L1

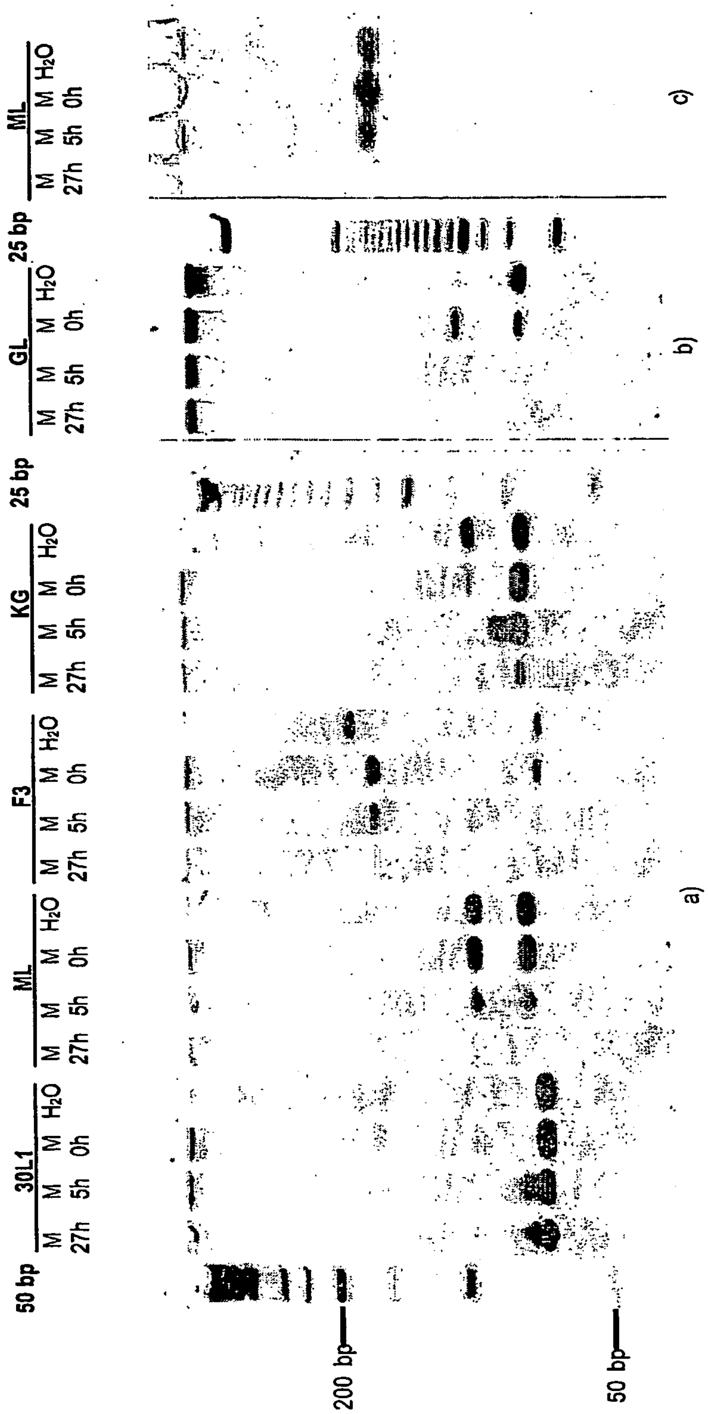


**Abbildung 3.1.10: Inkubation von dSLIM in Medium**

a) Native PAGE 8% Gel (5% C), Auftragungsmenge 0,25 µg, Marker 25 bp 0,3 µg, Marker 50 bp 0,3 µg (50 bp, 27 h Medium, 5 h Medium, 0 h Medium, H20: 30L1, ML, F3, KG, 25 bp)

b) Native PAGE 8% Gel (2,6% C), Auftragungsmenge 0,25 µg, 25 bp 0,35 µg (27 h Medium, 5 h Medium, 0 h Medium, H20: GL, 25 bp)

c) Ausschnitt Coomassie-Färbung native 8% PAGE (5% C) von Abbildung 3.1.11 a) (27 h Medium, 5 h Medium, 0 h Medium, H20: ML)

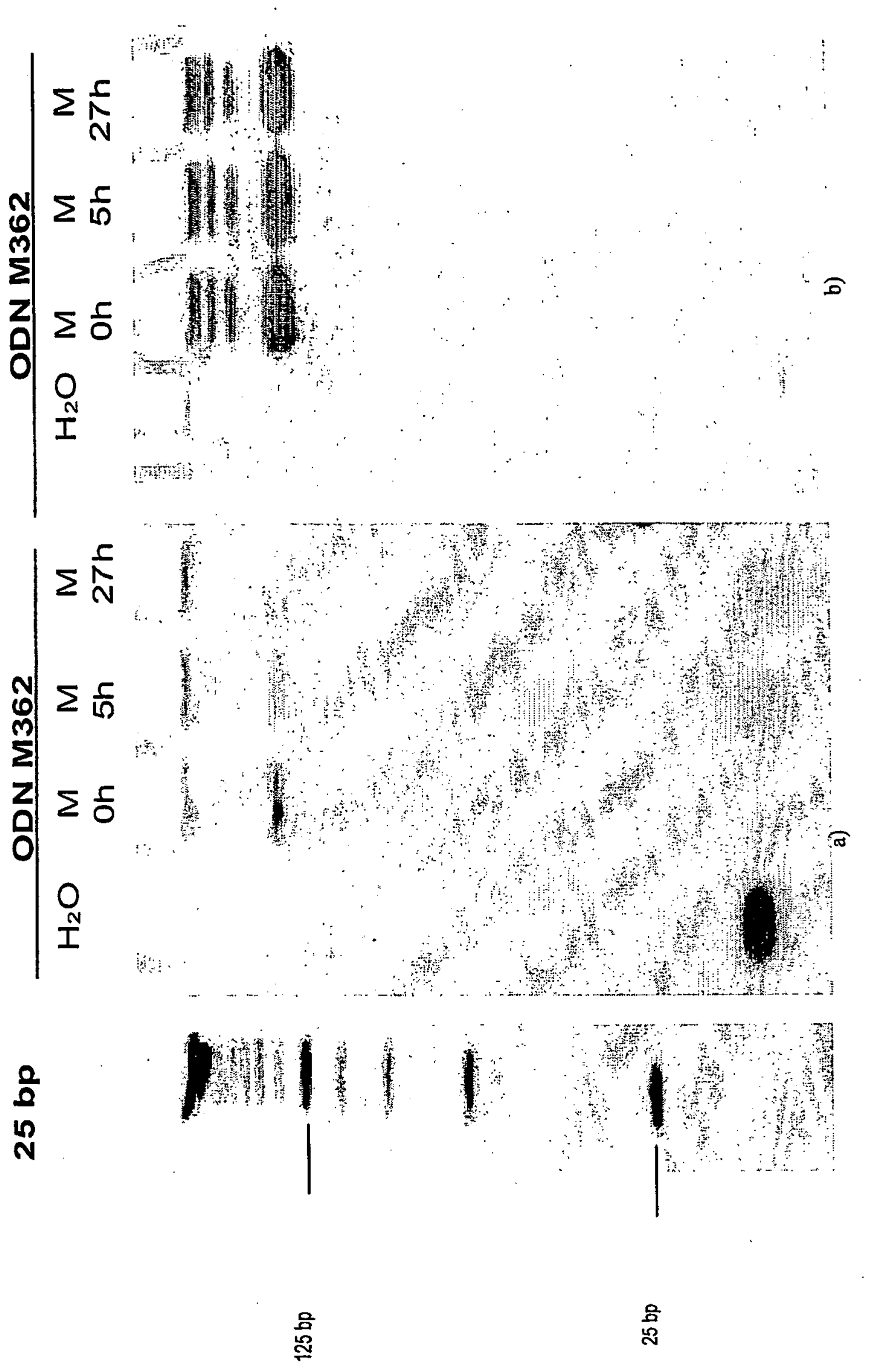


Abbildung 3.1.11: Inkubation von ODN M362 in Medium

a) Native PAGE 12% Gel (5% C), Auftragungsmenge 0,25 µg, Marker 25 bp 0,3 µg (25 bp, H2O, 0 h Medium, 5 h Medium, 27 h Medium)

b) Ausschnitt Coomassie-Färbung native PAGE 12% Gel (5% C) von Abbildung 3.1.12 a) (H2O, 0 h Medium, 5 h Medium, 27 h Medium)

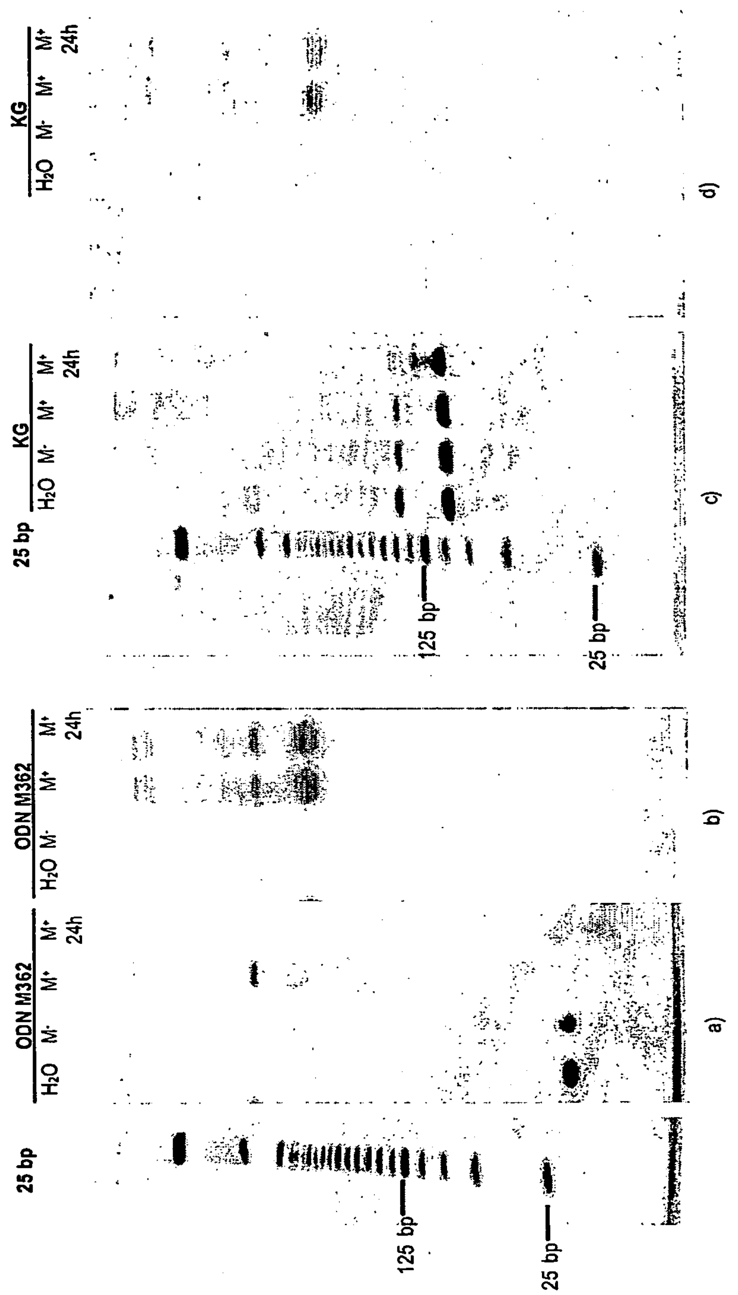


Abbildung 3.1.12: Inkubation von dSLIM und ODN M362 in Medium mit und ohne **FCS**

a) Native PAGE 4-20% Gel, Auftragungsmenge M362 0,05 µg, Marker 25 bp 0,2 µg (25 bp, H2O, Medium ohne FCS (M-), Medium mit FCS (M+), 24 h Medium mit FCS)

b) Ausschnitt Coomassie-Färbung native PAGE 4-20% Gel von Abbildung 3.1.13 a) (25 bp, H2O, Medium ohne FCS (M-), Medium mit FCS (M+), 24 h Medium mit FCS)

c) Native PAGE 4-20% Gel, Auftragungsmenge KG 0,12 µg, Marker 25 bp 0,2 µg (25 bp, H2O, Medium ohne FCS (M-), Medium mit FCS (M+), 24 h Medium mit FCS)

d) Ausschnitt Coomassie-Färbung native PAGE 4-20% Gel von Abbildung 3.1.13 c) (25 bp, H2O, Medium ohne FCS (M-), Medium mit FCS (M+), 24 h Medium mit FCS)

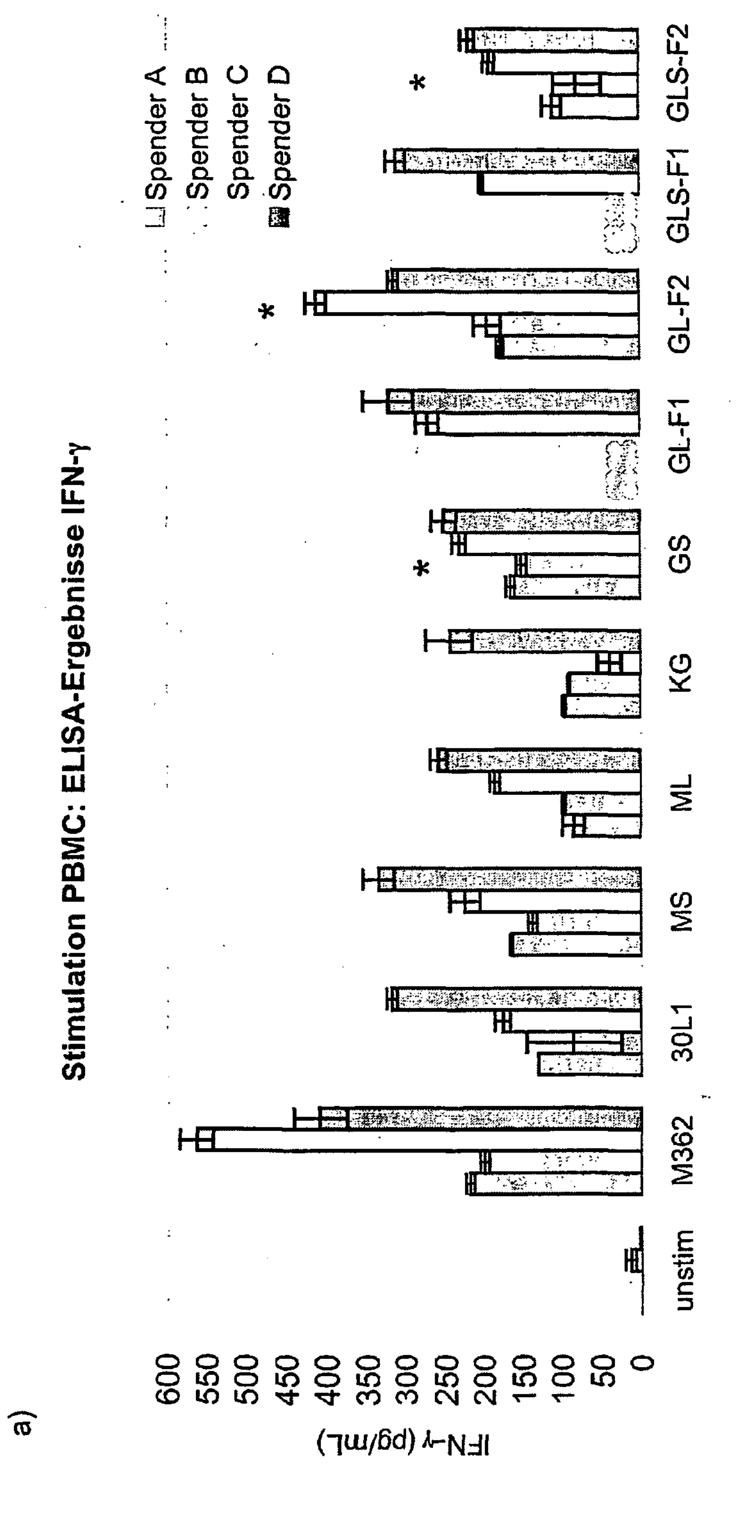


Endotoxin (EU/mg DNA): 3 <1 6 8 1 1 <1

b)

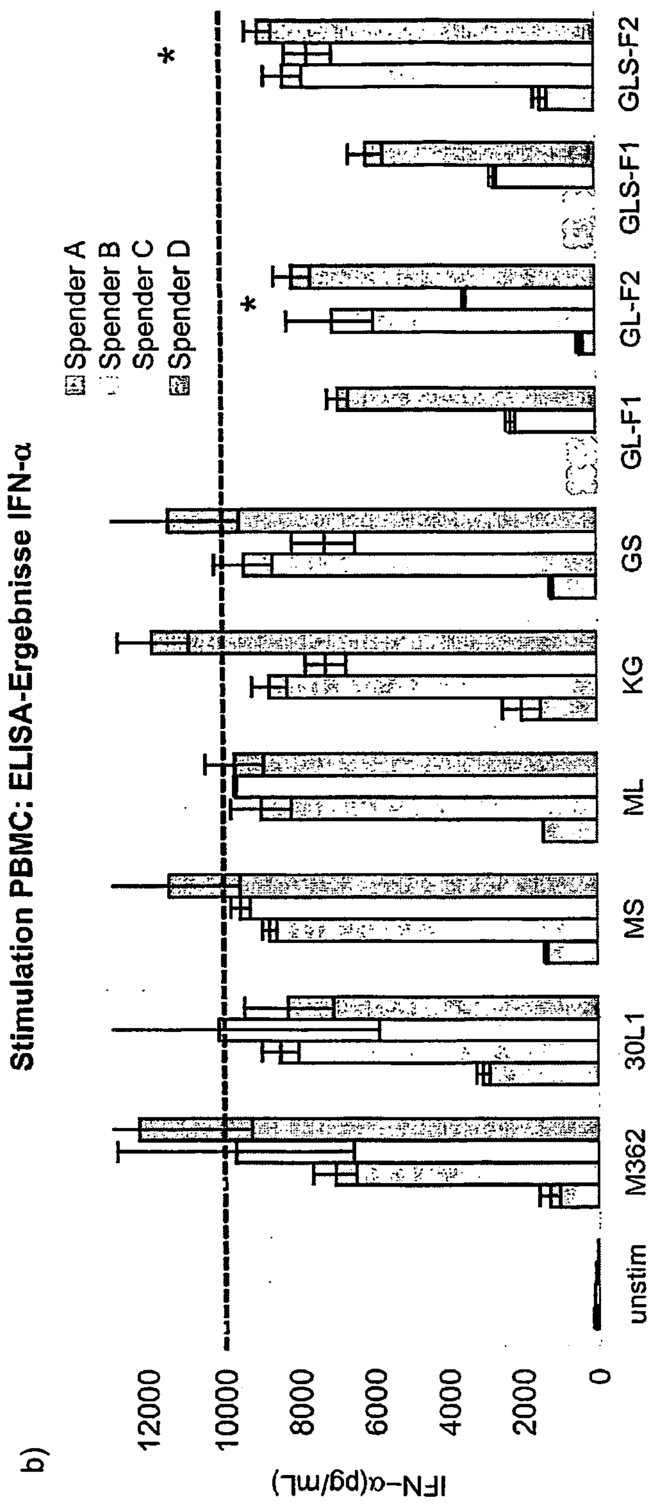
Stimulation PBMC: ELISA-Ergebnisse IFN-α
Spender A
Spender B
Spender C
Spender D
IFN-α(pg/mL)
12000
10000
8000
6000
4000
2000
0
unstim
M362
30L1
MS
ML
KG
GS
GL-F1
GL-F2
GLS-F1
GLS-F2
*
*

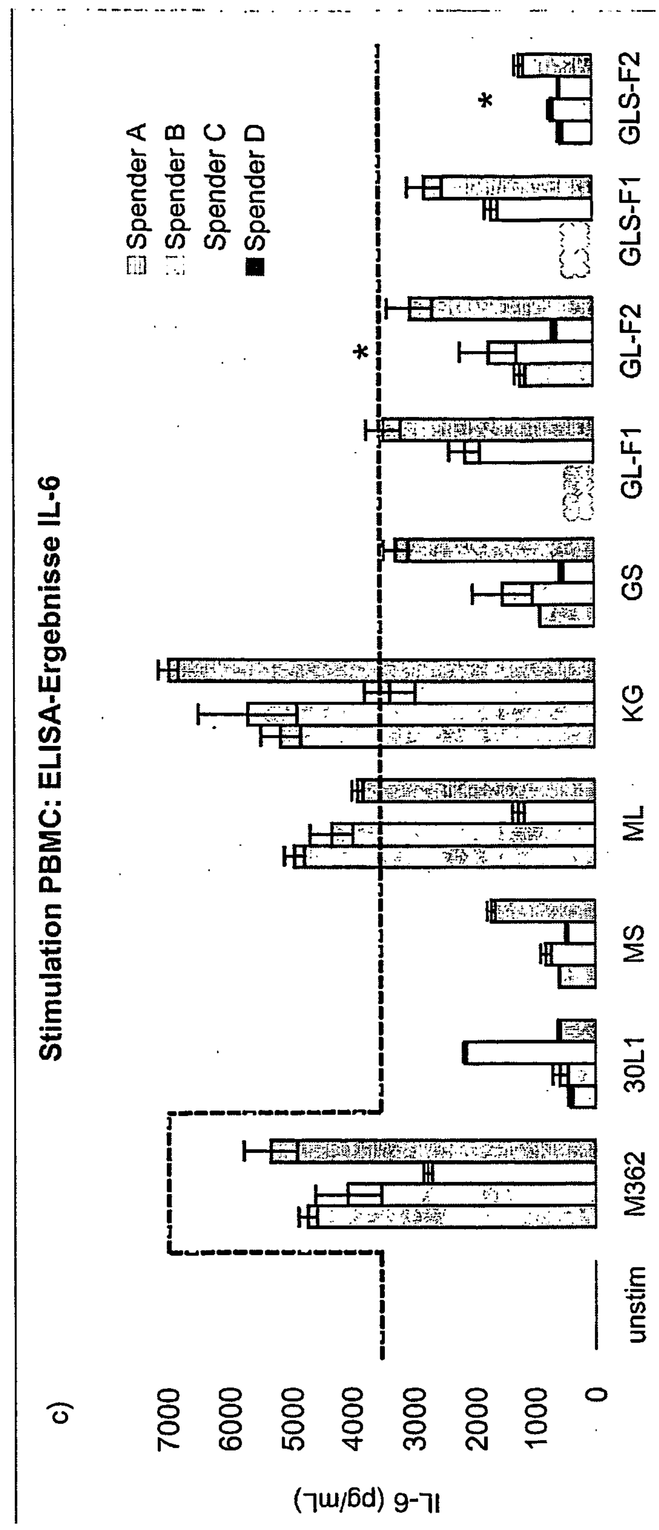


Abbildung 3.2.1 a-c: Ergebnisse ELISA IFN-g (a), IFN-a (b), IL-6 (c) PBMC
Überstände PBMC (2,4 x 106 Zellen pro Ansatz in 600 µL) verschiedener Spender A-D, Stimulation 48 h mit 1 µM dSLIM/ODN M362, Standardmeßbereich —, * Doppelbest Zellkultur, Fehlerbalken: 2 x Standardabweichung aus Doppelbestimmung ELISA (* Doppelbest. Zellkultur + Doppelbest. ELISA = 4 Werte)

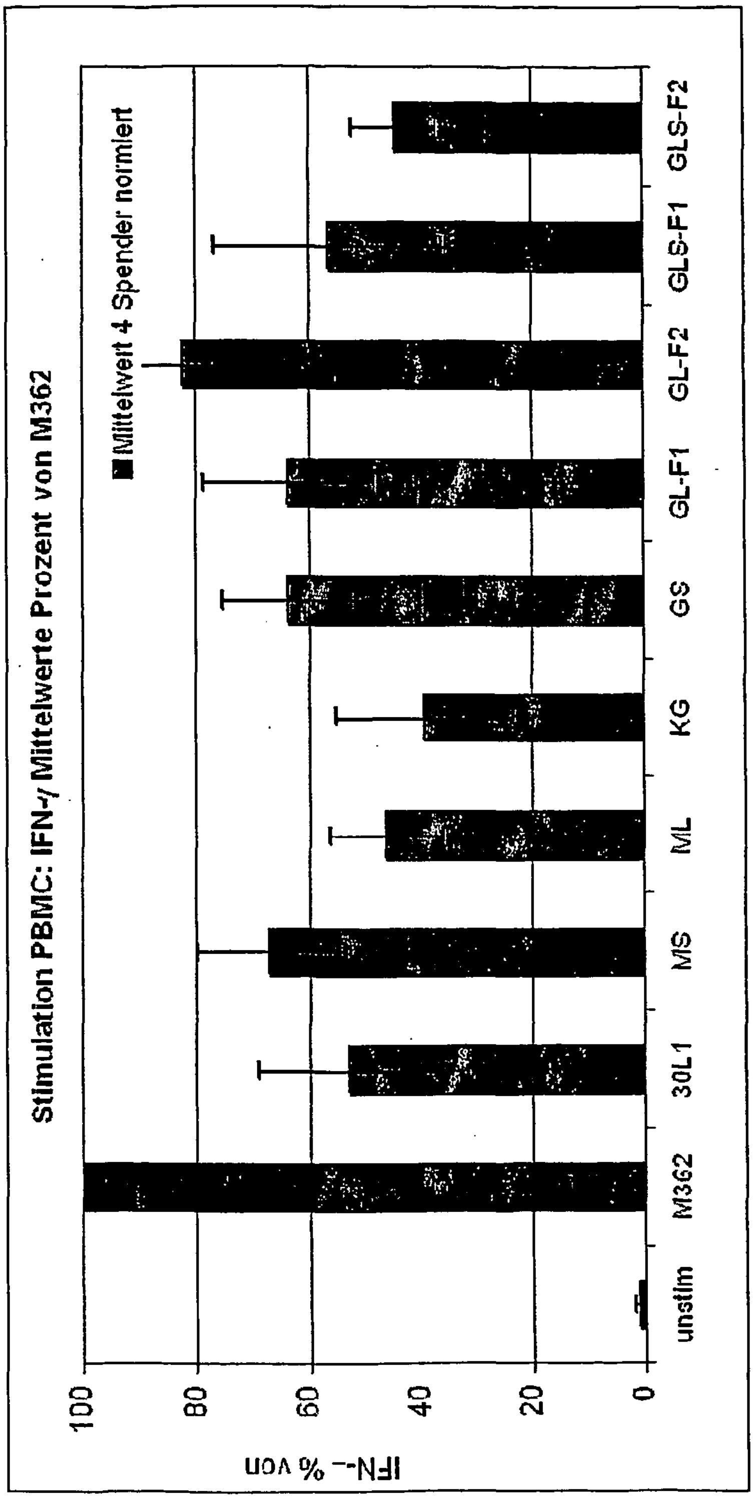
Stimulation PBMC: IFN-γ Mittelwerte Prozent von M362
Mittelwert 4 Spender normiert
IFN-- % von
100
80
60
40
20
0
unstim
M362
30L1
MS
ML
KG
GS
GL-F1
GL-F2
GLS-F1
GLS-F2

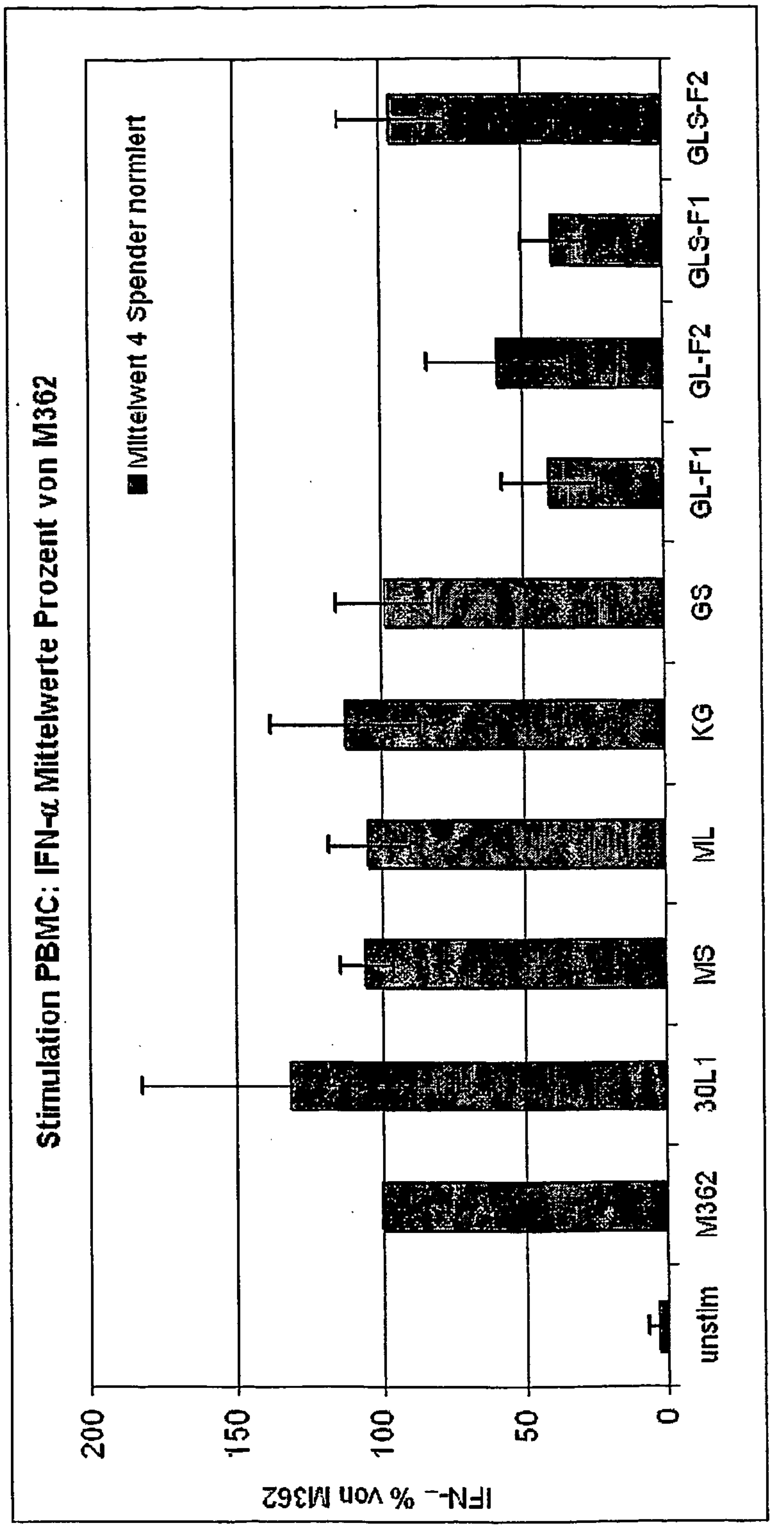
Stimulation PBMC: IFN-α Mittelwerte Prozent von M362
Mittelwert 4 Spender normiert
IFN-– % von M362
200
150
100
50
0
unstim
M362
30L1
MS
ML
KG
GS
GL-F1
GL-F2
GLS-F1
GLS-F2

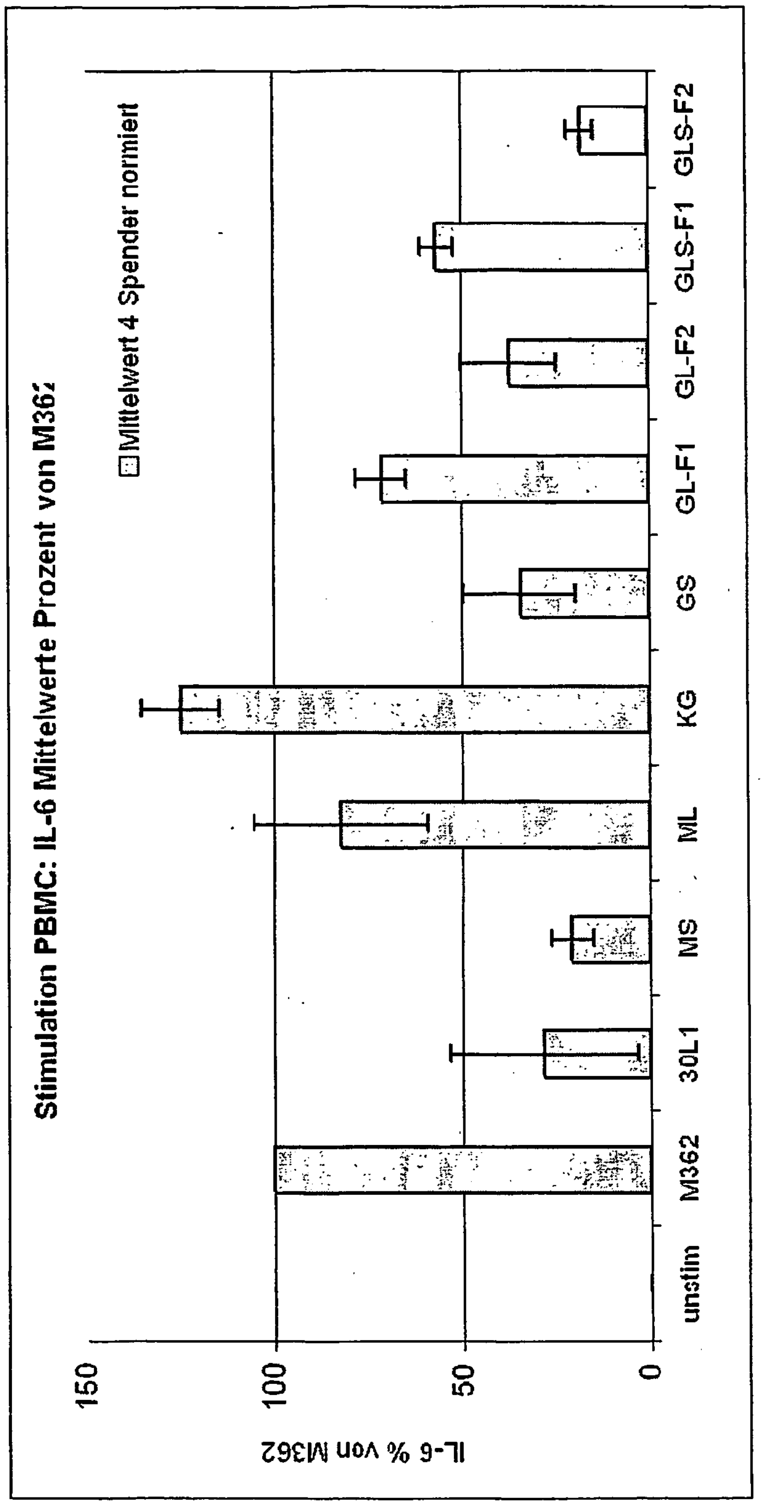


Abbildung 3.2.2 a-c: Normierte Mittelwerte der Ergebnisse ELISA IFN-g (a), IFN-a (b), IL-6 (c) Ergebnisse ELISA s. Abb. 3.2.1 normiert: Cytokin-Menge in % von M362 je Spender, Mittelwerte aus 4 Spendern, Fehlerbalken: Mittelwertabweichung.

a)

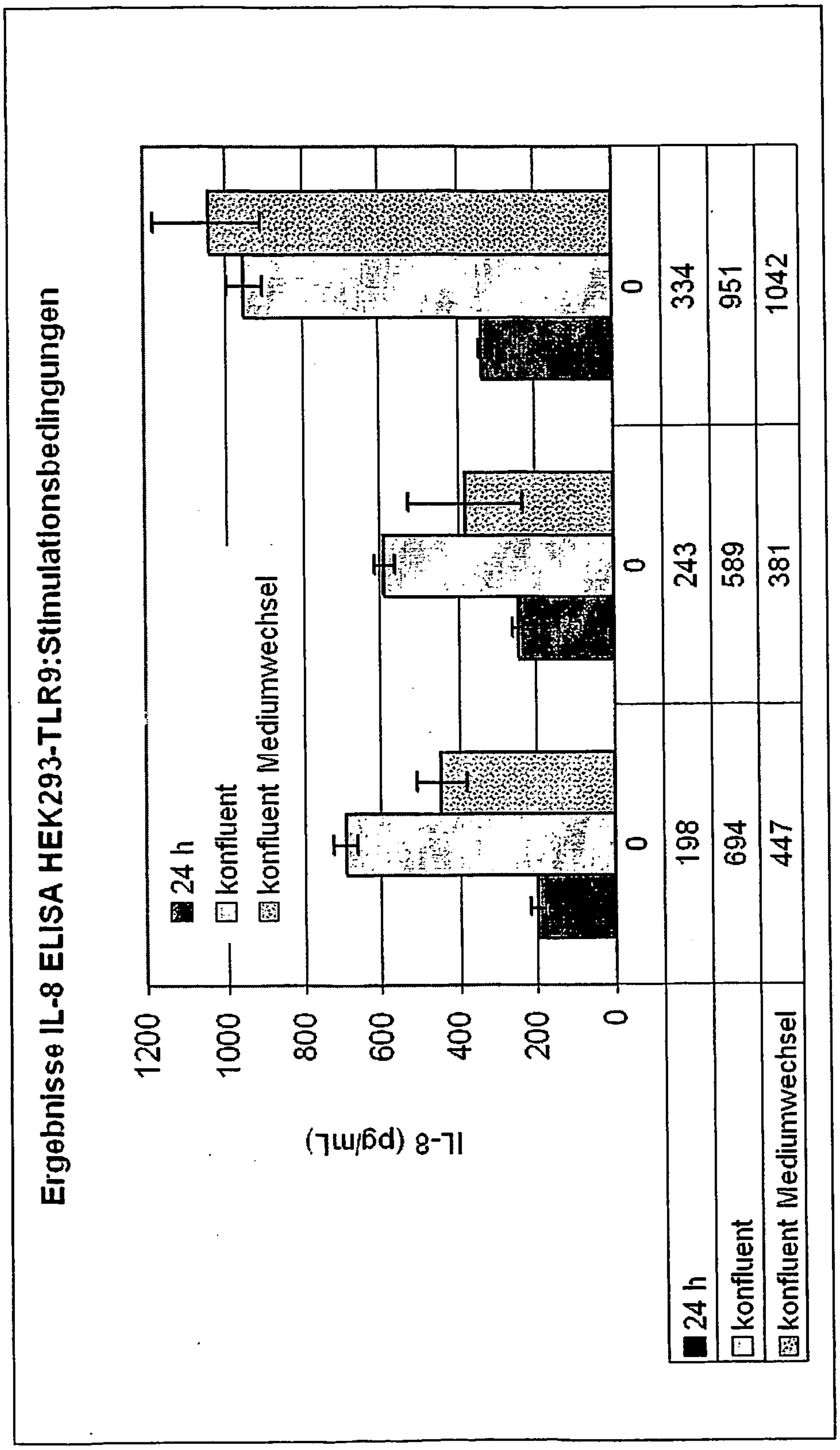
Ergebnisse IL-8 ELISA HEK293-TLR9:Stimulationsbedingungen
IL-8 (pg/mL)
1200
1000
800
600
400
200
0
24 h
konfluent
konfluent Mediumwechsel
0 0 0
24 h 198 243 334
konfluent 694 589 951
konfluent Mediumwechsel 447 381 1042

b)

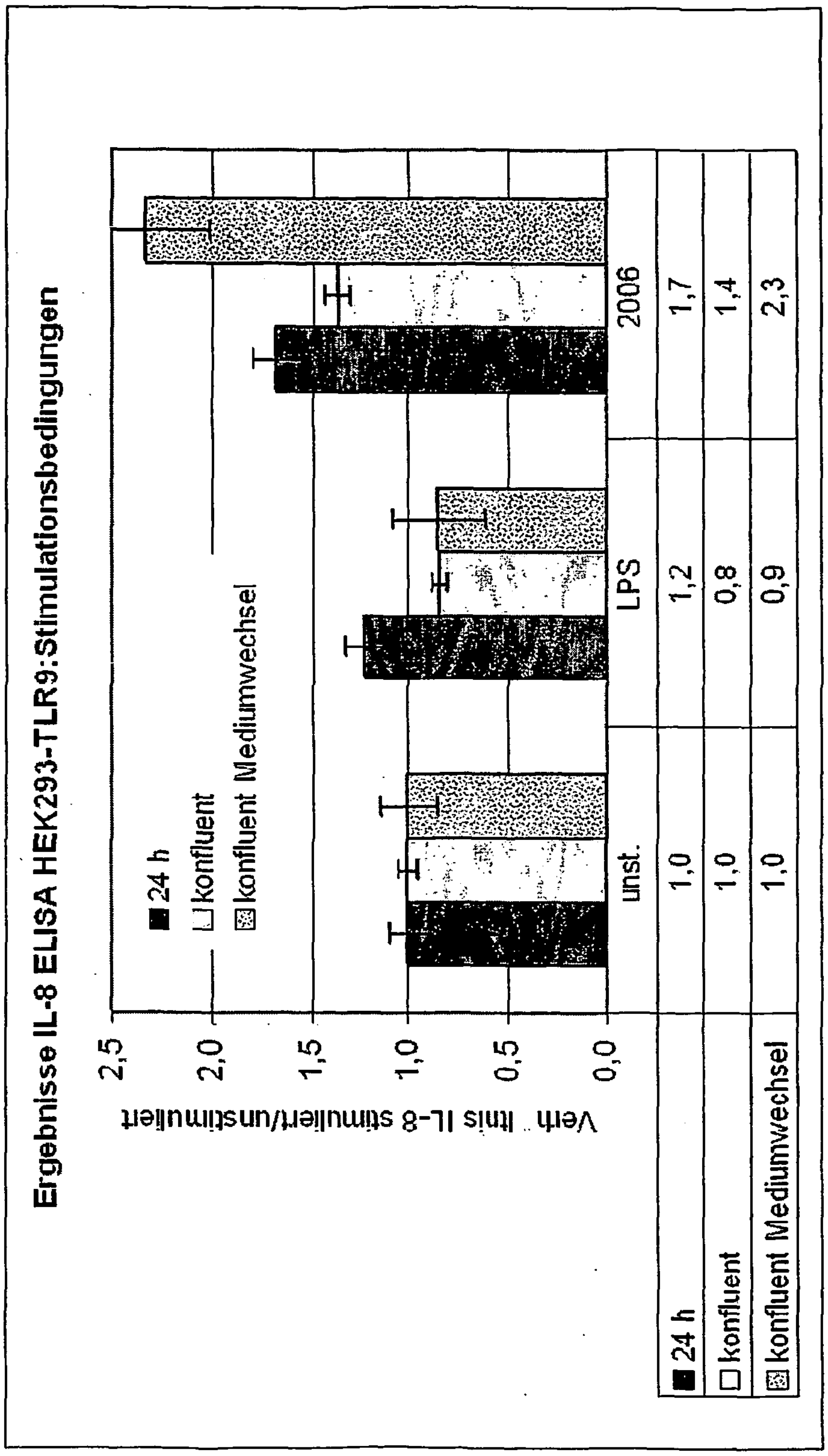


Abbildung 3.3.1: Ergebnisse ELISA IL-8 HEK293-TLR9 Stimulationsbedingungen

a)

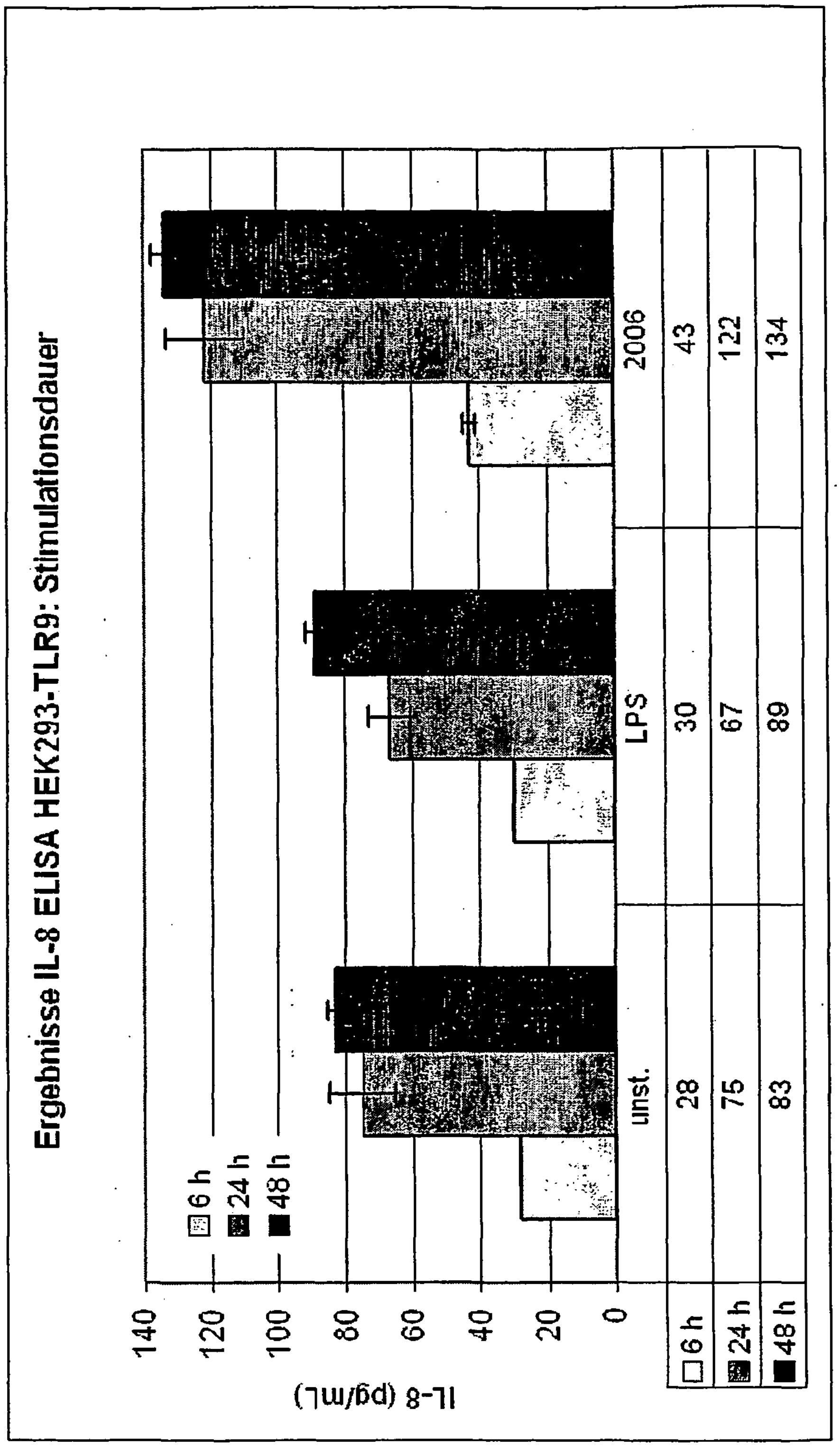
Ergebnisse IL-8 ELISA HEK293-TLR9: Stimulationsdauer
IL-8 (pg/mL)
140
120
100
80
60
40
20
0
6 h
24 h
48 h
unst.
LPS
2006
6 h 28 30 43
24 h 75 67 122
48 h 83 89 134

b)

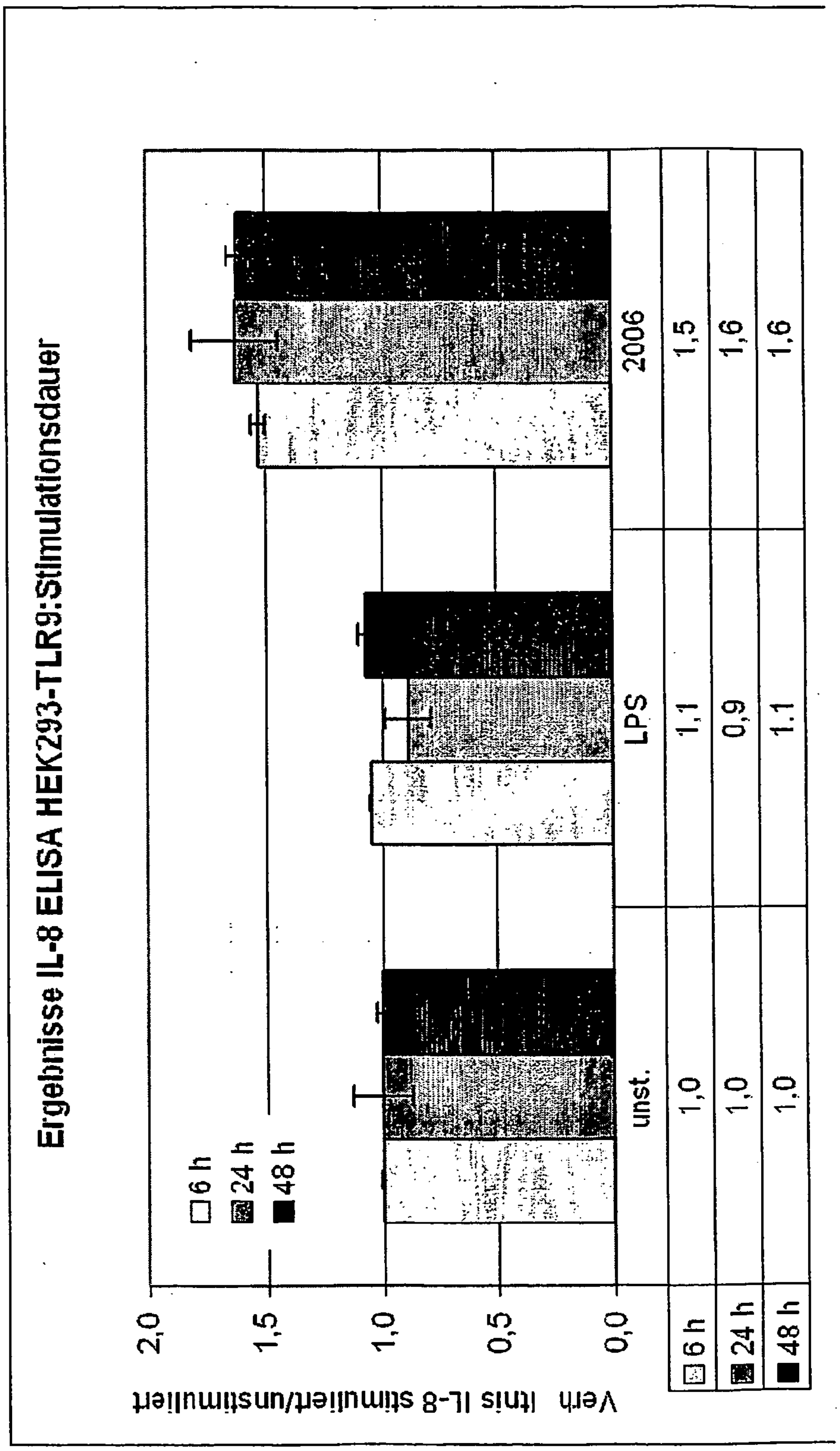


Abbildung 3.3.2: Ergebnisse ELISA IL-8 HEK293-TLR9 Stimulationsdauer

a)

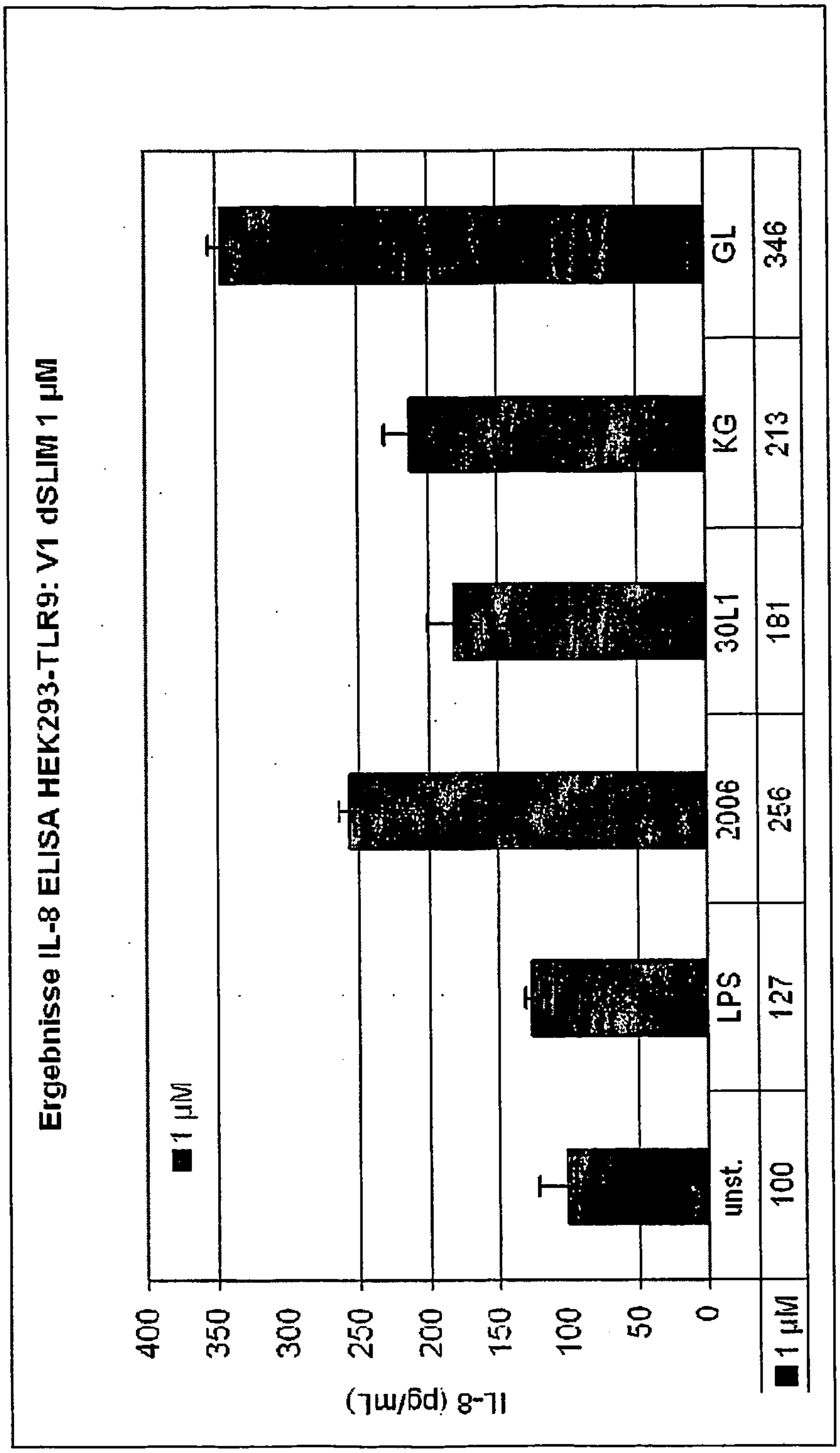
Ergebnisse IL-8 ELISA HEK293-TLR9: V1 dSLIM 1 µM
1 µM
IL-8 (pg/mL)
400
350
300
250
200
150
100
50
0
unst.
LPS
2006
30L1
KG
GL
1 µM
100
127
256
181
213
346

b)

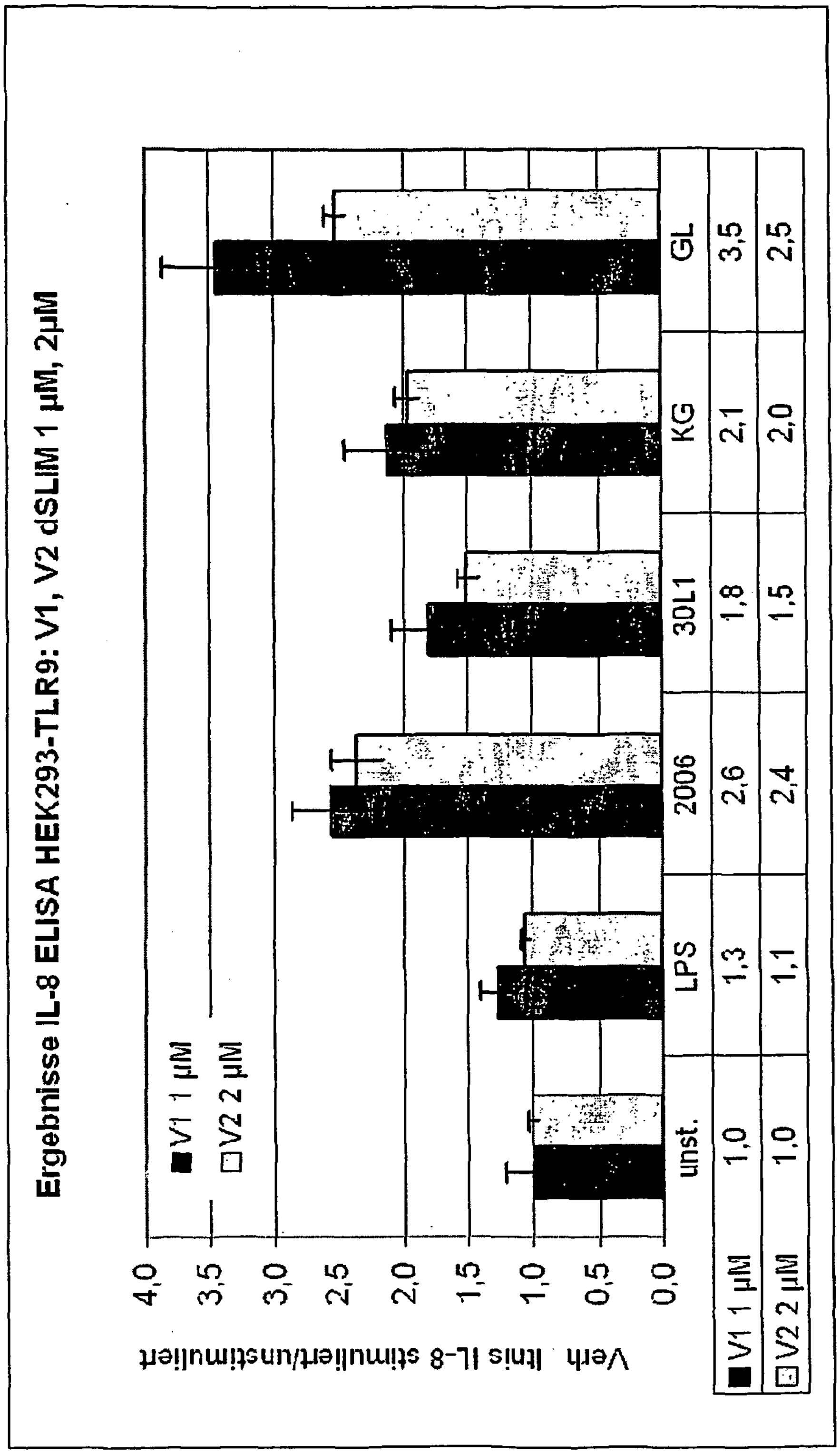
Ergebnisse IL-8 ELISA HEK293-TLR9: V1, V2 dSLIM 1 µM, 2µM
V1 1 µM
V2 2 µM
Verh ltnis IL-8 stimuliert/unstimuliert
4,0
3,5
3,0
2,5
2,0
1,5
1,0
0,5
0,0
unst. LPS 2006 30L1 KG GL
V1 1 µM 1,0 1,3 2,6 1,8 2,1 3,5
V2 2 µM 1,0 1,1 2,4 1,5 2,0 2,5

c)

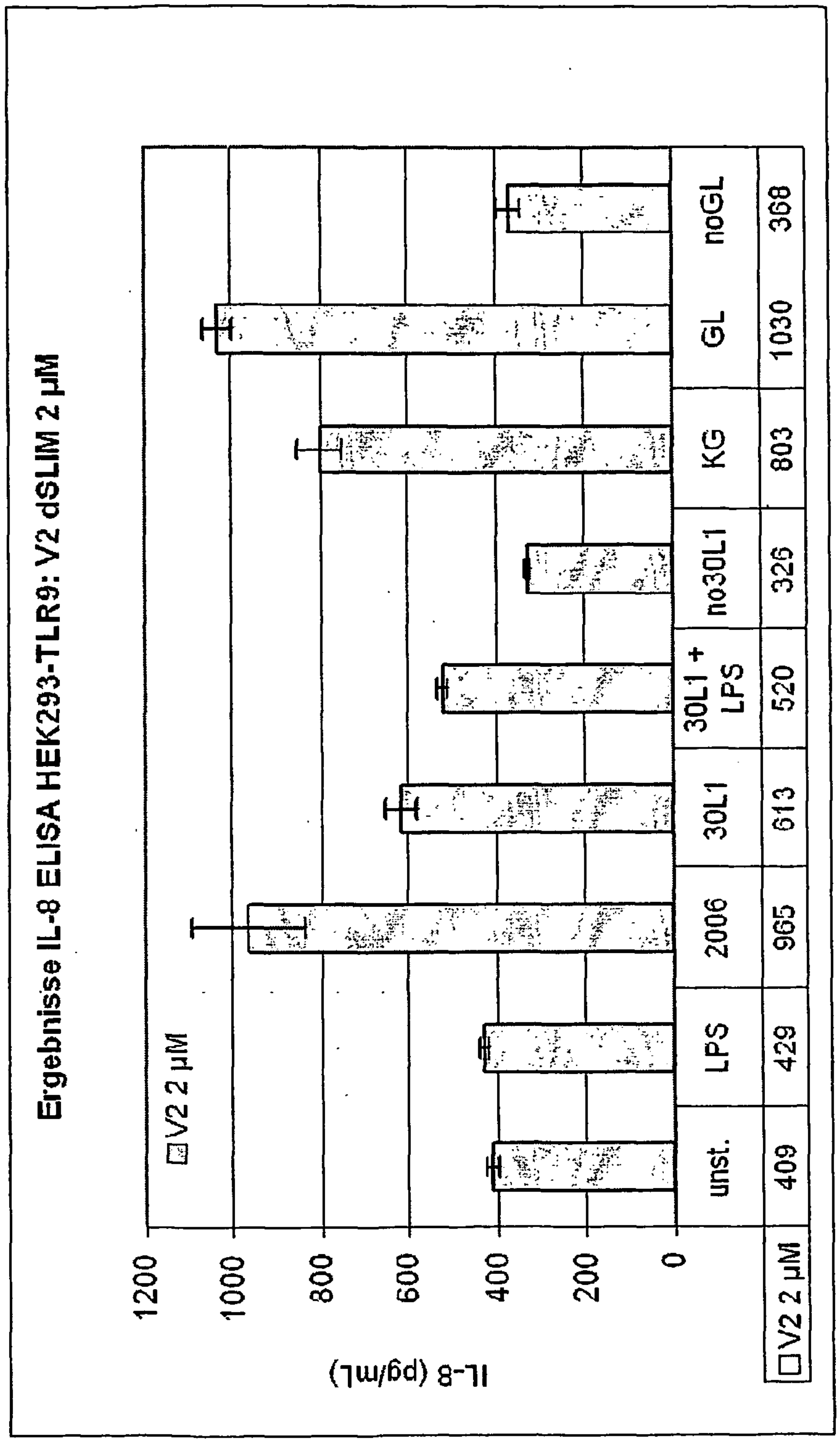
Ergebnisse IL-8 ELISA HEK293-TLR9: V2 dSLIM 2 µM
V2 2 µM
IL-8 (pg/mL)
1200
1000
800
600
400
200
0
unst.
LPS
2006
30L1
30L1 + LPS
no30L1
KG
GL
noGL
V2 2 µM
409
429
965
613
520
326
803
1030
368

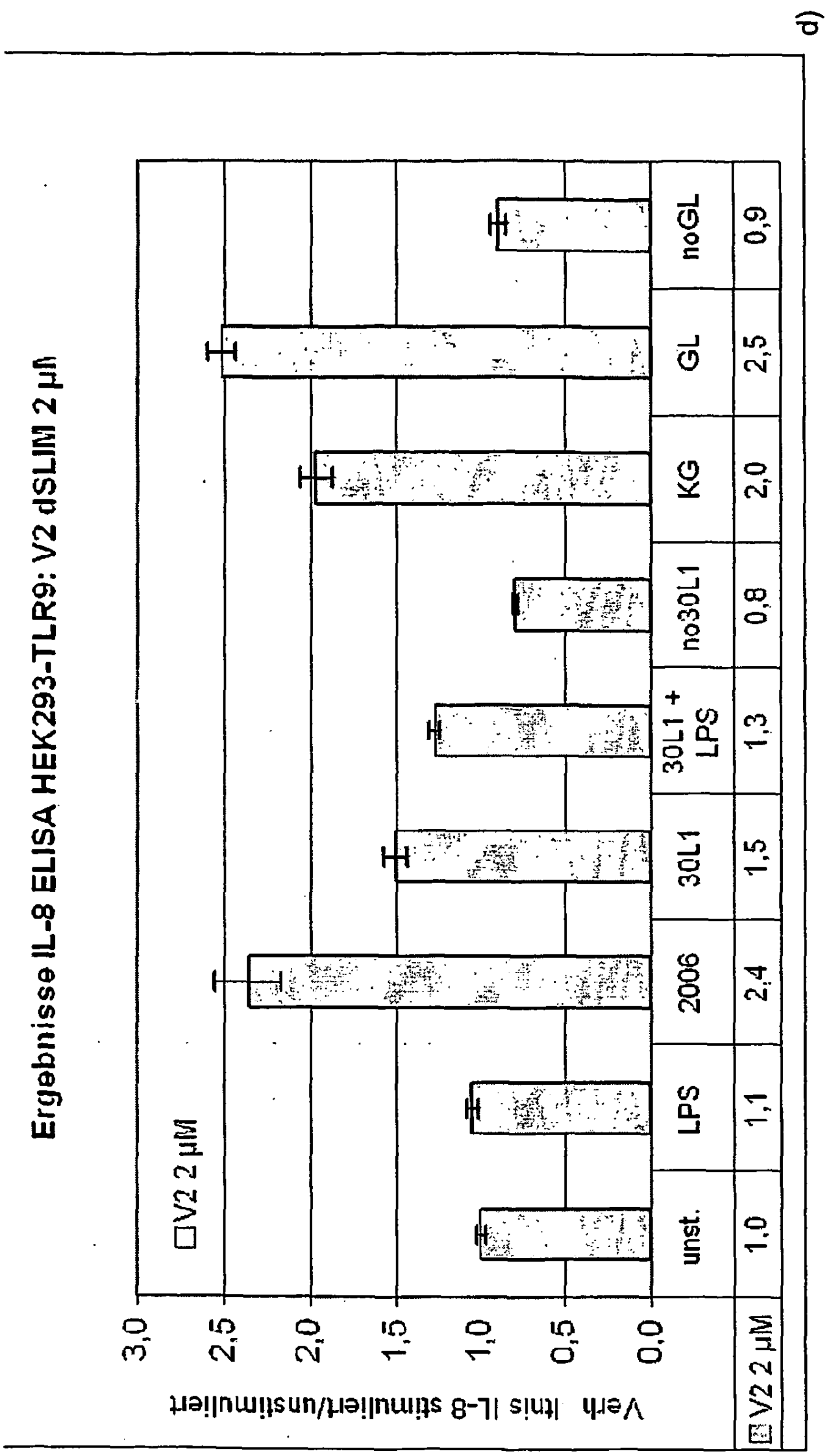


Abbildung 3.3.3: Ergebnisse ELISA IL-8 HEK293-TLR9 verschiedene dSLIM

a)

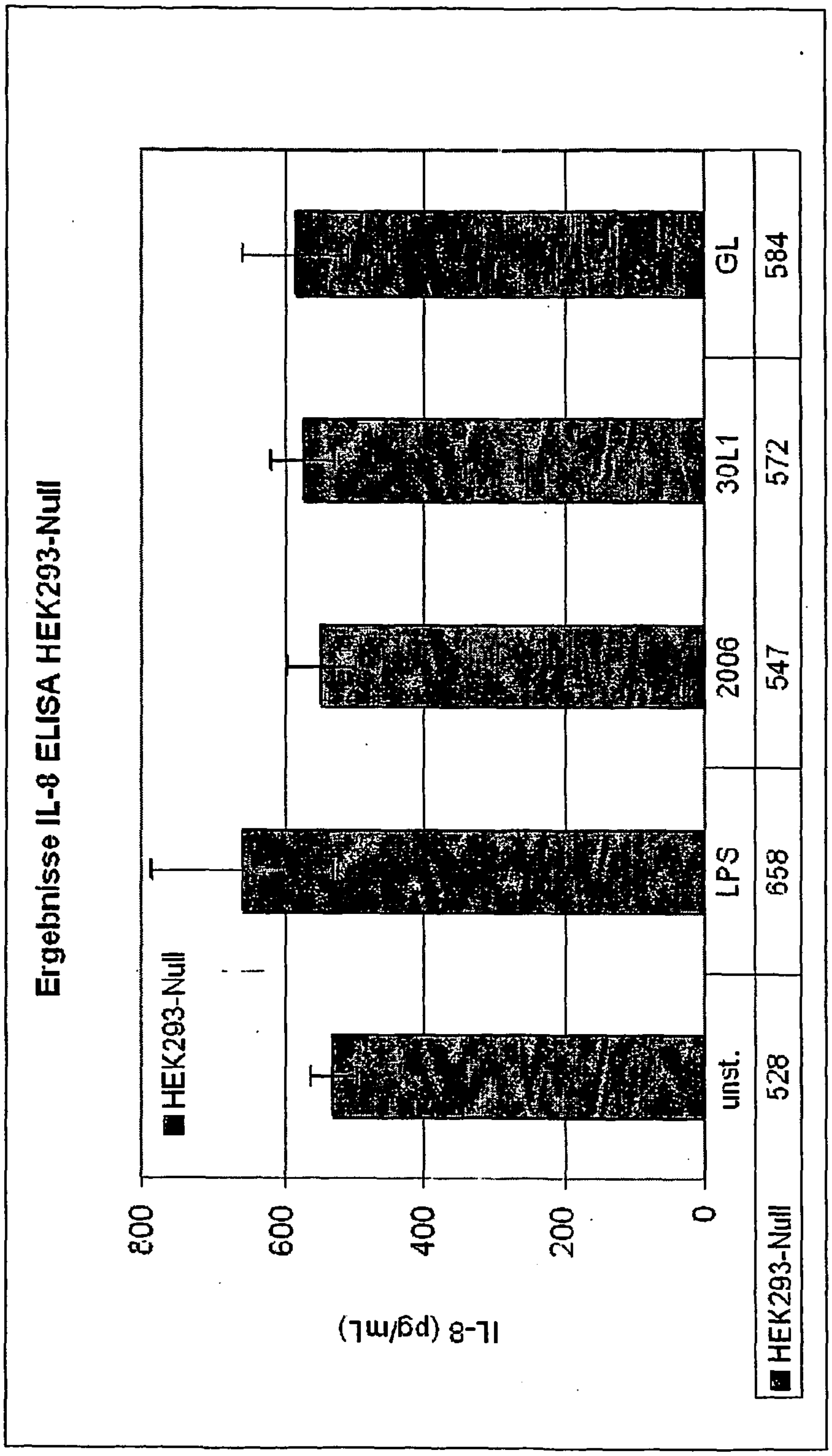
Ergebnisse IL-8 ELISA HEK293-Null
HEK293-Null
IL-8 (pg/mL)
800
600
400
200
0
unst.
LPS
2006
30L1
GL
HEK293-Null
528
658
547
572
584

b)

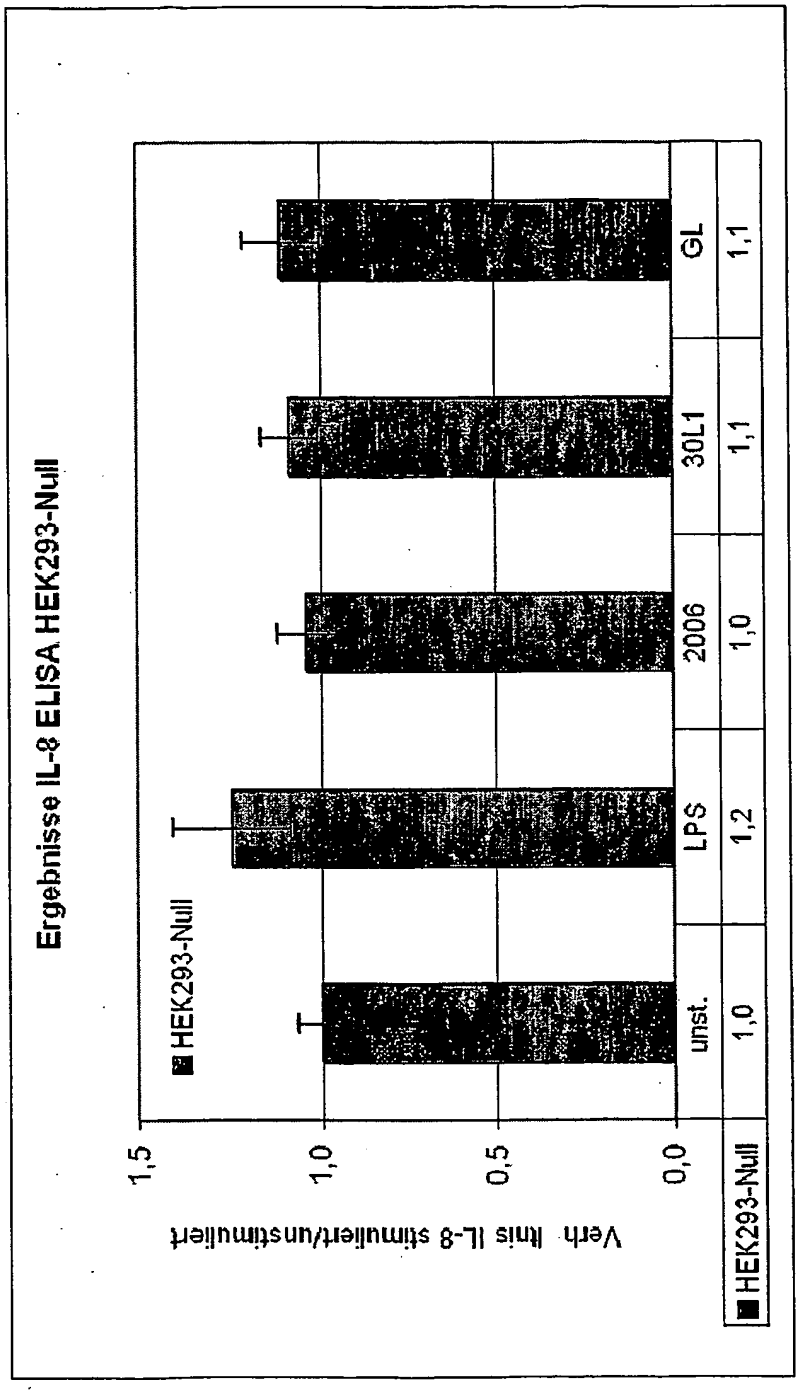


Abbildung 3.3.4: Ergebnisse ELISA IL-8 HEK293-Null verschiedene dSLIM-Konstrukte

a) Überstände HEK293-Null: 400 µL/Ansatz (1 x 106 Zellen/mL); Stimulation 24 h mit 2 µM ODN 2006/dSLIM; 0,5 µg/ml LPS; Stimulation 4 Tage nach Aussäen der Zellen (Mediumwechsel nach 48 h und vor Stimulation); Fehlerbalken: 2 x Standardabweichung aus Doppelbestimmung ELISA.

b) Verhältnisse der Chemokin-Mengen stimulierte zu unstimulierten Ansätzen aus a); Fehlerbalken: Summe relative Fehler aus Doppelbestimmung ELISA.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 1196178 A

### In der Beschreibung aufgeführte Nicht-Patentliteratur

- **Krieg.** *Nat. Med,* 2003, vol. 9, 831-835